# EUROPEAN PATENT SPECIFICATION

(11) **EP 4 642 386 B1**
(45) Date of publication and mention of the grant of the patent: **16.09.2026**
(21) Application number: 23848414.1
(22) Date of filing: 21.12.2023
(51) Int. Cl.: A61F 2/24, A61F 2/95, A61M 25/00

(54) **DELIVERY SYSTEMS AND TETHER ASSEMBLIES FOR PROSTHETIC VALVES**
FREISETZUNGSSYSTEME UND HALTEGURTANORDNUNGEN FÜR KLAPPENPROTHESEN
SYSTÈMES DE POSE ET ENSEMBLES D'ATTACHE POUR VALVES PROTHÉTIQUES

(30) Priority: 29.12.2022 US 202263436051 P; 18.08.2023 US 202363533458 P
(43) Date of publication of application: 05.11.2025
(73) Proprietor: Edwards Lifesciences Corporation, Irvine, CA 92614 (US)
(72) Inventor: VANEVERY, Zachary, Charles, Irvine, CA 92614 (US); DIXON, Eric, Robert, Irvine, CA 92614 (US); JULIAN, Paul, Warren, Irvine, CA 92614 (US); POULSEN, Nikolai, Brent, Irvine, CA 92614 (US); TYLER, Gregory, Scott, II, Irvine, CA 92614 (US); NAWALAKHE, Rupesh, Gajanan, Irvine, CA 92614 (US); EDWARDS, Jesse, Robert, Irvine, CA 92614 (US); GRANT, Ulysses, Tustin, CA 92780 (US); RICKERSON, Cooper, Ryan, Irvine, CA 92614 (US); LANDON, David, Robert, Irvine, CA 92614 (US)
(74) Representative: Eisenführ Speiser
(86) International application number: PCT/US2023/085397
(87) International publication number: WO 2024/145164

(56) References cited:
- WO-A1-2020/198101
- WO-A1-2021/194899
- WO-A1-2022/174057
- US-A1- 2021 244 916
- US-A1- 2021 259 839
- US-B2- 10 583 007

## Description

### CROSS REFERENCE TO RELATED APPLICATIONS

This application claims the benefit of U.S. Provisional Application No. 63/436,051, filed December 29, 2022, and U.S. Provisional Application No. 63/533,458, filed August 18, 2023.

### BACKGROUND

### Field

Certain examples disclosed herein relate generally to prostheses for implantation within a lumen or body cavity and delivery systems for a prosthesis. In particular, the prostheses and delivery systems relate in some examples to replacement heart valves, such as replacement mitral heart valves or replacement tricuspid heart valves.

### Description of the Related Art

Human heart valves, which include the aortic, pulmonary, mitral and tricuspid valves, function essentially as one-way valves operating in synchronization with the pumping heart. The valves allow blood to flow downstream, but block blood from flowing upstream. Diseased heart valves exhibit impairments such as narrowing of the valve or regurgitation, which inhibit the valves' ability to control blood flow. Such impairments reduce the heart's blood-pumping efficiency and can be a debilitating and life-threatening condition. For example, valve insufficiency can lead to conditions such as heart hypertrophy and dilation of the ventricle. Thus, extensive efforts have been made to develop methods and apparatuses to repair or replace impaired heart valves.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open heart surgery. Replacement valves are being designed to be delivered through minimally invasive procedures and even percutaneous procedures. Such replacement valves often include prosthetic valve leaflets that are connected to an expandable frame that is then delivered to the native valve's annulus.

Development of prostheses including but not limited to replacement heart valves that can be compacted for delivery and then controllably expanded for controlled placement has proven to be particularly challenging. An additional challenge relates to the ability of such prostheses to be secured relative to intralumenal tissue, e.g., tissue within any body lumen or cavity, in an atraumatic manner.

Delivering a prosthesis to a desired location in the human body, for example delivering a replacement heart valve to the mitral valve or tricuspid valve, can also be challenging. Obtaining access to perform procedures in the heart or in other anatomical locations may require delivery of devices percutaneously through tortuous vasculature or through open or semi-open surgical procedures. The ability to control the deployment of the prosthesis at the desired location can also be challenging.

WO 2021/194899 A1 relates to delivery systems for delivery of replacement heart valves. This can include mitral, aortic, tricuspid, and pulmonary valves. The delivery systems can include one or more different components and configurations that advantageously improve placement of the replacement heart valves during the operation of the delivery system.

US 2021/0259839 A1 provides apparatuses, systems, and methods for deploying a medical device. In certain instances, the medical device may be a shunt device. In addition, the apparatuses, systems, and methods may include one or more constraining lines arranged through a portion of the implantable medical device and one or more release lines configured to engage the one or more constraining lines.

### SUMMARY

The claimed invention is defined in independent claim 1 and relates to a system for replacing the function of a native atrioventricular valve. Some preferred configurations of the claimed invention are defined in the dependent claims. Also described herein are related aspects, examples, embodiments and arrangements useful for understanding the claimed invention, and which do not necessarily constitute embodiments of the claimed invention. The subject-matter for which protection is sought is defined by the claims.

Examples of the present disclosure may be directed to an implant, which may comprise a prosthesis such as but not limited to a replacement heart valve. Further examples are directed to methods of use to deliver and/or controllably deploy an implant, such as but not limited to a replacement heart valve, to a desired location within the body. In some examples, a replacement heart valve and methods for delivering a replacement heart valve to a native heart valve, such as a mitral valve, an aortic valve, or a tricuspid valve, are provided.

Configurations of delivery systems and release mechanisms for implants may be disclosed herein.

Examples of the present disclosure may include a delivery system for an implant. The delivery system may include an elongate shaft for advancement of the implant to an implantation site and including a proximal end portion and a distal end portion, at least a portion of the elongate shaft comprising a tether assembly. The tether assembly may include a plurality of coupling tethers configured to couple to the implant, a tether manifold for coupling to the plurality of coupling tethers, and a flexible retention tether coupled to the tether manifold and extending proximally from the tether manifold.

Examples of the present disclosure may include a method. The method may comprise delivering an implant to a native heart valve utilizing a delivery system. The delivery system may include an elongate shaft for advancement of the implant to the native heart valve and including a proximal end portion and a distal end portion, at least a portion of the elongate shaft comprising a tether assembly. The tether assembly may include a plurality of coupling tethers configured to couple to the implant, a tether manifold for coupling to the plurality of coupling tethers, and a flexible retention tether coupled to the tether manifold and extending proximally from the tether manifold.

Examples of the present disclosure may include a delivery system for an implant. The delivery system may include an elongate shaft for advancement of the implant to an implantation site and including a proximal end portion and a distal end portion, at least a portion of the elongate shaft comprising a tether assembly and a release assembly. The tether assembly may include one or more coupling tethers configured to couple to the implant, each coupling tether including a loop portion configured to protrude from a respective opening in a portion of the implant. The release assembly may include one or more release tethers configured to extend through one or more of the loop portions to retain the implant to the one or more loop portions, the one or more release tethers configured to be retracted from the one or more loop portions to release the implant from the one or more loop portions.

Examples of the present disclosure may include a method. The method may comprise delivering an implant to a native heart valve utilizing a delivery system. The delivery system may include an elongate shaft for advancement of the implant to the native heart valve and including a proximal end portion and a distal end portion. At least a portion of the elongate shaft may comprise a tether assembly and a release assembly. The tether assembly may include one or more coupling tethers configured to couple to the implant, each coupling tether including a loop portion configured to protrude from a respective opening in a portion of the implant. The release assembly may include one or more release tethers configured to extend through one or more of the loop portions to retain the implant to the one or more loop portions, the one or more release tethers configured to be retracted from the one or more loop portions to release the implant from the one or more loop portions.

Examples of the present disclosure may include a delivery system for an implant. The delivery system may include an elongate shaft for advancement of the implant to an implantation site and including a proximal end portion and a distal end portion. The delivery system may include one or more coupling tethers each including a first portion and a second portion, the first portion configured to couple to the implant to retain the implant to the elongate shaft. The delivery system may include a disintegration assembly configured to connect to the second portion of the one or more coupling tethers and disintegrate the connection to the second portion to release the implant from the elongate shaft.

Examples of the present disclosure may include a method. The method may comprise delivering an implant to a native heart valve utilizing a delivery system. The delivery system may include an elongate shaft for advancement of the implant to the native heart valve and including a proximal end portion and a distal end portion, one or more coupling tethers each including a first portion and a second portion, the first portion configured to couple to the implant to retain the implant to the elongate shaft, and a disintegration assembly configured to connect to the second portion of the one or more coupling tethers and disintegrate the connection to the second portion to release the implant from the elongate shaft.

Examples of the present disclosure may include a delivery system for an implant. The delivery system may include a delivery catheter for advancement of the implant to an implantation site, the delivery catheter including an elongate shaft that is adapted to be deflected in one or more planes. The elongate shaft may have an outer sheath having a distal end portion and a proximal end portion and a length, a pull tether having a distal end portion and a proximal end portion and extending along the length of the outer sheath, the distal end portion coupled to the outer sheath, a compression coil surrounding at least a portion of the pull tether and including a distal end portion and a proximal end portion, the compression coil being unconnected directly to the outer sheath and slidable relative to the pull tether, and a tube surrounding at least a portion of the pull tether and including a distal end portion and a proximal end portion, the tube being unconnected directly to the outer sheath and slidable relative to the pull tether, the distal end portion of the tube adapted to abut the proximal end portion of the compression coil. The delivery catheter may include a support plate including an opening for the pull tether to pass through, the support plate adapted to abut the proximal end portion of the tube. The delivery catheter may include a housing slidably engaged with the proximal end portion of the outer sheath. The delivery catheter may include an actuator assembly for applying a tension force to the pull tether to deflect the elongate shaft, whereby a force exerted against the compression coil is transmitted to the support plate through the tube.

Examples of the present disclosure may include a method. The method may comprise delivering an implant to a native heart valve utilizing a delivery system. The delivery system may include a delivery catheter for advancement of the implant to an implantation site, the delivery catheter including an elongate shaft that is adapted to be deflected in one or more planes. The elongate shaft may have an outer sheath having a distal end portion and a proximal end portion and a length, a pull tether having a distal end portion and a proximal end portion and extending along the length of the outer sheath, the distal end portion coupled to the outer sheath, a compression coil surrounding at least a portion of the pull tether and including a distal end portion and a proximal end portion, the compression coil being unconnected directly to the outer sheath and slidable relative to the pull tether, and a tube surrounding at least a portion of the pull tether and including a distal end portion and a proximal end portion, the tube being unconnected directly to the outer sheath and slidable relative to the pull tether, the distal end portion of the tube adapted to abut the proximal end portion of the compression coil. The delivery catheter may include a support plate including an opening for the pull tether to pass through, the support plate adapted to abut the proximal end portion of the tube. The delivery catheter may include a housing slidably engaged with the proximal end portion of the outer sheath. The delivery catheter may include an actuator assembly for applying a tension force to the pull tether to deflect the elongate shaft, whereby a force exerted against the compression coil is transmitted to the support plate through the tube.

Examples of the present disclosure may include a delivery system for an implant. The delivery system may include a delivery catheter for advancement of the implant to an implantation site, the delivery catheter including an elongate shaft that is adapted to be deflected in one or more planes. The elongate shaft may include an outer sheath having a distal end portion and a proximal end portion and a length, a pull tether having a distal end portion and a proximal end portion and extending along the length of the outer sheath, the distal end portion coupled to the outer sheath, a lumen surrounding at least a portion of the pull tether and including a distal end portion and a proximal end portion, the lumen being unconnected directly to the outer sheath and slidable relative to the pull tether. The delivery catheter may include an actuator assembly for applying a tension force to the pull tether to deflect the elongate shaft and simultaneously applying a distal compressive force to the lumen.

Examples of the present disclosure may include a method. The method may comprise delivering an implant to a native heart valve utilizing a delivery system. The delivery system may include a delivery catheter for advancement of the implant to an implantation site, the delivery catheter including an elongate shaft that is adapted to be deflected in one or more planes. The elongate shaft may include an outer sheath having a distal end portion and a proximal end portion and a length, a pull tether having a distal end portion and a proximal end portion and extending along the length of the outer sheath, the distal end portion coupled to the outer sheath, a lumen surrounding at least a portion of the pull tether and including a distal end portion and a proximal end portion, the lumen being unconnected directly to the outer sheath and slidable relative to the pull tether. The delivery catheter may include an actuator assembly for applying a tension force to the pull tether to deflect the elongate shaft and simultaneously applying a distal compressive force to the lumen.

Examples of the present disclosure may include a delivery system for an implant. The delivery system may include an elongate shaft for advancement of the implant to an implantation site and including a proximal end portion and a distal end portion, the elongate shaft adapted to deflect in a first plane about a bend portion of the elongate shaft. The delivery system may include a control mechanism adapted to control deflection of the elongate shaft. The control mechanism may include a deflection actuator adapted to deflect the elongate shaft in the first plane about the bend portion, a pull tether assembly including a pull tether and an adaptor, the pull tether including a distal end portion coupled to the elongate shaft and a proximal end portion coupled to the adaptor, and a knob assembly adapted to be rotated in a first direction to produce depth of the distal end portion of the elongate shaft relative to the bend portion, and be rotated in a second direction to retract the adaptor to deflect the elongate shaft to produce height of the elongate shaft in a direction opposed to the depth.

Examples of the present disclosure may include a method. The method may comprise delivering an implant to a native heart valve utilizing a delivery system. The delivery system may include an elongate shaft for advancement of the implant to an implantation site and including a proximal end portion and a distal end portion, the elongate shaft adapted to deflect in a first plane about a bend portion of the elongate shaft. The delivery system may include a control mechanism adapted to control deflection of the elongate shaft. The control mechanism may include a deflection actuator adapted to deflect the elongate shaft in the first plane about the bend portion, a pull tether assembly including a pull tether and an adaptor, the pull tether including a distal end portion coupled to the elongate shaft and a proximal end portion coupled to the adaptor, and a knob assembly adapted to be rotated in a first direction to produce depth of the distal end portion of the elongate shaft relative to the bend portion, and be rotated in a second direction to retract the adaptor to deflect the elongate shaft to produce height of the elongate shaft in a direction opposed to the depth.

Examples of the present disclosure may include a delivery system for an implant. The delivery system may include a delivery catheter for advancement of the implant to an implantation site, the delivery catheter including: a guide wire sheath having a proximal end portion and a distal end and an interior lumen for passage of a guide wire therethrough, the interior lumen having a diameter, and a spacer body positioned at the distal end of the guide wire sheath and protruding distally from the distal end, the spacer body including an opening for the guide wire to protrude therefrom and a cavity having a diameter that is greater than the diameter of the interior lumen and adapted for the guide wire to deflect within.

Examples of the present disclosure may include a method. The method may comprise delivering an implant to a native heart valve utilizing a delivery system. The delivery system may include a delivery catheter for advancement of the implant to an implantation site, the delivery catheter including: a guide wire sheath having a proximal end portion and a distal end and an interior lumen for passage of a guide wire therethrough, the interior lumen having a diameter, and a spacer body positioned at the distal end of the guide wire sheath and protruding distally from the distal end, the spacer body including an opening for the guide wire to protrude therefrom and a cavity having a diameter that is greater than the diameter of the interior lumen and adapted for the guide wire to deflect within.

### BRIEF DESCRIPTION OF THE DRAWINGS

FIG. 1 illustrates a side view of a delivery system for an implant.
FIG. 2 shows a perspective view of an outer sheath subassembly of a delivery device of the delivery system of FIG. 1.
FIG. 3 illustrates a side cross-sectional view of a capsule subassembly of the outer sheath subassembly of FIG. 2.
FIG. 4 shows a perspective view of a capsule stent, or distal hypotube, of the outer sheath subassembly of FIG. 2.
FIG. 5 schematically illustrates how a portion of a liner extending along a length of the outer sheath subassembly can have built-in slack to facilitate flexible bending of the outer sheath subassembly.
FIG. 6 shows a perspective view of a rail subassembly of the delivery device of the delivery system of FIG. 2.
FIG. 7 shows a side cross-sectional view of the rail subassembly of FIG. 6.
FIGS. 8 and 9 schematically illustrate how an outer compression coil and pull wire can have a longer length than an inner compression coil and pull wire of the rail subassembly.
FIG. 10 shows a perspective view of a mid-shaft subassembly of the delivery device of the delivery system of FIG. 2.
FIG. 11 illustrates a side cross-sectional view of the mid-shaft subassembly of FIG. 10.
FIG. 12 illustrates a side view of a tether assembly.
FIG. 13 illustrates a side view of an end portion of a tether assembly.
FIG. 14 illustrates a side view of a portion of a tether assembly.
FIG. 15 illustrates a side view of a tether assembly.
FIG. 16 illustrates a side view of a tether assembly.
FIG. 17 illustrates a perspective view of an end portion of a tether assembly.
FIG. 18 illustrates a perspective view of an implant coupled to a tether assembly.
FIG. 19 illustrates a side view of a release assembly.
FIG. 20 illustrates a perspective view of a release assembly extending through loop portions of a tether assembly.
FIG. 21 illustrates a perspective view of a release assembly retracted through a loop portion of a tether assembly.
FIG. 22 illustrates a perspective view of a release assembly retracted through loop portions of a tether assembly.
FIG. 23 illustrates a perspective view of a tether assembly released from an implant.
FIG. 24 illustrates a perspective view of a handle of a delivery system.
FIG. 25 illustrates a cross-sectional view of the handle shown in FIG. 24.
FIG. 26 illustrates a schematic view of a delivery system approaching an implantation site.
FIG. 27 illustrates a schematic view of a delivery system approaching a tricuspid valve.
FIG. 28 illustrates a schematic view of an implant being deployed to a tricuspid valve.
FIG. 29 illustrates a schematic view of an implant being deployed to a tricuspid valve.
FIG. 30 illustrates a schematic view of a tether assembly released from an implant.
FIG. 31 illustrates a side view of a prosthetic valve deployed to a tricuspid valve.
FIG. 32 illustrates a partially exploded perspective view of components of a disintegration assembly.
FIG. 33 illustrates a perspective view of an implant retained by a pusher.
FIG. 34 illustrates a side view of an implant extended from the pusher shown in FIG. 33.
FIG. 35 illustrates a side cross-sectional view of a delivery system including a disintegration assembly.
FIG. 36 illustrates a side cross-sectional view of the delivery system shown in FIG. 35 with the disintegration assembly activated.
FIG. 37 illustrates a side view of disintegrated coupling tethers extending to an implant.
FIG. 38 illustrates a side cross-sectional view of components of a disintegration assembly.
FIG. 39 illustrates a side cross-sectional view of the disintegration assembly of FIG. 38 with an intermediate body disintegrated.
FIG. 40 illustrates a side cross-sectional view of components of a disintegration assembly.
FIG. 41 illustrates a side cross-sectional view of the disintegration assembly of FIG. 40 with the disintegration assembly activated.
FIG. 42 illustrates a side view of a plurality of coupling tethers extended distally.
FIG. 43 illustrates a partial cross-sectional view of the coupling tethers shown in FIG. 42 forming loops.
FIG. 44 illustrates a partial cross-sectional view of the coupling tethers shown in FIG. 42 forming loops.
FIG. 45 illustrates a perspective view of a woven or braided cylinder.
FIG. 46 illustrates a perspective view of a ring cut from the woven or braided cylinder shown in FIG. 45.
FIG. 47 illustrates a side partial cross-sectional view of the ring shown in FIG. 46 coupled to a tether manifold.
FIG. 48 illustrates a plan view of a weave.
FIG. 49 illustrates a plan view of the weave shown in FIG. 48 cut.
FIG. 50 illustrates a plan view of the weave shown in FIG. 49 folded upon itself.
FIG. 51 illustrates a side cross-sectional schematic view of a weave pattern.
FIG. 52 illustrates a side schematic view of a length of sections of a weave pattern.
FIG. 53 illustrates a side schematic view of a braiding assembly.
FIG. 54 illustrates a side schematic view of braiding by a braiding assembly.
FIG. 55 illustrates a side view of a braided tether assembly.
FIG. 56A illustrates a perspective view of a release assembly.
FIG. 56B illustrates a cross-sectional view of the release assembly shown in FIG. 56A.
FIG. 57 illustrates a cross-sectional view of a release assembly.
FIG. 58 illustrates a plan view of a tether assembly.
FIG. 59 illustrates a partial cross-sectional view of the tether assembly shown in FIG. 58.
FIG. 60A illustrates a perspective view of a tether manifold.
FIG. 60B illustrates a partial cross-sectional view of a tether assembly utilizing the tether manifold shown in FIG. 60A.
FIG. 61 illustrates a cross-sectional view of the tether assembly of FIG. 60B along line I-I.
FIG. 62 illustrates a side view of a delivery system.
FIG. 63 illustrates a perspective view of the handle of the delivery system shown in FIG. 62.
FIG. 64 illustrates a side view of the handle of the delivery system shown in FIG. 62.
FIG. 65 illustrates a cross-sectional perspective view of the handle of the delivery system shown in FIG. 62 along a mid-line.
FIG. 66 illustrates a cross-sectional side view of the handle of the delivery system shown in FIG. 62 along a mid-line.
FIG. 67 illustrates a perspective view of a rail shaft or elongate shaft of a delivery system.
FIG. 68 illustrates a perspective view of the rail shaft shown in FIG. 67 with an outer sheath excluded from view.
FIG. 69 illustrates a perspective view of a distal insert of the rail shaft shown in FIG. 67.
FIG. 70 illustrates a side perspective view of an intermediate insert of the rail shaft shown in FIG. 67.
FIG. 71 illustrates a perspective view of a rail shaft with an outer sheath excluded from view.
FIG. 72 illustrates a perspective view of an intermediate insert of the rail shaft shown in FIG. 71.
FIG. 73 illustrates a perspective view of an adaptor of a rail shaft.
FIG. 74 illustrates a perspective view of the adaptor of the rail shaft with an outer sheath adaptor excluded from view.
FIG. 75 illustrates a cross-sectional view of the adaptor shown in FIG. 73.
FIG. 76 illustrates a perspective cross-sectional view of a handle utilizing an adaptor as shown in FIG. 73.
FIG. 77 illustrates a side cross-sectional view of actuator assemblies.
FIGS. 78 and 79 illustrate a schematic view of operation of one of the actuator assemblies shown in FIG. 77.
FIG. 80 illustrates a side view of the actuator assemblies of FIG. 77 positioned upon a handle.
FIG. 81 illustrates a side cross-sectional view of a knob assembly.
FIG. 82 illustrates a side partial cross-sectional view of the knob assembly shown in FIG. 81.
FIG. 83 illustrates a perspective partial cross-sectional view of the knob assembly shown in FIG. 81.
FIG. 84 illustrates a perspective cross-sectional view of the knob assembly shown in FIG. 81.
FIG. 85 illustrates a perspective partial cross-sectional view of the knob assembly shown in FIG. 81 from an opposite side of the handle than shown in FIG. 81.
FIG. 86 illustrates a side partial cross-sectional view of a handle and knob assembly.
FIG. 87 illustrates a side schematic view of a delivery system according to the configuration shown in FIG. 86.
FIG. 88 illustrates a side partial cross-sectional view of a handle and knob assembly.
FIG. 89 illustrates a side schematic view of a delivery system according to the configuration shown in FIG. 88.
FIG. 90 illustrates a side partial cross-sectional view of a handle and knob assembly.
FIG. 91 illustrates a side schematic view of a delivery system according to the configuration shown in FIG. 90.
FIG. 92 illustrates a side partial cross-sectional view of a handle and knob assembly.
FIG. 93 illustrates a side schematic view of a delivery system according to the configuration shown in FIG. 92.
FIG. 94 illustrates a side partial transparent view of a handle and knob assembly.
FIG. 95 illustrates a side cross-sectional view of the handle and knob assembly shown in FIG. 94.
FIG. 96 illustrates a perspective view of an alignment feature of the knob assembly shown in FIG. 94.
FIG. 97 illustrates a side cross-sectional view of the handle and knob assembly shown in FIG. 94.
FIG. 98 illustrates a side cross-sectional view of the handle and knob assembly shown in FIG. 94.
FIG. 99 illustrates a perspective view of a pull tether adaptor.
FIG. 100 illustrates a side cross-sectional view of a handle and knob assembly utilizing a pull tether adaptor as shown in FIG. 99.
FIG. 101 illustrates a side cross-sectional view of a handle and knob assembly utilizing a pull tether adaptor as shown in FIG. 99.
FIG. 102 illustrates a side cross-sectional view of a handle and knob assembly utilizing a pull tether adaptor as shown in FIG. 99.
FIG. 103 illustrates a side cross-sectional view of a handle and knob assembly utilizing a pull tether adaptor as shown in FIG. 99.
FIG. 104 illustrates a side cross-sectional view of a handle and knob assembly utilizing a pull tether adaptor as shown in FIG. 99.
FIG. 105 illustrates a perspective cross-sectional view of a proximal end portion of the handle of the delivery system shown in FIG. 62.
FIG. 106 illustrates a side view of a nose cone.
FIG. 107 illustrates a side cross-sectional view of a guide wire advancing through the nose cone shown in FIG. 106.
FIG. 108 illustrates a side cross-sectional view of a guide wire sheath and spacer body.
FIG. 109 illustrates a side cross-sectional view of the guide wire sheath and spacer body of FIG. 108 with a guide wire advancing through the spacer body.
FIG. 110 illustrates a perspective view of a guide wire sheath and spacer body.
FIG. 111 illustrates a side cross-sectional view of the guide wire sheath and spacer body of FIG. 110 with a guide wire advancing through the spacer body.
FIG. 112 illustrates a side cross-sectional view of a guide wire sheath and spacer body.
FIG. 113 illustrates a side view of a guide wire sheath and spacer body.
FIG. 114 illustrates a side view of a guide wire sheath and spacer body.
FIG. 115 illustrates an end view of a spacer body.
FIG. 116 illustrates a side view of a guide wire sheath and spacer body.
FIG. 117 illustrates a side view of a guide wire sheath and spacer body.
FIG. 118 illustrates a transverse cross-sectional view of an elongate shaft of a delivery catheter.
FIG. 119 illustrates a transverse cross-sectional view of an elongate shaft of a delivery catheter.
FIG. 120 illustrates a perspective view of a stabilizer assembly.
FIG. 121 illustrates a perspective view of a prosthetic heart valve.
FIG. 122 illustrates a distal end view or outflow end view of the prosthetic heart valve shown in FIG. 121.
FIG. 123 illustrates a cross-sectional schematic view of the prosthetic heart valve shown in FIG. 121.

### DETAILED DESCRIPTION

The present specification and drawings provide aspects and features of the disclosure in the context of several examples of implants such as prosthetic valves or replacement heart valves, and delivery systems and methods that are configured for use in the vasculature of a patient, such as for replacement of natural heart valves in a patient. These examples may be discussed in connection with replacing specific valves such as the patient's aortic, tricuspid, or mitral valve. However, it is to be understood that the features and concepts discussed herein can be applied to products other than heart valve implants. For example, features described herein can be applied to other medical implants, for example other types of prostheses, for use elsewhere in the body, such as within an artery, a vein, or other body cavities or locations. In addition, particular features of a valve, delivery system, etc. should not be taken as limiting, and features of any one example discussed herein can be combined with features of other examples as desired and when appropriate. While certain of the examples described herein are described in connection with a transfemoral delivery approach, it should be understood that these examples can be used for other delivery approaches such as, for example, transapical or transjugular approaches. Moreover, it should be understood that certain of the features described in connection with some examples can be incorporated with other examples, including those which are described in connection with different delivery approaches. In the following, 1 inch (or 1")=2.54 cm.

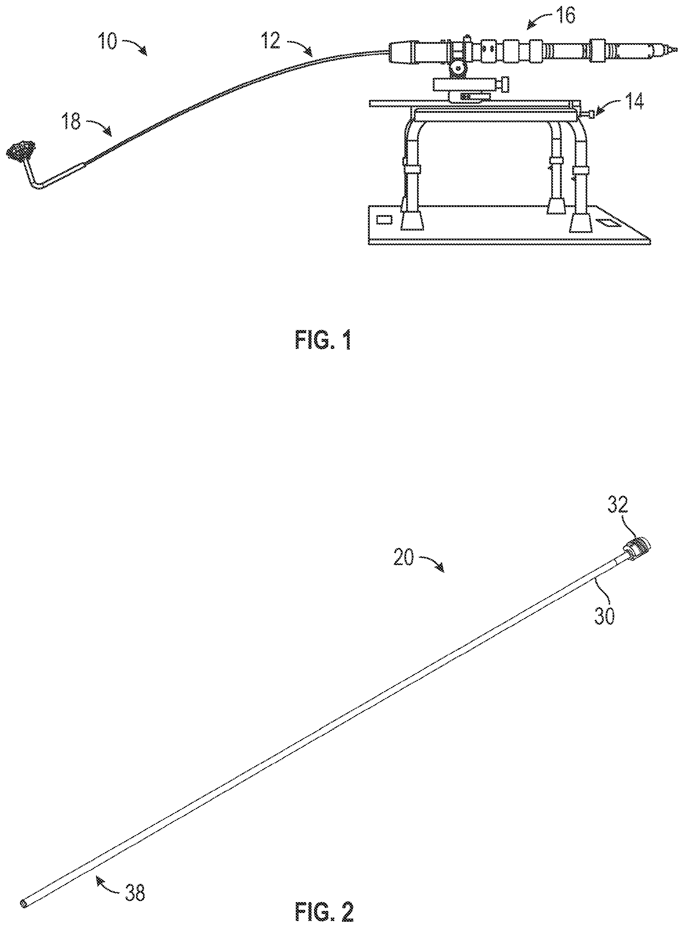

FIG. 1 illustrates an example of a delivery system 10. The delivery system 10 can be used to deploy an implant as disclosed herein, or another form of implant. Features of implants or prosthetic heart valves that may be utilized are disclosed in U.S. Provisional Application No. 63/436,051, filed December 29, 2022, and U.S. Provisional Application No. 63/533,458, filed August 18, 2023.

An implant such as a prosthetic heart valve may be delivered to a subject's mitral or tricuspid valve annulus or other heart valve location in various manners, such as by open surgery, minimally-invasive surgery, and percutaneous or transcatheter delivery through the subject's vasculature. Example transfemoral approaches are described further in U.S. Pat. Publ. No. 2015/0238315, published August 27, 2015. While the delivery system 10 is described in connection with a percutaneous delivery approach, and more specifically a transfemoral delivery approach, it should be understood that features of the delivery system 10 can be applied to other delivery approaches, including delivery systems for a transapical delivery approach.

The delivery system 10 may be used to deploy a prosthesis, such as a replacement heart valve, to a location within the body of a subject. The delivery system 10 may include multiple components, devices, or subassemblies. As shown in FIG. 1, the delivery system 10 may include an elongate catheter, delivery catheter, or delivery device 12, and a stabilizer assembly 14, and other components as desired. The delivery device 12 may include an elongate shaft or shaft assembly 18 and a housing in the form of a handle 16. The housing may be at the proximal end portion of the elongate shaft or shaft assembly 18. The shaft assembly 18 may include one or more shafts. A plurality of shafts may be provided according to examples herein, although in examples a single shaft may be utilized.

The elongate catheter or delivery device 12 can be pre-attached to an implant (e.g., a valve prosthesis or replacement heart valve) and the delivery device 12 may be configured to facilitate delivery and implantation of the implant to and at a desired target location (e.g., a mitral or tricuspid heart valve annulus, among other locations). The implant may be pre-attached within a distal end portion of the shaft assembly 18 and removably tethered to one or more retention components of the shaft assembly 18 during manufacturing or assembly. The pre-loaded delivery device 12 may then be packaged, sterilized, and shipped for use by one or more clinicians. In accordance with several examples, the delivery device 12 is ready for use upon removal from its packaging and may not require loading of the implant by a clinician. In examples, the delivery device 12 may be flushed and loaded prior to use.

The elongate catheter or delivery device 12 can include an elongate shaft or shaft assembly 18 comprising a proximal end portion and a distal end portion, with a handle 16 coupled to the proximal end portion of the shaft assembly 18. The elongate catheter or delivery device 12 can be used to hold the implant (e.g., prosthesis, replacement heart valve) for advancement of the same through the vasculature to a treatment location. The elongate shaft or shaft assembly 18 may be for advancing the implant through a patient's vasculature to an implantation site (e.g., a native atrioventricular valve). In some examples, the elongate shaft or shaft assembly 18 can hold at least a portion of an expandable implant (e.g., prosthesis, replacement heart valve) in a compressed state for advancement of the implant within the body. The elongate shaft or shaft assembly 18 may then be used to allow controlled expansion of the implant at a desired implantation location (e.g. treatment location). In some examples, the shaft assembly 18 may be used to allow for sequential controlled expansion of the implant as discussed in detail below.

The elongate shaft or shaft assembly 18 of the delivery device 12 can include one or more shafts. In examples, a plurality of shafts may be provided. The plurality of shafts may include one or more subassemblies or shafts, such as an outer sheath shaft or subassembly 20, a rail shaft or subassembly 22, a mid shaft or mid shaft subassembly 24, a tether assembly or subassembly 26, a release assembly or subassembly 28, and/or a nose cone shaft or subassembly. In some examples, the shaft assembly 18 of the elongate catheter or delivery device 12 may not have all of the subassemblies or shafts disclosed herein. The delivery device 12 may include multiple layers of concentric shafts, subassemblies, or lumens. The various lumen or shaft subassemblies may be described starting from an outermost layer. In some examples, the shafts or subassemblies described may be in a different radial order than is discussed.

FIG. 2 shows a perspective view of an example of the outer sheath shaft or subassembly 20 of the elongate catheter or delivery device 12 of the delivery system 10. The outer sheath shaft or subassembly 20 forms a radially outer covering, or sheath, to cover and surround an implant retention area for retaining the implant, and prevent at least a portion of the implant (e.g., replacement heart valve or valve prosthesis) from radially expanding until ready for implantation. Specifically, the outer sheath subassembly 20 can prevent a distal end portion of an implant from radially expanding.

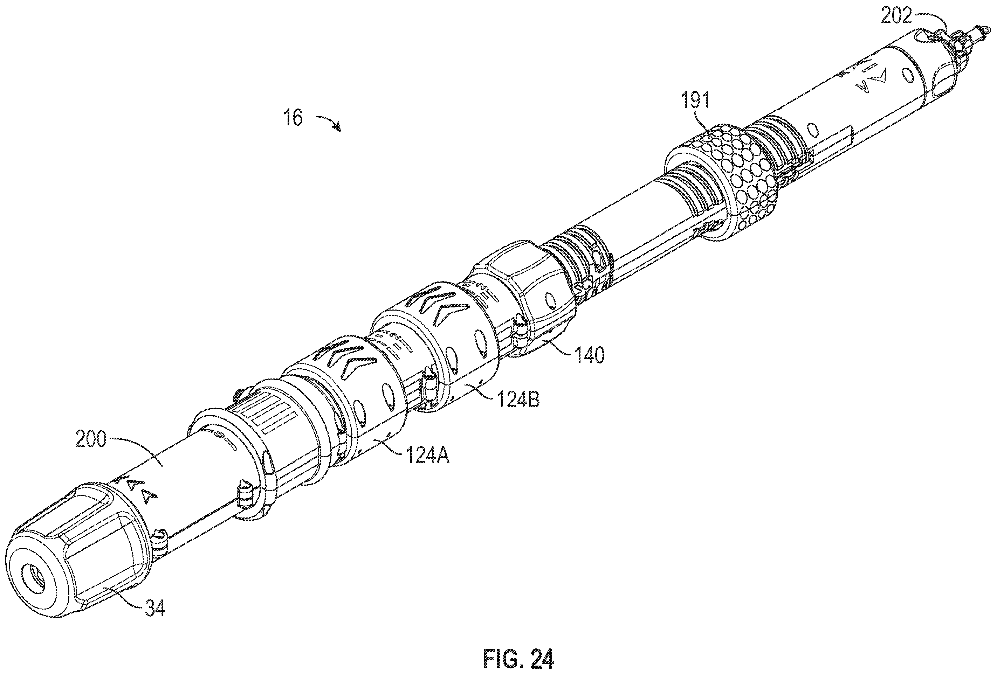

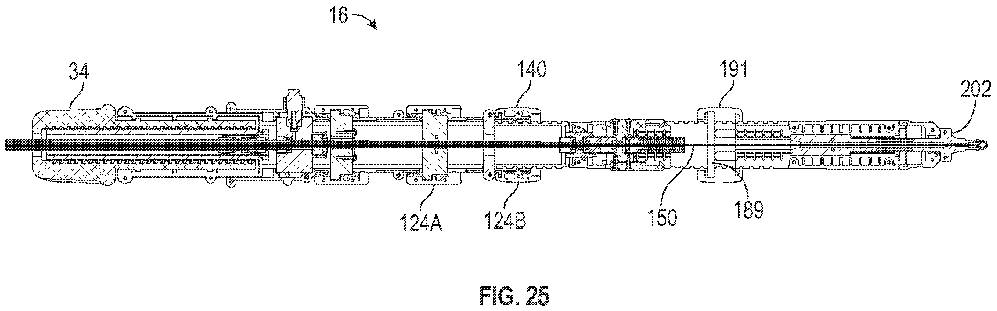

The outer sheath shaft or subassembly 20 can include an outer proximal shaft 30 having a proximal end portion operably coupled (e.g., via threaded outer sheath adaptor 32 at a proximal portion of the outer sheath shaft or subassembly 20) to a capsule actuator or knob 34 (which may be a distal-most actuator or knob, as shown in FIGS. 24 and 25) of the handle 16 such that rotation of the capsule knob 34 causes proximal and distal movement in the form of translation of the outer sheath subassembly 20 (e.g., clockwise and counter-clockwise rotation). A capsule subassembly 36 can be attached to a distal end of the outer proximal shaft 30. The capsule of the capsule subassembly 36 is at the distal end portion of the elongate shaft or shaft assembly 18 and is adapted to maintain the prosthetic heart valve in a compressed state. The components of the outer sheath shaft or subassembly 20 can form an outer-most lumen for the other shafts or subassemblies to pass through.

The outer proximal shaft 30 may be a tube formed of a plastic, but could also be formed of a metal hypotube or other material. The outer proximal shaft 30 may include an outer jacket or liner made of fluorinated ethylene propylene (FEP) material, polytetrafluoroethylene (PTFE) material, ePTFE material, or other polymeric material so as to make the outer surface of the outer proximal shaft 30 smooth and hemostatic. The outer proximal shaft 30 may include a connector (e.g., flexible reflow member) at its distal end to facilitate connection or coupling to the capsule subassembly 36. At least a portion of the outer proximal shaft 30 may comprise a laser cut hypotube with a flexible pattern, such as a universally flexible pattern. An interrupted spiral pattern or an interrupted coil may be utilized.

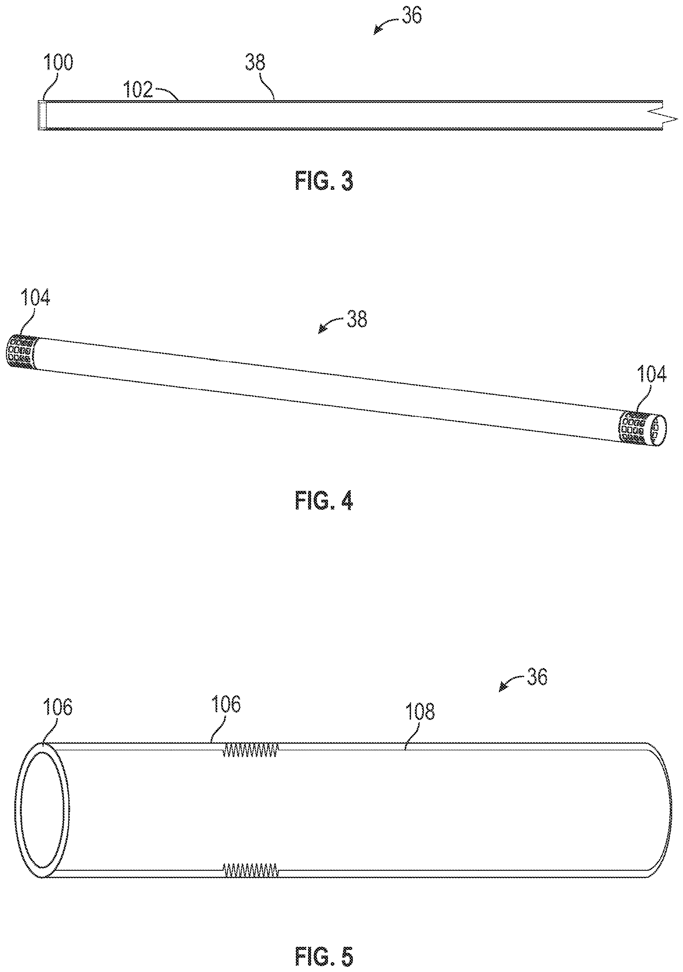

FIG. 3 shows a side cross-section view of the capsule subassembly 36. The capsule subassembly 36 may include a distal hypotube, or capsule stent 38, an inner liner inside of the hypotube 38, a distal capsule tip 100, and one or more outer liners or jackets 102 surrounding the hypotube 38. The one or more outer liners or jackets 102 may comprise PEBAX or other suitable polymer or thermoplastic elastomer material, such as polytetrafluoroethylene (PTFE) or expanded polytetrafluoroethylene (ePTFE). The inner liner may comprise PTFE, which may be pre-compressed before application to the inside of the hypotube 38. The distal capsule tip 100 may comprise an atraumatic tip adapted to act as a funnel to facilitate recapture (e.g., crimping) of a valve prosthesis or other implant. The distal capsule tip 100 may be comprised of polyetheretherketone (PEEK) or other thermoplastic, polymeric, or metallic material. The distal capsule tip 100 may be loaded with radiopaque material (e.g., 5-40% barium sulfate loading) to facilitate detection (e.g., made fluorogenic) under radiographic imaging (e.g., fluoroscopy). The distal capsule tip 100 may fit within an open distal end of the hypotube 38.

FIG. 4 shows a perspective view of the distal hypotube, or capsule stent 38. The capsule stent 38 can be formed from one or more materials, such as PTFE, ePTFE, polyether block amide (Pebax^{®}), polyetherimide (Ultem^{®}), PEEK, urethane, Nitinol, stainless steel, and/or any other biocompatible material. The capsule stent 38 is preferably flexible while still maintaining a sufficient degree of radial strength to maintain an implant (e.g., replacement valve) within the capsule stent 38 without substantial radial deformation, which could increase friction between the capsule stent 38 and an implant contained therein. The capsule stent 38 also preferably has sufficient column strength to resist buckling, and sufficient tear resistance to reduce or eliminate the possibility of the implant tearing and/or damaging the capsule stent 38. The proximal end and/or distal end of the distal hypotube, or capsule stent 38 may include multiple laser cut windows 104 adapted to make the proximal and/or distal end fluorogenic and/or echogenic to facilitate visualization under certain imaging modalities (e.g., noninvasive ultrasound imaging or invasive fluoroscopic imaging). In several implementations, a separate radiopaque element or member is not added to the hypotube 38 to facilitate imaging because of the presence of the laser cut windows 104. The laser cut windows 104 may also promote adhesion of the outer jacket 102 to the capsule stent 38 and to the inner liner(s) by allowing glue or other adhesive to flow through the laser cut windows 104. One or more layers of connection members made of PEBAX or other suitable material may surround the laser cut windows 104 to facilitate coupling of the hypotube, or capsule stent 38 to the distal capsule tip 100.

The hypotube 38 may be formed of a plastic or metallic material. In some implementations, the hypotube 38 can be a metal hypotube. If metallic, the metallic material of the hypotube 38 may comprise cobalt chrome, stainless steel, titanium or metal alloy, such as nickel-titanium alloy material. The coil construction or cut patterns of the proximal outer shaft 30 and/or the hypotube 38 can allow the proximal shaft 30 to follow the rail shaft or subassembly 22 in any desired direction. A cut pattern of the proximal outer shaft 30 and/or the hypotube 38 may be modified (e.g., cut per revolution, pitch, spine distance) to control tension resistance, compression resistance, flexibility, and torque resistance. For example, cuts per revolution may range between 1.5 and 5.5, pitch may range between 0.005" and 0.15", and spine distance may range between 0.015" and 0.125". The hypotube 38 may advantageously provide both tension and compression. The one or more outer liners or jackets 102 may allow the capsule subassembly 36 to be more flexible. The capsule hypotube 38 can bend in multiple directions. In some implementations, a distal terminus of the outer liner or jacket 102 may be positioned proximal of the distal terminus of the hypotube 38.

The capsule subassembly 36 may have a similar diameter as the outer proximal shaft 30 or a different diameter. In some examples, the capsule subassembly 36 has a uniform or substantially uniform diameter along its length. In some examples, the capsule subassembly 36 can be 28 French or less in size (e.g., 27 French). In some examples, the capsule subassembly 36 may include a larger diameter distal portion and a smaller diameter proximal portion. The capsule subassembly 36 or capsule can be configured to retain the implant (e.g., valve prosthesis) in the compressed position within the capsule subassembly 36 (e.g., within an implant retention area 106 marked in FIG. 5 occupying a distal-most 5 cm or inches of the capsule subassembly 36). Additional structural and operation details of a capsule subassembly, such as those described in connection with capsules in U.S. Publication No. 2019/0008640 and U.S. Publication No. 2019/0008639 may be incorporated into the capsule subassembly 36.

The outer sheath shaft or subassembly 20 is configured to be individually movable or slidable with respect to the other shafts or assemblies by operation of a control mechanism. The control mechanism may include an actuator in the form of a capsule knob 34 (marked in FIG. 24). The capsule knob 34 may be rotated to move or slide the outer sheath shaft or subassembly 20. Further, the outer sheath subassembly 20 can slide distally and proximally relative to the rail subassembly 22 together with the mid shaft subassembly 24, tether assembly 26, release assembly 28, and/or nose cone subassembly. The control mechanism may be configured for controlling deflection of a portion of the elongate catheter or delivery device 12 of the delivery system 10, including a deflectable portion of the elongate catheter or delivery device 12.

FIG. 5 schematically illustrates how at least a portion of a length of one or more components of the capsule subassembly 36 (e.g., inner liner 108) can include excess material such that the capsule subassembly 36 includes built-in slack along a portion of its length (e.g., a portion of the length proximal to the implant retention area 106) to facilitate flexible bending of the capsule subassembly 36 (e.g., to navigate tight turns within a heart or vasculature surrounding the heart).

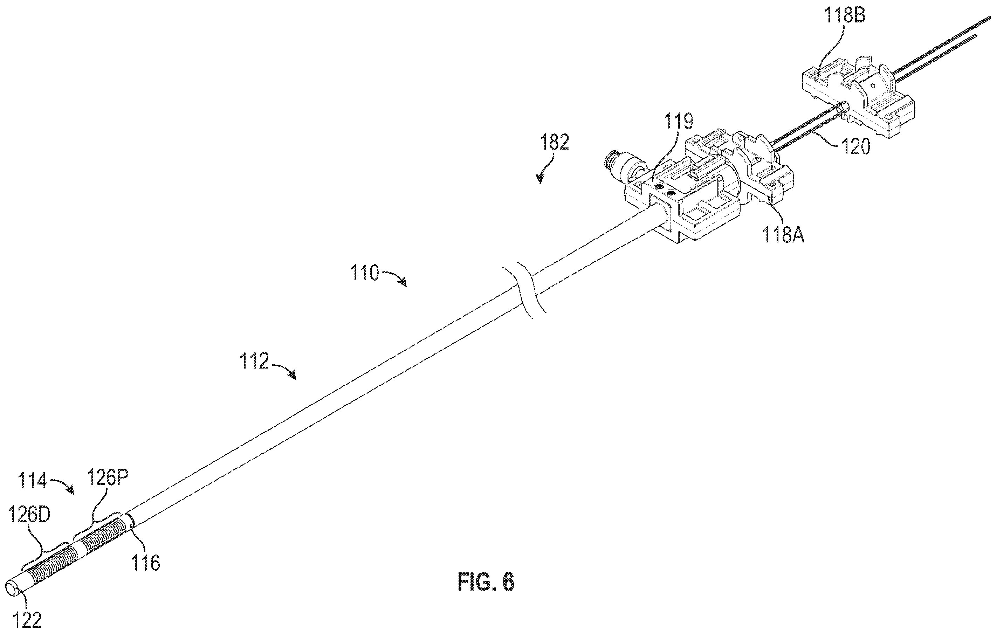

FIG. 6 shows a perspective view of a rail shaft or subassembly 22 or an elongate shaft of the elongate catheter or delivery device 12 of the delivery system 10 of FIG. 1. FIG. 6 shows approximately the same view as FIG. 2, but with the outer sheath subassembly 20 removed, thereby exposing the rail subassembly 22.

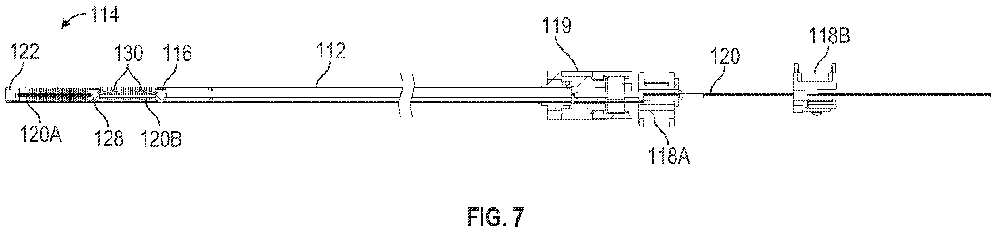

FIG. 7 further shows a cross-section of the proximal and distal end portions of the rail subassembly 22 to view the pull wires or pull tethers that facilitate steering of the rail subassembly 22. The rail subassembly 22 can include a rail shaft 110 (or rail) generally attached (and operably coupled) at its proximal end to the handle 16. The rail shaft 110 can be made up of a rail proximal shaft 112 directly attached to the handle 16 at a proximal end and a rail hypotube 114 attached to the distal end of the rail proximal shaft 112 (e.g., via a connector, ring-like structure, or insert 116). The rail subassembly 22 is operably coupled to the handle 16 via primary flex adaptor 118A at a proximal portion of the rail subassembly 22 (which controls medial-lateral trajectory of the distal end portion of the rail subassembly 22 via one or more distal pull tethers or wires 120A (marked in FIG. 7), via secondary flex adaptor 118B at a proximal portion of the rail subassembly 22 (which controls anterior-posterior trajectory of the distal end portion of the rail subassembly 22 via one or more proximal pull tethers or wires 120B), and via rail adaptor 119 at a proximal portion of the rail subassembly 22 (which includes a side needleless injection port to facilitate flushing and de-airing functions)). The rail proximal shaft 112 may include an interrupted spiral cut pattern along a large portion of its length to facilitate compression. The rail hypotube 114 can further include an atraumatic rail tip 122 at its distal tip. The atraumatic rail tip 122 may not comprise slits and is configured to extend up to 1 inch beyond the distal terminus of the rail hypotube 114 and is configured not to dig into the outer shaft subassembly 20 to avoid friction and fatigue and to prolong use. These components of the rail subassembly 22 can form a lumen for the other inner subassemblies to pass through.

FIG. 7 shows a side cross-section view of the rail shaft or subassembly 22 of FIG. 6. As shown in FIG. 7, attached to an inner surface of the rail hypotube 114 are one or more pull wires 120 which can be used apply forces to the rail hypotube 114 and steer the rail subassembly 22. The pull wires 120 can extend distally from the primary and secondary flex knobs 124A, B (illustrated in FIGS. 24 and 25) in the handle 16 to the rail hypotube 114. In some examples, pull wires 120 can be attached at different longitudinal locations on the rail hypotube 114, thus providing for multiple bending locations in the rail hypotube 114, allowing for multidimensional steering. For example, the rail hypotube 114 may provide a primary bend or flex along a medial/lateral trajectory and a secondary bend or flex along an anterior/posterior trajectory. Alternative directions of bend may be provided for deployment to a tricuspid valve. The rail hypotube 114 may form a bend portion for bending other of the shafts of the elongate shaft or shaft assembly 18.

The rail hypotube 114 may include a number of circumferential slots (e.g., laser cut into the hypotube) to facilitate bending and flexibility. The rail hypotube 114 can generally be broken into a number of different sections. At the most proximal end is an uncut (or unslotted) hypotube section corresponding to the location of insert 116. Moving distally, the next section is the proximal slotted hypotube section 126P. This section includes a number of circumferential slots cut into the rail hypotube 114. Generally, two slots are cut around each circumferential location forming almost half of the circumference. Accordingly, two backbones are formed between the slots extending up the length of the rail hypotube 114. This is the section that can be guided by the proximal pull wire(s) 120B. Moving further distally is the location where the proximal pull wires 120 connect, and thus slots can be avoided. This section is just distal of the proximally slotted section 126P and may correspond to the location of insert, or pull wire connector 128.

Distally following the proximal pull wire connection area is the distal slotted hypotube section 126D. This section is similar to the proximal slotted hypotube section 126P, but may have significantly more slots cut out in an equivalent length. Thus, the distal slotted hypotube section 126D may provide easier bending and an increased bend angle than the proximal slotted hypotube section 126P. In some examples, the proximal slotted section 126P can be configured to experience a bend of approximately 90 degrees with a half inch radius whereas the distal slotted section 126D can bend at approximately 180 degrees with a half inch radius. Further, as shown in FIGS. 6 and 7, the spines of the distally slotted hypotube section 126D are circumferentially offset from the spines of the proximally slotted hypotube section 126P. Accordingly, the two sections will achieve different bend patterns, allowing for three-dimensional steering of the rail subassembly 22. In some examples, the spines can be offset 30, 45, or 90 degrees, though the particular offset is not limiting. At the distal-most end of the distal slotted hypotube section 126D is the distal pull wire connection area which is again a non-slotted section of the rail hypotube 114.

In some examples, one distal pull wire 120A can extend to a distal section (e.g., to rail tip 122) of the rail hypotube 114 and two proximal pull wires 120B can extend to a proximal section of the rail hypotube 114; however, other numbers of pull wires can be used, and the particular amount of pull wires is not limiting. For example, two distal pull wires 120A can extend to a distal location and a single proximal pull wire 120B can extend to a proximal location. In some examples, ring-like structures or inserts attached inside the rail hypotube 114, known as pull wire connectors, can be used as attachment locations for the proximal pull wires 120B, such as insert 128. In some examples, the pull wires 120 can directly connect to an inner surface of the rail hypotube 114.

The distal pull wire(s) 120A can be connected (either on its own or through rail tip connector 122) generally at the distal end of the rail hypotube 114. The proximal pull wire(s) 120B can connect (either on their own or through the insert 128) at a location approximately one quarter, one third, or one half of the length up the rail hypotube 114 from the proximal end. In some examples, the distal pull wire(s) 120A can pass through a small diameter pull wire lumen (e.g., tube, hypotube, cylinder) attached on the inside of the rail hypotube 114. This can prevent the pull wires 120 from pulling on the rail hypotube 114 at a location proximal to the distal connection. Further, the lumen can comprise compression coils to strengthen the proximal portion of the rail hypotube 114 and prevent unwanted bending. Thus, in some examples the lumen is only located on a proximal portion (e.g., proximal half) of the rail hypotube 114. In some examples, multiple lumens, such as spaced longitudinally apart or adjacent, can be used per distal pull wire 120A. In some examples, a single lumen is used per distal wire 120A. In some examples, the lumen can extend into the distal portion (e.g., distal half) of the rail hypotube 114. In some examples, the lumen is attached on an outer surface of the rail hypotube 114. In some examples, the lumen is not used. In some examples, one or more compression coils 130 extend from the insert 116 to the insert 128. The compression coils 130 may be configured to bypass load in length between a distal primary flex point and a proximal secondary flex point. The compression coils 130 facilitate independent flex planes so that both planes of flex do not activate when one plane of flex is desired to flex. The compression coils 130 may allow for the proximally slotted hypotube section 126P to retain rigidity for specific bending of the distally slotted hypotube section 126D. The compression coils 130 may isolate force so only the primary flex is flexed.

For the pair of proximal pull wires 120B, the wires can be spaced approximately 180° from one another to allow for steering in both directions. Similarly, if a pair of distal pull wires 120A is used, the wires can be spaced approximately 180° from one another to allow for steering in both directions. In some examples, the pair of distal pull wires 120A and the pair of proximal pull wires 120B can be spaced approximately 90° from each other. Opposing wires could be used to provide anti-flex mechanism. In some examples, the pair of distal pull wires 120A and the pair of proximal pull wires 120B can be spaced approximately 0° from each other. However, other locations for the pull wires can be used as well, and the particular location of the pull wires is not limiting. In some examples, the distal pull wire 120A can pass through a lumen attached within the lumen of the rail hypotube 114. This can prevent an axial force on the distal pull wire 120A from creating a bend in a proximal section of the rail hypotube 114. The rail subassembly 22 is disposed so as to be slidable over the radially inner subassemblies. As the rail hypotube 114 is bent, it presses against the other subassemblies to bend them as well, and thus the other subassemblies of the delivery device 12 can be configured to steer along with the rail subassembly 22 as a cooperating single unit, thus providing for full steerability of the distal end of the delivery device 12. The rail hypotube 114 is adapted to bend in a first direction in a first plane (the plane of deflection of the distal slotted hypotube section 126D) and in a second direction in a second plane (the plane of deflection of the proximally slotted hypotube section 126P), with the second plane extending transverse or perpendicular relative to the first plane. Additional structural and operation details of a rail subassembly, such as those described in connection with rail assemblies in U.S. Publication No. 2019/0008640 and U.S. Publication No. 2019/0008639 may be incorporated into the rail subassembly 22.

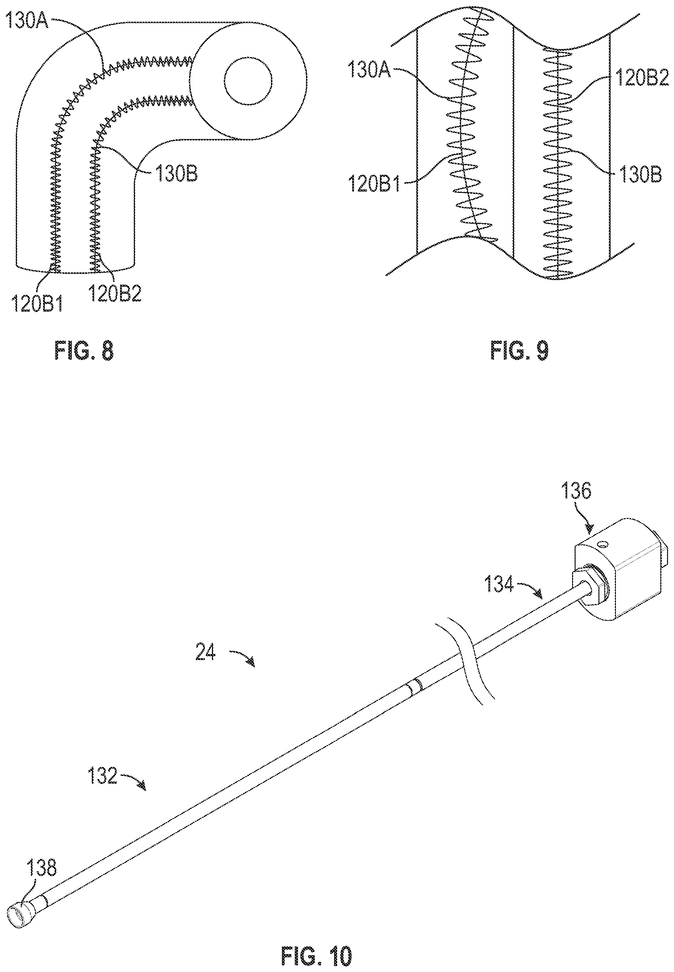

FIGS. 8 and 9 schematically illustrate how an outer compression coil 130A and proximal pull wire 120B1 can have a longer length than an inner compression coil 130B and proximal pull wire 120B2 of the rail subassembly 22 so that they don't occupy the same space and to facilitate ease of bending in one direction and reduce lumen obstruction during bending.

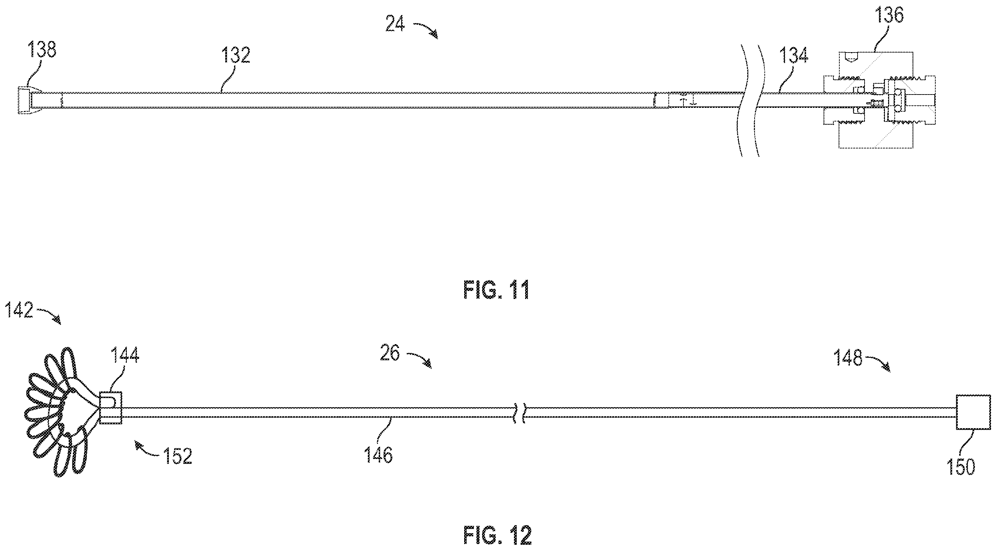

Moving radially inwardly, the next subassembly is the mid shaft or mid shaft subassembly 24. FIG. 10 shows a perspective view of the mid-shaft subassembly 24 of the delivery device 12 of the delivery system 10. FIG. 11 illustrates a side view. The mid-shaft subassembly 24 can include a distal mid-shaft hypotube 132 generally attached at its proximal end to a proximal shaft 134, which in turn can be attached at its proximal end to the handle 16 (e.g., via mid-shaft adaptor 136 at a proximal portion of the mid-shaft subassembly 24), and a distal pusher 138 located at the distal end of the mid-shaft hypotube 132. These components of the mid-shaft subassembly 24 can form a lumen for other inner subassemblies to pass through.

The mid-shaft subassembly 24 can be located within a lumen of the rail subassembly 22. The mid-shaft hypotube 132 can be formed of metallic alloy (e.g., cobalt chrome, nickel-chromium-cobalt alloy, nickel-cobalt base alloy, nickel-titanium alloy, stainless steel, and titanium). The mid-shaft hypotube 132 may comprise an interrupted spiral cut pattern. FIG. 10 shows a similar view as FIG. 6, but with the rail subassembly 22 removed, thereby exposing the mid-shaft subassembly 24.

Similar to the other subassemblies, the mid-shaft hypotube 132 and/or mid-shaft proximal tube 134 can comprise a tube, such as a hypodermic tube or hypotube (not shown). The tubes can be made from one of any number of different materials including Nitinol, stainless steel, and medical grade plastics. The tubes can be a single piece tube or multiple pieces connected together. Using a tube made of multiple pieces can allow the tube to provide different characteristics along different sections of the tube, such as rigidity and flexibility. The mid-shaft hypotube 132 can be a metal hypotube. The mid-shaft hypotube 132 can have a number of slots/apertures cut into the hypotube. In some examples, the cut pattern can be the same throughout. In some examples, the mid shaft hypotube 132 can have different sections having different cut patterns. The mid-shaft hypotube 132 can be covered or encapsulated with a layer of ePTFE, PTFE, or other material so that the outer surface of the mid-shaft hypotube 132 is generally smooth. At least a portion of a length of the mid-shaft proximal tube 134 may be covered with a heat shrink tubing or wrap.

The pusher 138 may be configured for radially retaining a portion of the implant (e.g., prosthesis) in a compacted configuration, such as a proximal end of the implant. The pusher 138 may compress an inlet end portion of the prosthetic heart valve. For example, the pusher 138 may be a ring or covering that is configured to radially cover the proximal end portion (e.g., suture eyelets portion) of the implant. The pusher 138 can also be considered to be part of the implant retention area 106, and may be at the proximal end of the implant retention area 106. The pusher 138 may comprise a frustoconical or cup shape that is riveted or fastened on its opposite sides to the distal end of the mid-shaft hypotube 132. The pusher 138 may be formed of PEEK material, ferrous material, platinum iridium, or other fluorogenic material to facilitate radiographic imaging. The mid-shaft subassembly 24 may be disposed so as to be fixed with respect to the handle. In some examples, the mid-shaft subassembly 24 may be individually slidable with respect to the other subassemblies. The mid-shaft adaptor 136 may operably couple to a depth knob 140 (shown in FIG. 24). The depth knob 140 may be utilized to effect ventricular/atrial movement of shafts of the elongate catheter within a heart. Additional structural and operational details of a mid-shaft subassembly 24, such as those described in connection with mid assemblies in U.S. Publication No. 2019/0008640 and U.S. Publication No. 2019/0008639 may be incorporated into the mid-shaft subassembly 24.

Referring to FIG. 12, a tether assembly 26 is utilized with the delivery system 10. The tether assembly 26 may comprise a portion of the elongate shaft 18 of the delivery device 12. In examples, the tether assembly 26 may extend within the mid-shaft subassembly 24, and may extend within the lumen of the mid-shaft hypotube 132 for example. The tether assembly 26 extends through the elongate shaft of the delivery device. The mid-shaft subassembly 24 may comprise a sheath extending over the tether assembly 26. Other sheaths of the delivery system 10 may comprise sheaths extending over the tether assembly 26. In examples, the tether assembly 26 may extend external of the mid-shaft subassembly 24 or external of any other portion of the delivery device 12 as desired.

The tether assembly 26 includes a plurality of coupling tethers 142 that each is configured to couple to an implant. The coupling tethers 142 may be positioned at a distal end portion of the tether assembly 26. The tether assembly 26 may include a tether manifold 144 for coupling to the plurality of coupling tethers 142. The tether assembly 26 may include a flexible retention tether 146 that may be coupled to the tether manifold 144 and may extend proximally from the tether manifold 144. The flexible retention tether 146 may extend proximally to a proximal end portion 148, which may couple to an adaptor 150 or other component for coupling to the handle 16.

The flexible retention tether 146 may have a variety of forms and may comprise a wire or a suture in examples. Other forms of flexible retention tethers 146 may be utilized. The use of a wire or suture may produce a flexibility for the retention tether 146 that may allow the flexible retention tether 146 to more easily deflect or flex with a deflection or flexure of the elongate shaft 18. Polymer extrusions may further be utilized. For example, a portion of the elongate shaft 18 such as the rail shaft or subassembly 22 may form a bend of the elongate shaft 18. The elongate shaft 18 accordingly may have a deflectable portion that is configured to deflect transverse to a longitudinal axis of the elongate shaft 18. The flexible retention tether 146 may be configured to deflect at the deflectable portion. The flexible retention tether 146 may have an increased flexibility and reduced stiffness relative to a tubular or hypotube shaft that may extend along the elongate shaft 18.

The flexible retention tether 146 may extend distally to a distal end portion 152 of the flexible retention tether 146. The flexible retention tether 146 may be configured to extend longitudinally along the elongate shaft 18 from the handle 16 (marked in FIG. 24) to the distal end of the elongate shaft 18. The flexible retention tether 146 extends proximally from the tether manifold 144 for engaging a tether actuator.

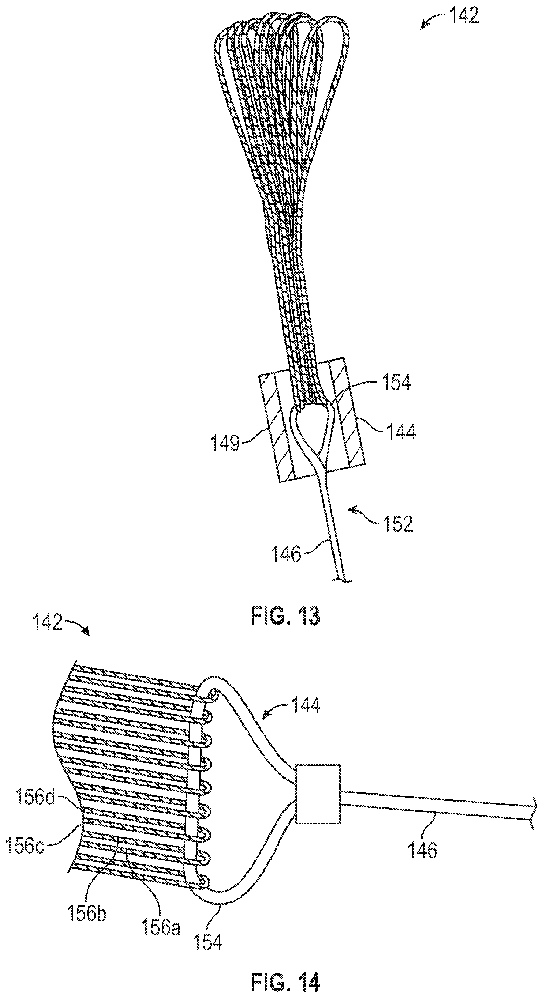

FIG. 13 illustrates a close-up view of the distal end portion 152 of the flexible retention tether 146. A cross-sectional view of the tether manifold 144 is shown.

The tether manifold 144 is configured to collect the plurality of coupling tethers 142. The tether manifold 144 may couple the plurality of coupling tethers 142 to the distal end portion 152 of the flexible retention tether 146. As shown in FIG. 13, the tether manifold 144 may comprise a loop of material of the flexible retention tether 146 and a sheath 149 extending over the loop of material. The tether manifold 144 may comprise a loop of the material comprising a wire of the flexible retention tether 146 or may have another configuration in examples. For example, the tether manifold 144 may comprise a loop of a suture material comprising the flexible retention tether 146.

The sheath 149 may comprise a tube that may retain the plurality of coupling tethers 142 to the loop of the manifold 144. The tube may comprise a shrink tube or other form of tube that may be placed upon the loop of the manifold. The sheath 149 may be flexible. As such, a flexible and conformable configuration for the manifold 144 may result. In examples, a material may be melted or reflowed at the connection of the coupling tethers 142 to the manifold. The material may comprise a polymer material that may be melted or reflowed (e.g., PEBAX, HDPE, LDPE, etc.). In examples, a combination of such a material and a sheath may be utilized. In examples, the length of the sheath 149 may be extended to improve the ability of the tether assembly 26 to be pushed through a lumen of a sheath (e.g., a lumen of the mid-shaft). The tether manifold may have other configurations in examples.

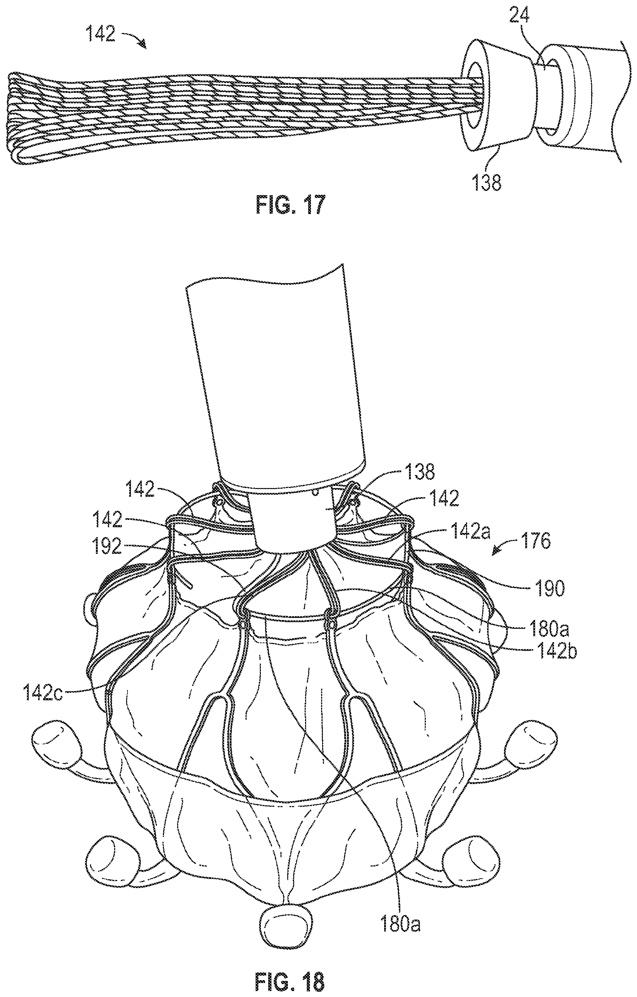

The plurality of coupling tethers 142 may extend from the tether manifold 144. The plurality of coupling tethers 142 may be configured to couple to an implant in a variety of manners. For example, the plurality of coupling tethers 142 is each configured to pass through an opening in a portion of an implant. FIG. 18, for example, illustrates loop portions of the plurality of coupling tethers 142 passing through respective eyelets of an implant. The plurality of coupling tethers 142 may couple to the implant in other manners as desired.

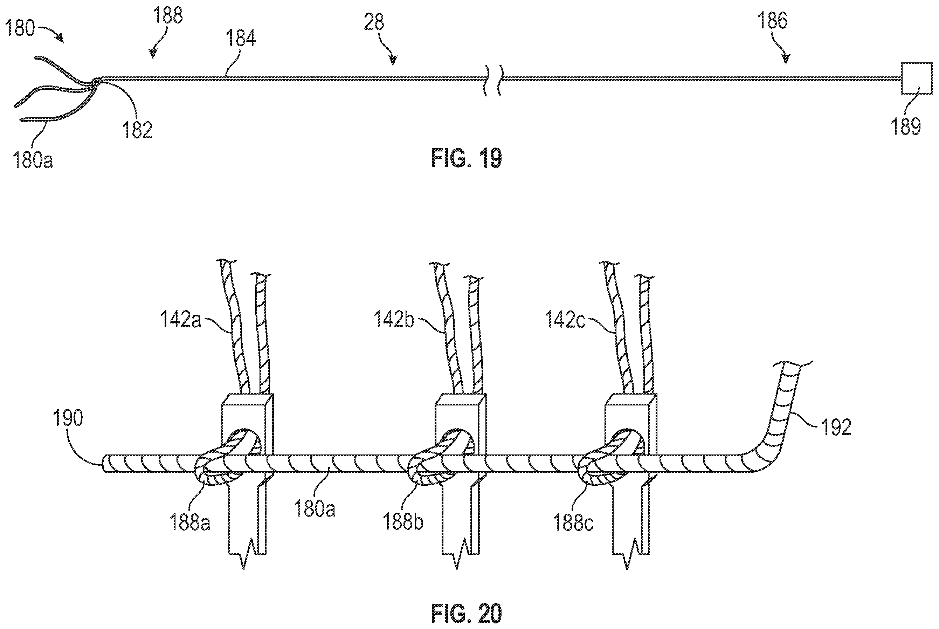

In examples, the plurality of coupling tethers 142 may be flexible. The plurality of coupling tethers 142 may each comprise a suture or other form of flexible material. The plurality of coupling tethers 142 each comprises a loop, with the loop configured to pass through an opening in a portion of an implant. FIG. 20, for example, illustrates loops extending through eyelets of an implant.

The plurality of coupling tethers 142 may comprise a continuous suture in examples. For example, referring to FIG. 14, the plurality of coupling tethers 142 may comprise loops formed by a continuous suture being looped multiple times around an arm 154 of the tether manifold 144, with the arm 154 forming a loop. A first length 156a of a tether 142, for example, may be looped around the arm 154 to produce a second length 156b of the tether 142. The second length 156b may be looped at its distal end to form a distal loop and produce a third length 156c of the tether 142. The third length 156c may be looped around the arm 154 to produce a fourth length 156d. Lengths may be iteratively looped to produce a desired number of distal loops for coupling to the implant. The plurality of coupling tethers 142 may comprise a continuous suture length. In examples, a sheath 149 may be positioned over the proximal loops of the coupling tethers 142 to secure the proximal loops to the arm 154. The arm 154 may comprise a wire in examples. Other configurations may be utilized in examples.

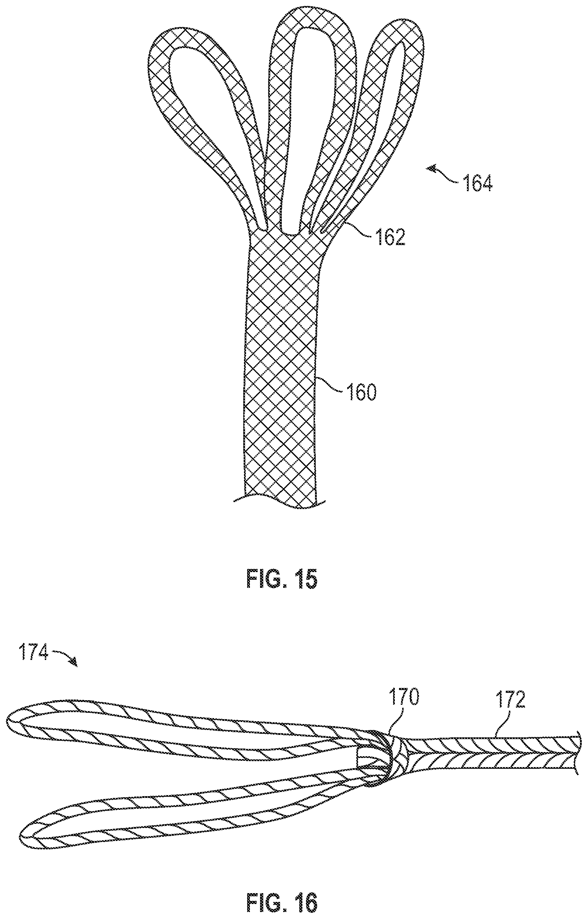

For example, FIG. 15 illustrates an example in which a flexible retention member or tether 160 is woven or braided. The flexible retention tether 160 may comprise a plurality of sutures woven or braided into a larger suture. The tether manifold 162 may comprise a separation of the plurality of coupling tethers 164 from the flexible retention tether 160. The plurality of coupling tethers 164 may be woven or braided out from the flexible retention tether 160 and may comprise sub-portions of the weave or braid of the flexible retention tether 160. The plurality of coupling tethers 164 may comprise loops that are woven or braided with the tether manifold 162. The braid may comprise a cylindrical braid or flat braid or may have another configuration in examples.

FIG. 16 illustrates an example in which a tether manifold 170 comprises a knot coupling the flexible retention tether 172 to the plurality of coupling tethers 174. The tether assemblies of FIGS. 15 and 16 may be entirely suture-based, and thus may maintain flexibility along the length of the tether assembly. In examples, a combination of a polymer or sleeve or wire may be provided to improve the ability of a tether assembly 26 to be pushed through the lumen of a sleeve.

The plurality of coupling tethers may be positioned at a distal end portion of the elongate shaft 18 for coupling to the implant. FIG. 17, for example, illustrates the plurality of coupling tethers 142 extending from the lumen of the mid shaft 24 and particularly the lumen of the pusher 138 of the mid shaft 24. The plurality of coupling tethers 142 may be positioned to couple to the implant.

FIG. 18, for example, illustrates a coupled configuration of the plurality of coupling tethers 142 to the implant 176. The coupling tethers 142 may extend through the eyelets of the implant 176 to couple to the implant. The coupling tethers 142 may extend radially outward from the lumen of the mid shaft.

In examples, a plurality of tether assemblies 26 may be utilized. For example, each tether assembly may utilize one or more of the coupling tethers 142. Each of the tether assemblies 26 may be controlled independently for selective control of implant expansion. For example, a first portion of the implant may be expanded prior to a second portion, or may be retracted to control the position of anchors. Controlled placement of the anchors may result. A plurality of tether assemblies 26 may have other beneficial results.

A release assembly 28 is utilized for releasing the plurality of coupling tethers 142 from the implant 176. FIG. 19, for example, illustrates a side view of a release assembly 28. The release assembly 28 may include one or more release tethers 180. The release tethers 180 may couple to a release tether manifold 182 in examples. The release tether manifold 182 may couple to a retractable tether 184 in examples. The release tethers 180 may comprise flexible tethers in examples.

The retractable tether 184 may extend proximally to a proximal end portion 186. The proximal end portion 186 may couple to an adaptor 189 in examples. The adaptor 189 may be configured to engage with a release actuator 191 (marked in FIG. 25) or other component of the delivery system in examples. The retractable tether 184 may be flexible and may comprise a wire or suture in examples. The retractable tether 184 may comprise a filament that may be configured to extend along the elongate shaft 18 of the delivery device 12. In examples, the retractable tether 184 may extend within the mid-shaft subassembly 24 and may extend within the lumen of the mid-shaft hypotube 132 for example. The mid-shaft subassembly 24 for example, may comprise a sheath extending over at least a portion of the release assembly. Other sheaths may be utilized as desired. In examples, the retractable tether 184 may extend external of the mid-shaft subassembly 24 or external of any other portion of the delivery device 12 as desired.

The release assembly 28 may be configured to be flexible and deflectable with the elongate shaft 18 of the delivery device 12. For example, at least a portion of the release assembly 28 may be configured to deflect at a deflectable portion of the elongate shaft 18.

The retractable tether 184 may extend to a distal end portion 188. The release tether manifold 182 may be positioned at the distal end portion 188. The release tether manifold 182 may comprise a collection of the release tethers 180.

In examples, a use of the manifold 182 may be excluded and one or more of the release tethers 180 may extend along the length of the elongate shaft 18. As such, the release assembly may comprise one or more of the release tethers 180 in examples, which may be utilized for releasing the plurality of coupling tethers 142 from the implant 176. In examples, only one release tether 180 may be utilized (with the single release tether passing through a plurality of the loop portions).

One or more of the release tethers 180 is utilized to extend through one or more of the loop portions of the coupling tethers 142 to secure the implant to the loop portions. FIG. 20, for example, illustrates one of the release tethers 180a extending through respective loop portions 188a, b, c of respective coupling tethers 142a, b, c. The passage of the release tether 180a through the loop portions 188a, b, c, may retain the implant to the loop portions 188a, b, c, due to the loop portions 188a, b, c extending through the eyelets of the implant. In examples, a single release tether 180a may pass through multiple loop portions. In examples, a single release tether 180a may pass through a single loop portion (e.g., a one-to-one correspondence between release tethers and loop portions).

The release tethers 180 may have a variety of forms and may comprise a suture, a cable, a wire, monofilament, a tape, or an extrusion. Other forms of release tethers 180 may be utilized in examples.

In examples, a single release tether may be utilized to couple to the loop portions of the coupling tethers. In examples, multiple release tethers may be utilized. For example, FIG. 18 illustrates a configuration in which three release tethers are utilized, each configured to extend through three loop portions. One or more release tethers may be configured to extend circumferentially between a plurality of the loop portions of the coupling tethers. A greater or lesser number of release tethers may be utilized as desired. In examples, a sheath or material for melting or reflow may be utilized at the release tether manifold 182. In examples, a knot, or weaving, or braiding with the release tethers may be utilized. Other forms of connection at the release tether manifold 182 may be utilized.

Referring to FIG. 20, an end 190 of a release tether may comprise a free end that may be retracted from the loop portions 188a, b, c, to release the implant from the loop portions 188a, b, c. A retraction portion 192 or proximal end portion of a release tether may extend to the retractable tether 184, for being retracted from the loop portions 188a, b, c. The retraction portion 192 may extend radially inward to a retention sheath of the release assembly, which may comprise the mid shaft 24, or another sheath in examples.

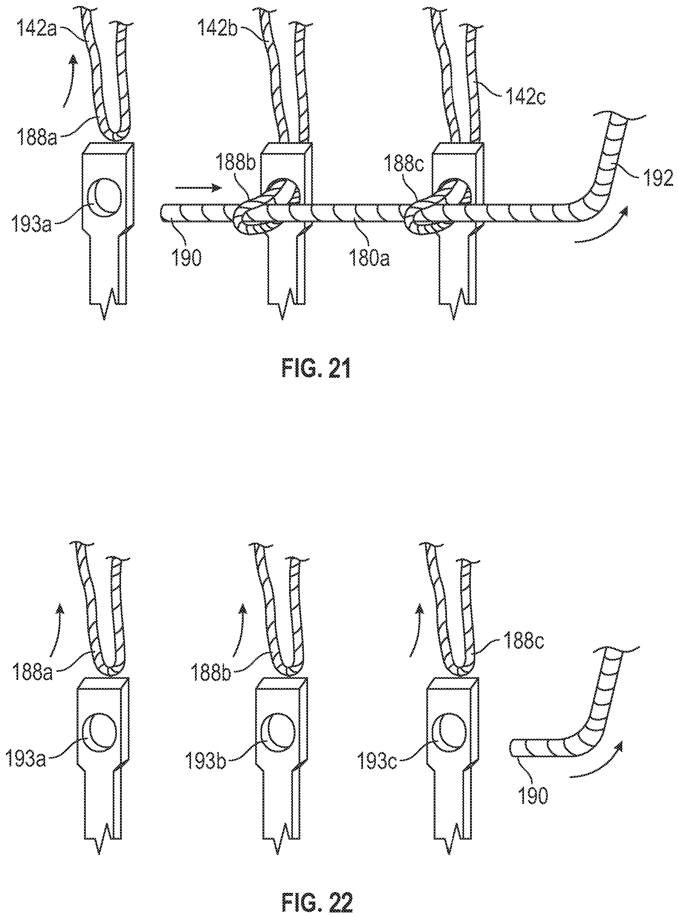

FIGS. 21 and 22, for example, illustrate a retraction sequence, in which the release tether 180a may be retracted from the loop portions 188a, b, c. FIG. 21, for example, illustrates the retraction portion 192 having been retracted, with the end 190 withdrawn from the loop portion 188a. The loop portion 188a accordingly may release from the eyelet 193a. The retraction portion 192 may continue to be retracted to release the remaining loop portions 188b, c from the respective eyelets 193b, c. Other release tethers may be released from respective loop portions in a similar manner.

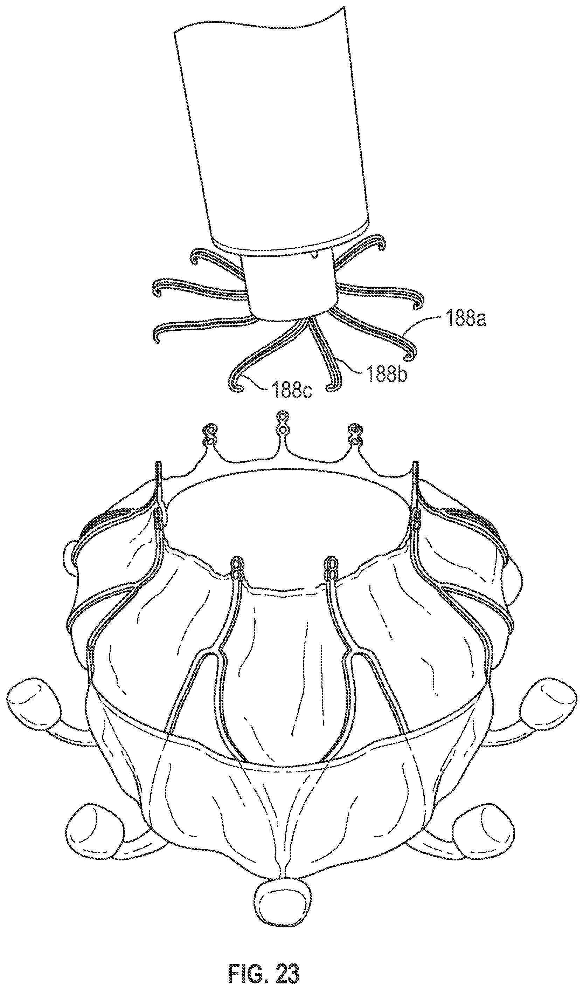

FIG. 23, for example, illustrates the loop portions of the coupling tethers 142 released from the implant. The implant may be in an implanted or deployed configuration, positioned at an implantation site such as a native heart valve.

The routing of the coupling tethers may be varied in examples. For example, a coupling tether may pass through multiple eyelets of an implant (e.g., first through an eyelet in an outer valve frame, and then through an eyelet in an inner frame). A release tether may lock the coupling tether at its most distal connection. When the release tether is retracted, the coupling tether may be free to pull through and decouple from both valve eyelets. Other forms of routing may be utilized.

The release assembly 28 may be utilized to retain the implant to the tether assembly 26 during a recapture process of the implant. For example, the tether assembly 26 may retract the implant into the mid shaft 24 if recapture is desired. The release assembly 28 may retain the implant to the tether assembly 26 during such a process. The release assembly 28 may be actuated at a desired time of final release of the implant at an implantation site.

FIG. 24 shows a perspective view of the housing or handle 16 of the delivery device 12. FIG. 25 shows a side cross-section view of the handle 16. The handle 16 includes a control mechanism for moving the one or more shafts of the elongate catheter. The control mechanism may include multiple actuators or actuator assemblies, such as rotatable knobs, that can manipulate different components (e.g., cause movement of respective shafts or subassemblies of the shaft assembly 18) of the delivery system 10. The distal end of the handle 16 includes an actuator in the form of a capsule knob 34. Rotation of the capsule knob 34 in one direction may cause proximal movement of the outer sheath subassembly 20 in an axial direction so as to unsheathe and deploy a distal portion (e.g., ventricular portion) of the implant from the capsule subassembly 36. The capsule knob 34, for example, may comprise a retraction mechanism for retracting the capsule or capsule subassembly 36 to release the implant from the capsule. Rotation of the capsule knob 34 in the opposite direction causes distal movement of the outer sheath subassembly 20 (including the capsule subassembly 36) so as to recapture, retrieve, or resheath, the implant within the capsule subassembly 36. The outer sheath subassembly 20 may be individually translated with respect to the other subassemblies in the delivery device 12. The distal end of the implant can be released first, while the proximal end of the implant can remain radially compressed within the pusher 138 of the mid-shaft subassembly 24.

Moving proximally, the handle 16 includes a stabilizer mounting area 200 adapted to interface with a clamp of a stabilizer assembly configured to control the medial / lateral position of the delivery device 12. Moving further proximally are the actuators in the form of primary flex rail knob 124A and the secondary flex rail knob 124B. Rotation of the primary flex rail knob 124A causes flexing of the primary flex portion, or distal slotted hypotube section 126D of the rail hypotube 114 to effect changes in medial/lateral trajectory. Rotation of the secondary flex rail knob 124B causes flexing of the primary flex portion, or proximal slotted hypotube section 126P of the rail hypotube 114 to effect changes in anterior/posterior trajectory. However, the number of flex rail knobs 124A, B can vary depending on the number of pull wires used.

Proximal to the secondary flex rail knob 124B is the depth knob 140 that controls movement of the outer sheath subassembly 20, the mid-shaft subassembly 24, the tether assembly 26, the release subassembly 28 and the nose cone shaft or subassembly relative to the rail subassembly 22. The depth knob 140 may also move the other subassemblies together as well as relative to the rail subassembly 22 in some configurations.

Further proximal is the actuator in the form of the release actuator 191 or release knob. The release actuator 191 may be rotated proximally to put tension on the release assembly during a deployment procedure for the implant. The retraction of the release assembly may release the implant from the tether assembly.

The proximal-most knob is the nose cone knob 202, rotation of which causes proximal and distal movement of the nose cone subassembly. The nose cone subassembly is the most radially inward subassembly and may include a nose cone shaft having a distal end connected to a nose cone.

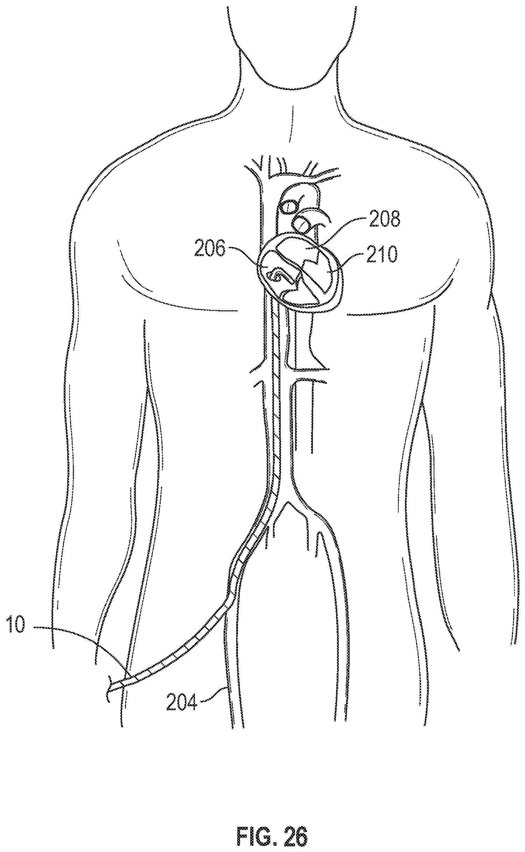

FIG. 26 illustrates a schematic representation of a delivery approach to a native tricuspid valve. As shown in FIG. 26, in one example the delivery system 10 can be placed in the ipsilateral femoral vein 204 and advanced toward the right atrium 206. The approach can be from the inferior vena cava (or from the superior vena cava) in examples.

FIG. 26 shows the delivery system 10 extending from the ipsilateral femoral vein 204 to the right atrium 206. In examples of the disclosure, a guide wire is not necessary to position the delivery system 10 in the proper position, although in other examples, one or more guide wires may be used.

Accordingly, it can be advantageous for a user to be able to steer the delivery system 10 through the complex areas of the heart in order to position a replacement tricuspid valve in line with the native tricuspid valve. This task can be performed with or without the use of a guide wire with the above disclosed system. The distal end of the delivery system 10 can be advanced into the right atrium 206. A user can then manipulate the rail subassembly 22 to target the distal end of the delivery system 10 to the appropriate area. Further, a user can torque the entire delivery system 10 to further manipulate and control the position of the delivery system 10. In the fully bent configuration, a user can then place the replacement valve in the proper location. This can advantageously allow delivery of a replacement valve to an in-situ implantation site, such as a native tricuspid valve.

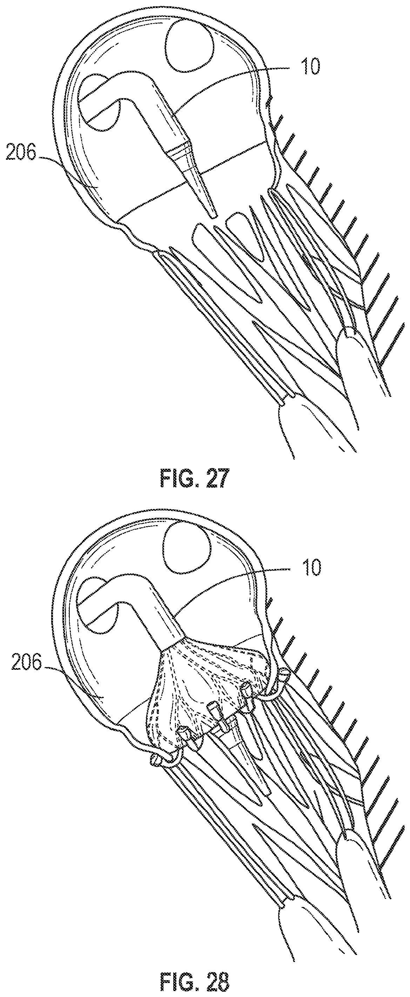

FIG. 27 illustrates a schematic representation of a distal end of the delivery system 10 approaching a native tricuspid valve. The distal end of the delivery system 10 may be positioned as desired relative to the implantation site prior to release of an implant from the implant retention area.

FIG. 28 illustrates that the implant may be released from the delivery system 10 with the tether assembly 26 coupled to the implant. The position of anchors relative to the native valve leaflets may be determined, and if in proper position the implantation procedure may proceed.

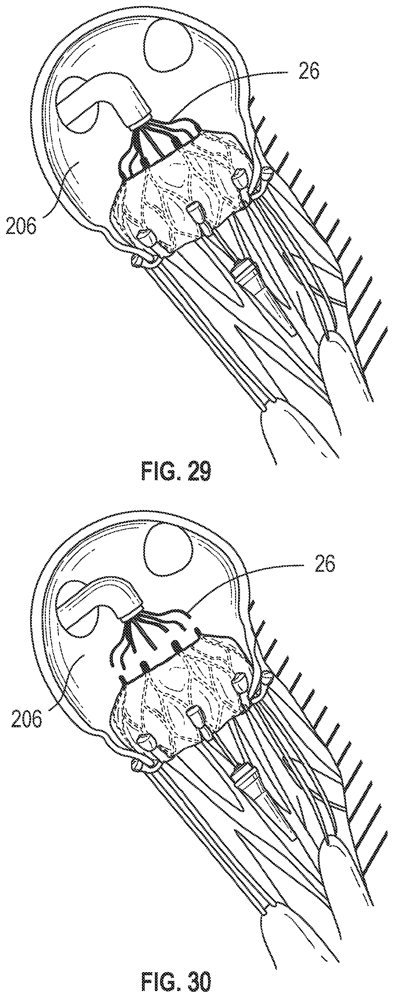

FIG. 29 illustrates the implant in an expanded configuration, with the tether assembly 26 coupled to the implant. The release assembly 28 may retain the tether assembly 26 to the implant in such a configuration.

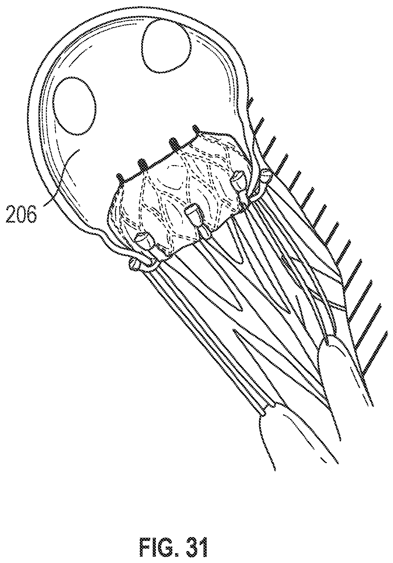

The tether assembly 26 may be released from the implant utilizing the release assembly 28 for example. FIG. 30 illustrates release of the tether assembly 26. FIG. 31 illustrates the implant deployed in position.

A similar deployment procedure may be utilized with a mitral valve as desired. For example, a transseptal puncture can be performed from the right atrium 206 (marked in FIG. 26) to obtain access to the left atrium 208. A user can pass the bent delivery system 10 through the transseptal puncture and into the left atrium 208. A user can then further manipulate the delivery system 10 to create an even greater bend in the rail subassembly 22. The delivery system 10 can then be advanced into the left atrium 208 and then towards the left ventricle 210. The implant may be deployed in a similar manner as shown in FIGS. 27-31.

In examples, a release assembly may be provided in the form of a disintegration assembly 212. The disintegration assembly 212 may be configured to connect to a portion 213 of one or more coupling tethers 215 and disintegrate the connection to the portion 213 to release the implant from the elongate shaft of the delivery system.

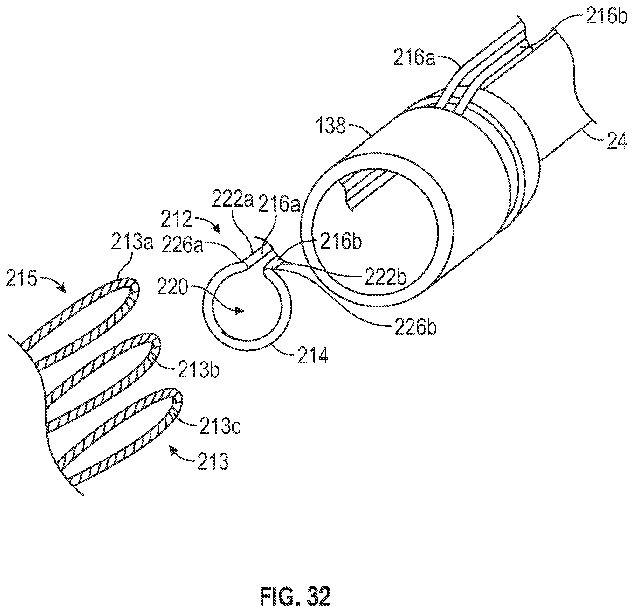

The disintegration assembly 212 as shown in FIG. 32 may comprise a heating element 214 in examples. The heating element 214 may be configured to disintegrate the connection to the portion 213 of the one or more coupling tethers 215. The heating element 214, for example, may be configured to heat to melt, break, vaporize, erode, dissolve, or otherwise disintegrate the connection to the portion 213 of the one or more coupling tethers 215. In examples, one or more electrical conduits 216a, b may be provided that may pass electrical energy through the heating element 214 to activate the heating element 214. The disintegration assembly 212 may have other forms in examples.

The heating element 214 may be configured as a ring as shown in FIG. 32, or may have other configurations in examples (e.g., a strip of material, a coil, one or more pins, one or more hooks, one or more terminals, among others). The heating element 214 may comprise a heating filament configured to become hot upon electrical energy (e.g., electrical current) passing through the heating element 214 or may have another configuration in examples. The heating element 214 may be made of a material such as Nichrome (i.e., alloy of nickel and chromium), stainless steel, tungsten, an alloy of platinum or tungsten, an alloy of nickel-iron-chromium, or ceramic materials (e.g., molybdenum disilicide), among other forms of materials. Other forms of heating elements (e.g., semiconductor or polymer heating elements) may be utilized in examples. A heating element may produce heat utilizing other methods in examples.

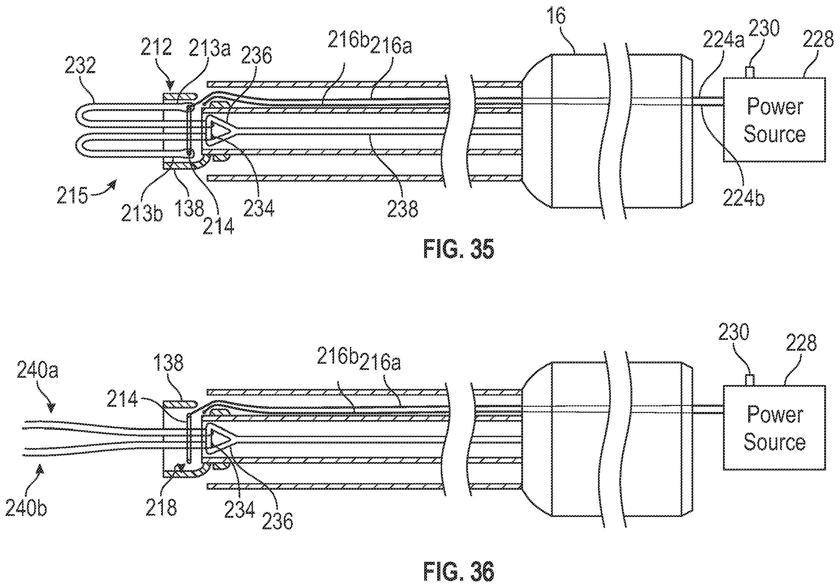

In examples, the heating element 214 may be configured to be positioned within a portion of one or more shafts of the elongate shaft or shaft assembly 18. For example, as represented in an exploded view of FIG. 32, the heating element 214 may be positioned within a lumen of the mid shaft or mid shaft subassembly 24 (and correspondingly within a lumen of the outer sheath shaft or subassembly 20). The heating element 214 particularly may be positioned within the distal pusher 138 of the mid shaft or mid shaft subassembly 24. For example, the heating element 214 may be positioned interior of an inner surface 218 (marked in FIG. 36) of the distal pusher 138. The heating element 214 may be positioned at a proximal end portion of the distal pusher 138, or at another position as desired (e.g., a distal end portion). The heating element 214 may comprise a ring extending circumferentially interior of the inner surface 218 of the distal pusher 138. The ring shape may provide a central opening 220 (marked in FIG. 32) for other components of the system to pass through (e.g., other sheaths or shafts or assemblies). Other configurations may be utilized in examples.

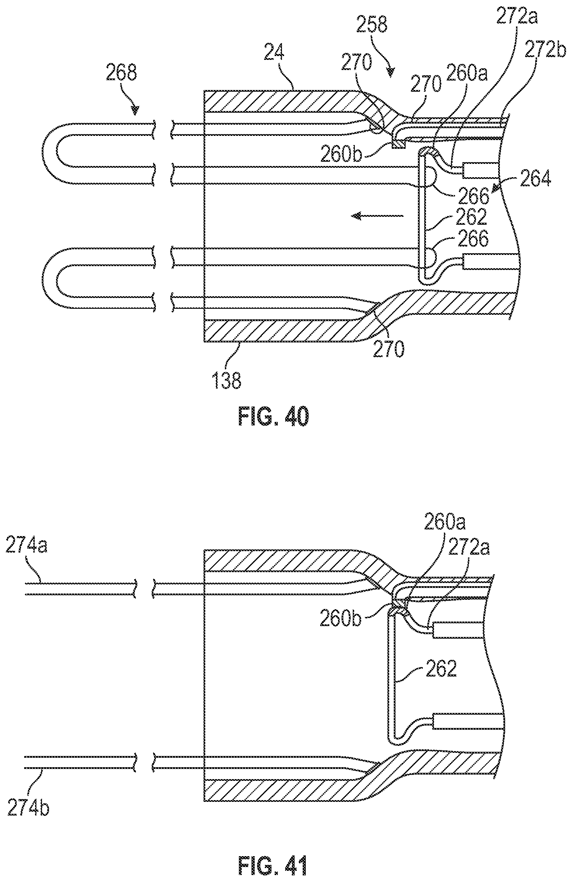

The one or more electrical conduits 216a, b may extend along a length of the elongate catheter or delivery device 12. The one or more electrical conduits 216a, b may each include a respective distal end portion 222a, b and a proximal end portion 224a, b (marked in FIG. 35). The distal end portion 222a, b may couple to the heating element 214 at a respective juncture 226a, b (marked in FIG. 32). The proximal end portion 224a, b may couple to a power source 228 (marked in FIG. 35). The one or more electrical conduits 216a, b may extend along a length of the elongate shaft or shaft assembly 18, and particularly along the length of the shaft or subassembly that includes the heating element 214. For example, the one or more electrical conduits 216a, b may extend along the length of the mid shaft or mid shaft subassembly 24. The one or more electrical conduits 216a, b may extend exterior of the mid shaft or mid shaft subassembly 24 (as represented in FIGS. 32, 35, and 36), or interior of the mid shaft or mid shaft subassembly 24 (e.g., within the lumen of the mid shaft or mid shaft subassembly 24) or may be fully or partially embedded in a wall of the mid shaft or mid shaft subassembly 24 (as represented in FIG. 40). The one or more electrical conduits 216a, b may extend in a similar manner along any other subassembly or shaft of the elongate shaft or shaft assembly 18.

In examples, the distal end portions 222a, b of the one or more electrical conduits 216a, b may pass through a portion of the distal end of the mid shaft or mid shaft subassembly 24 to connect to the heating element 214 (as shown in the exploded view of FIG. 32). The one or more electrical conduits 216a, b may pass from an exterior of the mid shaft or mid shaft subassembly 24 to an interior of the mid shaft or mid shaft subassembly 24 (e.g., the distal pusher 138) in examples.

The one or more electrical conduits 216a, b may be configured to be insulative or otherwise not transmit heat to the surrounding system or environment. For example, the one or more electrical conduits 216a, b may be insulated or may be covered with an insulative material (e.g., a coating) to reduce the possibility of heat transmitting to the surrounding system or environment. In examples, the one or more electrical conduits 216a, b may be made of a material that remains at substantially the same temperature upon electrical energy (e.g., electrical current) passing through the one or more electrical conduits 216a, b. The juncture 226a, b, for example, may comprise a transition of material from a heating material of the heating element 214 to a non-heating material of the one or more electrical conduits 216a, b. Other configurations may be utilized in examples.

The proximal end portions 224a, b of the one or more electrical conduits 216a, b may be configured to couple to a power source 228 (marked in FIG. 35) to provide electrical energy through the one or more electrical conduits 216a, b to the heating element 214. The power source 228 may be configured to provide electrical power to the heating element 214. The power source 228 may have any form as desired (e.g., battery, power connector, capacitor, among others). The power source 228 may be positioned on the handle 16 or may have another position as desired (e.g., a power connector may comprise a mains connector that connects to a power outlet, which may be a wall outlet or other form of outlet). An actuator 230 (marked in FIG. 35) may be utilized to selectively control transmission of electrical power through the one or more electrical conduits 216a, b.

Referring to FIG. 32, the heating element 214 of the disintegration assembly 212 may be configured to connect to the portions 213 of the one or more coupling tethers 215. The heating element 214, for example, may be configured as a ring, to allow the portions 213 to comprise loop portions 213a, b, c for extending over the heating element 214. The loop portions 213a, b, c may comprise the apices of the coupling tethers 215. The loop portions 213a, b, c may comprise end portions of the one or more coupling tethers 215 that may loop over the filament comprising the ring. In examples, the loop portions 213a, b, c may correspond to the loop portions 188a, b, c shown in FIG. 20. The loop portions 213a, b, c shown in FIG. 32 (which may be referred to as a second portion of coupling tethers 215), however, may be directed proximally towards the heating element 214 and may loop over the heating element 214. A mid portion of the coupling tethers 215 (which may be referred to as a first portion of the coupling tethers 215) may form a loop portion 232 (marked in FIG. 35) that may couple to the implant to retain the implant to the elongate shaft 18. The loop portion 232, for example, may protrude through a respective opening (e.g., an eyelet) in a portion of the implant 176 to couple to the implant as discussed with respect to FIG. 21.

The coupling tethers 215 may include an end portion 234 that may couple to a tether manifold or other coupling point for the tethers 215. The tether manifold 236 (marked in FIG. 35), for example, may be configured similarly as the tether manifold 144 represented in FIG. 13 or may have another configuration in examples. The end portion 234 may loop around an arm of the tether manifold 236 or may have another configuration in examples. The tether manifold 236 may couple to a flexible retention tether 238 that may be configured similarly as the flexible retention tether 146 shown in FIG. 13 or may have another configuration as desired. Other configurations of tether assemblies or subassemblies may be utilized as desired, including other configurations as disclosed herein.

In examples, the use of a tether manifold and/or flexible retention tether may be excluded. For example, a first portion of the tethers 215 may comprise an end portion that couples to the implant. A second portion of the tethers 215 may comprise an opposite end portion that couples to the heating element 214.

In examples, a coupling tether may have a first end that couples to the heating element on the mid shaft or mid shaft subassembly 24, and a mid-portion that forms a loop for coupling to the implant. The second end of the coupling tether may couple back to the mid shaft or mid shaft subassembly 24. The heating element may heat to release the first end of the coupling tether. The second end of the coupling tether may be retained by the mid shaft or mid shaft subassembly 24. As such, the use of the tether manifold and/or flexible retention tether may be excluded.

In examples, a coupling tether may have a first end that couples to the heating element, and a mid-portion that forms a loop for coupling to the implant. The heating element may be positioned on the tether manifold and/or flexible retention tether. The second end of the coupling tether may couple back to the tether manifold and/or flexible retention tether. The heating element may heat to release the first end of the coupling tether. The second end of the coupling tether may be retained by the tether manifold and/or flexible retention tether.

Other configurations and positions of coupling may be utilized in examples.

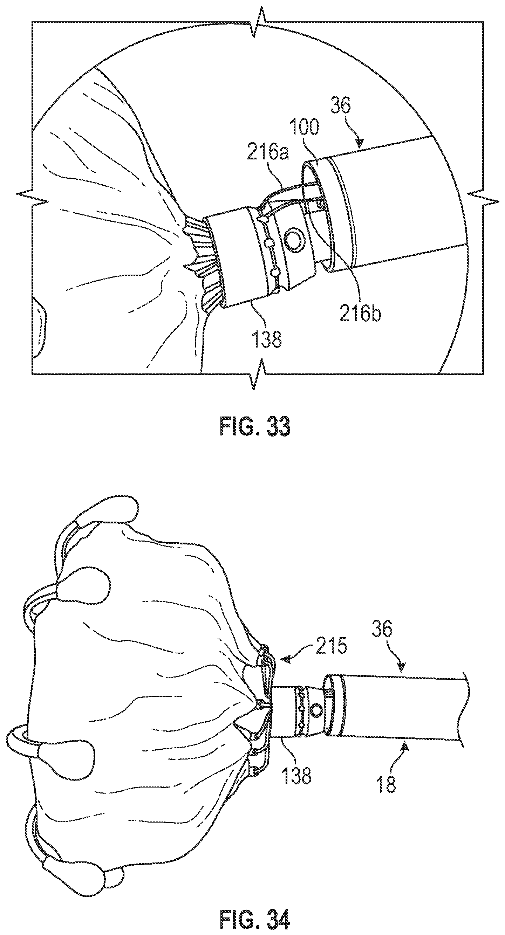

In a deployment procedure, the implant may be retained by the elongate catheter or delivery device 12 in a similar manner as disclosed herein. The outer sheath shaft or subassembly 20, for example, may extend over the implant and retain the implant in a compressed configuration. The outer sheath shaft or subassembly 20 (e.g., the capsule subassembly 36) may be retracted proximally to allow the implant to expand radially outward in a similar manner as disclosed herein. FIG. 33, for example, illustrates a retraction of the capsule subassembly 36 to allow the implant to expand radially outward. The proximal end of the implant is shown to be retained by the distal pusher 138.

The tether assembly or subassembly may be advanced distally to allow the implant to release from the distal pusher 138 when desired. The tether assembly or subassembly may be advanced to allow the coupling tethers 215 to expand radially outward and thereby allow the implant to expand more fully. FIG. 34, for example, illustrates the proximal end of the implant released from the distal pusher 138. The end portions 234 of the coupling tethers 215 have advanced distally with the flexible retention tether 238, relative to the mid shaft or mid shaft subassembly 24, to allow the coupling tethers 215 to expand radially outward.

At a desired time, the disintegration assembly 212 may be activated to release the implant from the elongate shaft or shaft assembly 18. Referring to FIG. 35, for example, a cross-sectional view of the disintegration assembly 212 prior to disintegration of the connection to the coupling tethers 215 is shown. The loop portions 232 of the coupling tethers 215 may extend outward from the distal pusher 138, yet with the end portions 213a, b remaining connected to the heating element 214. The opposite end portions 234 may remain connected to the tether manifold 236.

The actuator 230 may be activated at a desired time of release. FIG. 36, for example, illustrates a configuration in which the actuator 230 has been activated. Electrical energy may pass through the electrical conduits 216a, b and may activate the heating element 214. The heating element 214 may be heated and the heat may disintegrate the loop portions 213a, b to disintegrate the connection to the loop portions 213a, b. The heating element 214, for example, may melt or otherwise disintegrate the loop portions 213a, b. The loop portions 213a, b may be severed. The loop portion 213a, b accordingly may comprise free ends 240a, b (marked in FIG. 36) that are uncoupled to the heating element 214. The end portions 234 may remain connected to the tether manifold 236.

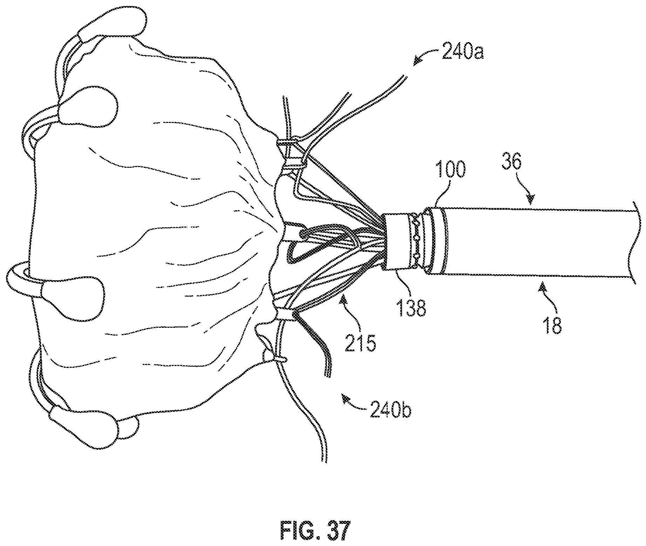

FIG. 37, for example, illustrates a side view of a resulting configuration of the coupling tethers 215. The free ends 240a, b (with additional free ends of respective tethers 215 shown in FIG. 37) of the coupling tethers 215 may be able to be pulled through the eyelets of the implant to fully release the implant from the elongate shaft 18. The end portions 234 (marked in FIG. 36) may remain connected to the tether manifold 236 and may be withdrawn with the tether manifold 236 upon retraction of the elongate shaft 18 from the implantation site. The end portions 234 may be pulled proximally with the tether manifold 236 to cause the coupling tethers 215 to be pulled through the eyelets of the implant.

In examples, other forms of disintegration assemblies may be utilized. For example, a direct connection of a disintegration assembly to a frame of the implant may be utilized, with the direct connection being disintegrated at a desired time. A material that may disintegrate at a desired time may be utilized to connect the frame of the implant to the disintegration assembly. Electrical energy or other forms of energy (e.g., thermal) may be utilized to disintegrate the connection. A chemical actuation may be utilized to disintegrate the connection in examples. Other forms of disintegration assemblies may be utilized in examples.

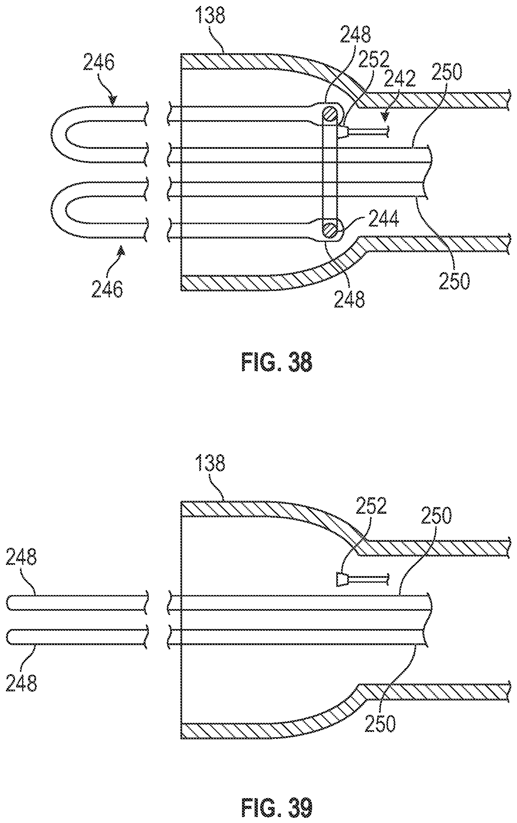

Other forms of disintegration with coupling tethers may be utilized. FIGS. 38 and 39, for example, illustrate an example in which the disintegration assembly 242 includes an intermediate body 244 that is configured to disintegrate. The disintegration of the intermediate body 244 may disintegrate the connection to the coupling tethers 246.

For example, the coupling tethers 246 may be configured similarly as the coupling tethers 215 and having an end portion or loop portion 248 that may connect to the intermediate body 244. The loop portion 248 may extend over the intermediate body 244 or otherwise couple to the intermediate body 244. The intermediate body 244 may have a ring shape or may have any other configuration as desired (e.g., a strip of material, a coil, one or more pins, one or more hooks, one or more terminals, among others). The opposite end portion 250 of the coupling tethers 246 may be configured similarly as the end portions 234 shown in FIG. 35 (e.g., coupled to a tether manifold) or may have another configuration as desired.

A heating element 252 may be utilized to disintegrate the intermediate body 244. The heating element 252, for example, may be in contact with the intermediate body 244 and may be configured to transmit heat to the intermediate body 244 that may cause the intermediate body 244 to melt or otherwise disintegrate. For example, the intermediate body 244 may be made of a disintegrable material such as a meltable filament, biocompatible material, or other disintegrable material.

FIG. 39, for example, illustrates the intermediate body 244 having disintegrated. The loop portions 248 are released from the intermediate body 244, which allows for release from the implant.

Other configurations of cutting and/or disintegration assemblies may be utilized. Any means for cutting, melting, disintegrating or detaching coupling tethers from an implant to facilitate implantation in the body are contemplated and are therefore within the scope of this disclosure.

FIGS. 40 and 41, illustrate another example of a disintegration assembly 258 wherein a first electrical terminal 260a is displaceable relative to a second electrical terminal 260b and is configured to contact the second electrical terminal 260b to complete a circuit for electrical energy to pass through a heating element 262.

Referring to FIG. 40, the heating element 262 may comprise a portion of a tether manifold 264 that couples to end portions or loop portions 266 of the coupling tethers 268. The end portions or loop portions 266 may extend over or loop over the heating element 262. The heating element 262 may have a straightened or linear shape and may comprise an arm of the tether manifold 264.

The coupling tethers 268 may have an end portion 270 that couples to a coupling point such as a point on an inner surface of the distal pusher 138 or another point as desired.

The first electrical terminal 260a may be coupled to a first electrical conduit 272a that may extend to the power source 228. The first electrical terminal 260a may be configured to move along with the movement of the tether manifold 264 as desired. For example, the first electrical terminal 260a may retract or advance as desired with the tether manifold 264.

The second electrical terminal 260b may be positioned on a shaft of the elongate catheter, for example, on an inner surface of a lumen of the mid shaft or mid shaft subassembly 24. The second electrical terminal 260b may be positioned on an inner surface of the distal pusher 138 or at another location as desired. The second electrical terminal 260b may be in a fixed position or may be movable with respect to one or more of the shafts of the elongate shaft or shaft assembly 18. As represented in FIG. 40, the second electrical terminal 260b may be in a fixed position with respect to the mid shaft or mid shaft subassembly 24. The first electrical terminal 260a may be movable with respect to the mid shaft or mid shaft subassembly 24.

The second electrical terminal 260b may be coupled to a second electrical conduit 272b that may extend to the power source 228. In examples, the second electrical conduit 272b may be embedded in a wall of the mid shaft or mid shaft subassembly 24. In examples, the second electrical conduit 272b may comprise the wall of the mid shaft or mid shaft subassembly 24. For example, the wall may be electrically conductive to allow for electrical energy transmission. Other configurations may be utilized as desired.

The first electrical terminal 260a may be unconnected to the second electrical terminal 260b at a time in which the disintegration assembly 258 is not activated. Such a configuration is represented in FIG. 40.

At a desired time, the first and second electrical terminals 260a, b may be moved relative to each other to contact the terminals 260a, b. Such a movement may be produced in a variety of manners. In examples, the movement may occur based on the tether manifold 264 being moved distally to advance the coupling tethers 268 distally. Such movement may comprise a deployment movement of the tether manifold 264. In examples, the movement may be produced based on other sliding movement of one or more of the shafts of the elongate catheter relative to each other.

In an example in which the movement occurs based on the tether manifold 264 being moved distally, the movement may be utilized to assure that activation of the disintegration assembly cannot occur until a desired alignment of the shafts relative to each other. In an example as shown in FIGS. 40 and 41, the disintegration assembly 258 could not be activated until the tether manifold 264 is advanced distally relative to the mid shaft or mid shaft subassembly 24. Such a feature may be utilized to assure that the implant will not be released prematurely or until the tether manifold 264 is advanced to a deployment position.

FIG. 41, for example, illustrates the tether manifold 264 having been advanced distally. The first and second electrical terminals 260a, b contact each other to complete the circuit for electrical energy to pass through the heating element 262. The heating element 262 may disintegrate the connection to the coupling tethers 268 and produce the free ends 274a, b shown in FIG. 41. By doing so, the coupling tethers 268 may be released from the implant.

Other configuration of disintegration assemblies may be utilized in examples. The features of the disintegration assemblies may be utilized solely or in combination with any other example disclosed herein.

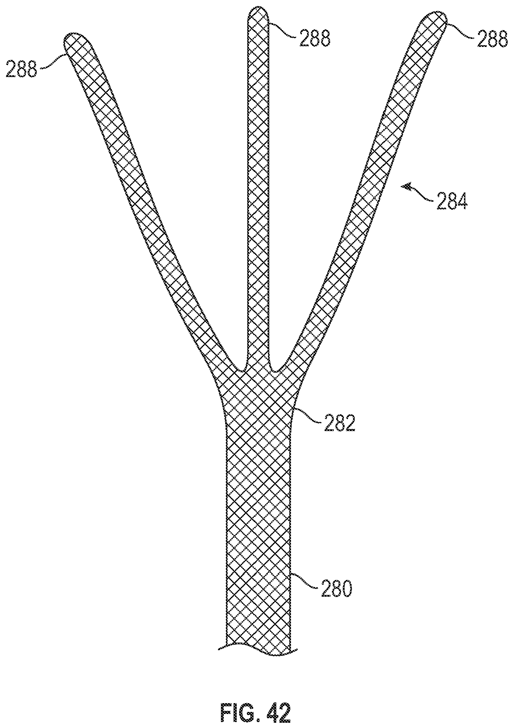

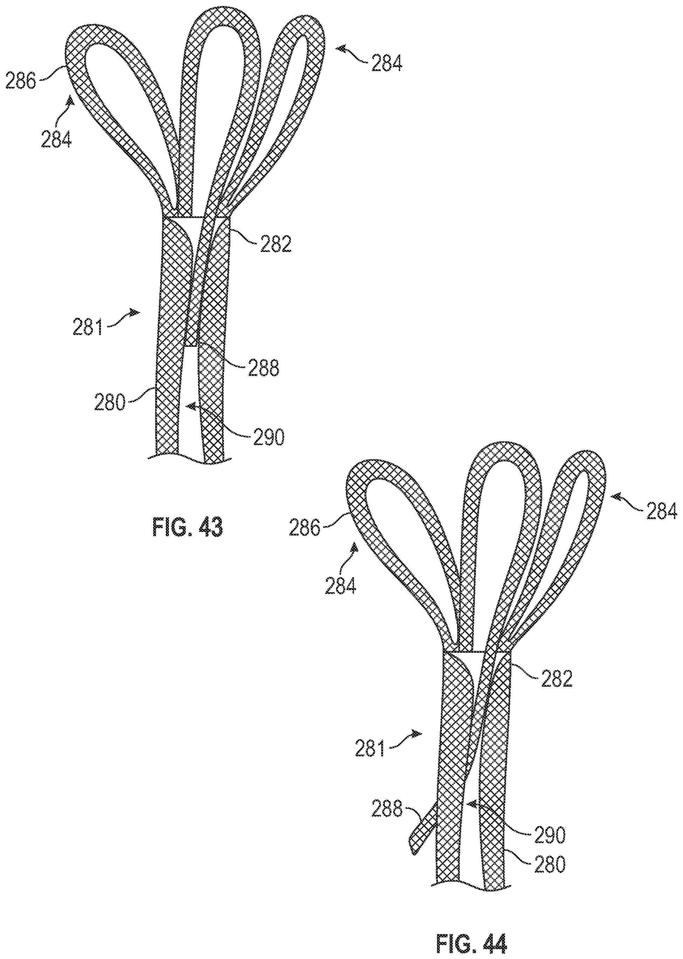

Various forms of coupling tethers may be utilized, and the scope of the present disclosure should not be limited to any particular coupling tether configuration. FIGS. 42 and 43, for example, illustrate a configuration in which a flexible retention member or tether 280 is woven or braided. The flexible retention tether 280 may comprise a plurality of sutures woven or braided into a larger suture. The tether manifold 282 may comprise a separation of the plurality of coupling tethers 284 from the flexible retention tether 280. The plurality of coupling tethers 284 may be woven or braided out from the flexible retention tether 280 and may comprise sub-portions of the weave or braid of the flexible retention tether 280. For example, the flexible retention tether 280 may include twenty-four strands, which may be broken out into three groups of eight strands each (for three coupling tethers 284). Various divisions may be utilized as desired (e.g., a greater or lesser number of coupling tethers, or of strands per coupling tether). The braid may comprise a cylindrical braid or flat braid or may have another configuration in examples.

The separate coupling tethers 284 shown in FIG. 42 may be looped back towards the flexible retention tether 280 to form loop portions 286 (marked in FIG. 43) of the coupling tethers 284. The free ends 288 of the coupling tethers 284, for example, may be looped back to be buried in the tether manifold 282 or the flexible retention tether 280. FIG. 43, for example, illustrates such a configuration in partial cross-sectional view. The free end 288 of the coupling tether 284, for example, inserts into and is buried within a central channel 290 of the tether manifold 282 or the flexible retention tether 280. The free end 288 may be buried within a main portion 281 of the flexible retention tether 280 that is proximal of the tether manifold 282. The friction with the walls of the central channel 290 may retain the free end 288 of the coupling tether 284 within the central channel 290. Other configurations may be utilized to form the loop portions 286 in examples. In examples, the cross-sectional area of the main portion 281 of the flexible retention tether 280 may be the same as the cross-sectional area of the plurality of coupling tethers 284 combined. In examples, the length of each of plurality of coupling tethers 284 from the tether manifold 282 may be greater than 0.5 inches (or greater than 1 inch), or other lengths may be utilized in examples.

Referring to FIG. 44, the free end 288 may pass through the wall of the tether manifold 282 to be locked to the tether manifold 282 or the flexible retention tether 280. A portion of the coupling tether 284 may be positioned within the central channel 290 and retained within the central channel 290. In examples, the lock may occur due to gluing or melting (e.g., reflow) of the coupling tether 284 or other forms of bonding.

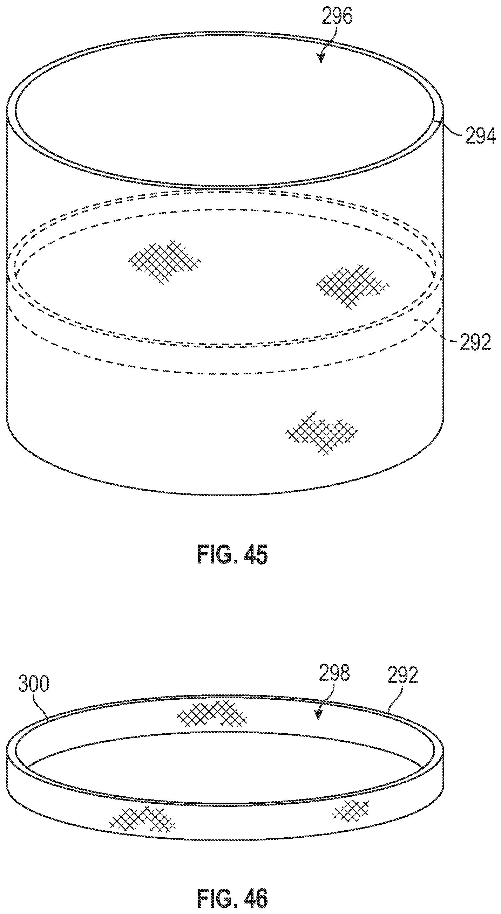

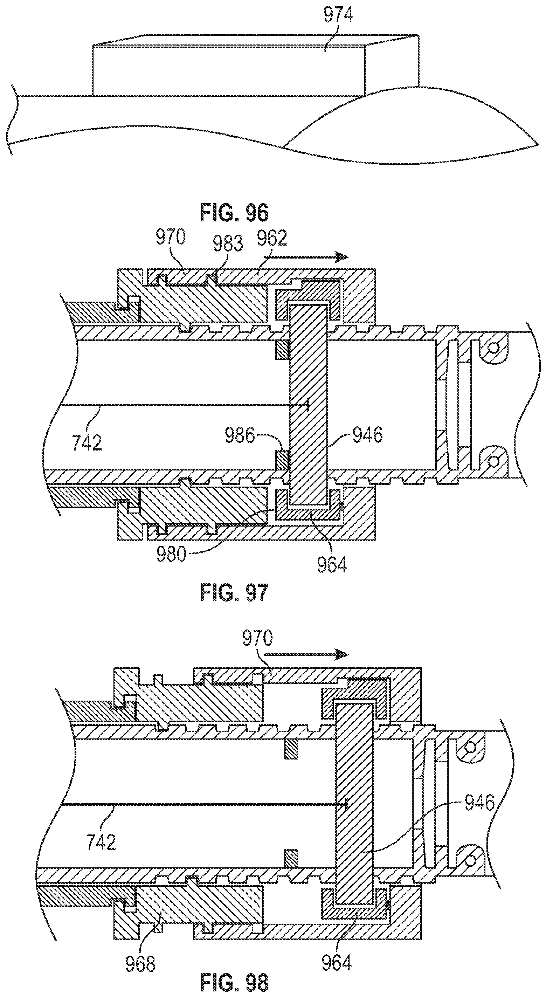

FIGS. 45 and 46 illustrate another example in which one or more coupling tethers may comprise a woven or braided ring 292. FIG. 45, for example, illustrates a woven or braided cylinder 294 that may be formed with weaving or braiding. The cylinder 294 may include a central opening 296 of a central lumen that is formed in the weaving or braiding process. A central axis may extend within and along the central lumen. The cylinder 294 may be cut in a direction transverse to the central axis of the cylinder 294 to form the ring 292 as shown in FIG. 96. One or more horizontal cuts may be made, which may be perpendicular to the central axis of the cylinder 294. The ring 292 is indicated in dashed lines in FIG. 45 and may form a portion of the cylinder 294.

FIG. 46 illustrates the ring 292 cut from the remainder of the cylinder 294 and shown isolated from the remainder of the cylinder 294. The woven or braided ring 292 may surround the central opening 298.

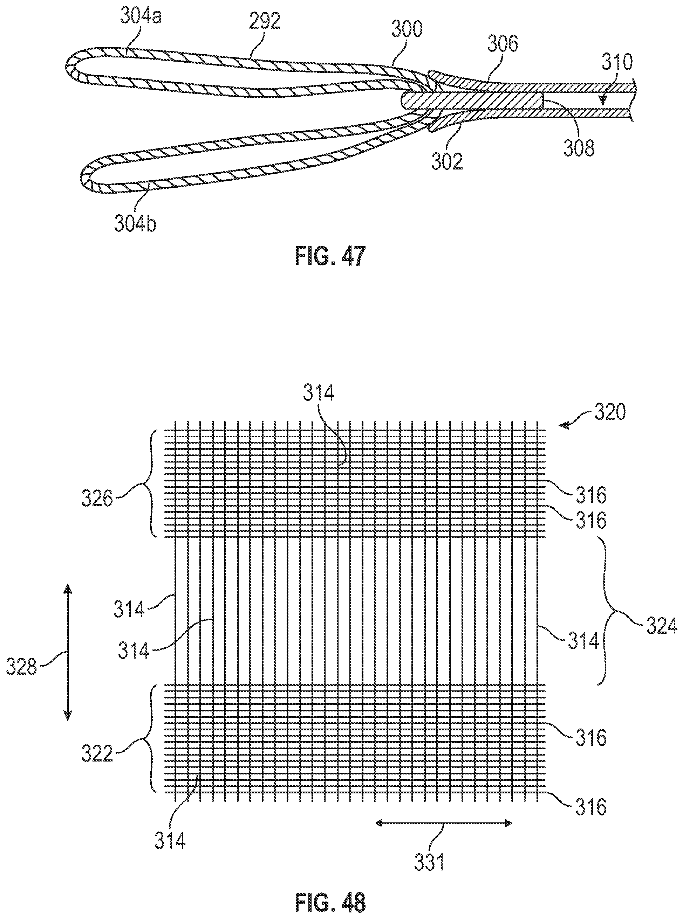

The ring 292 may form one or more coupling tethers. In examples, a portion of the ring 292 may be coupled to a tether manifold or a flexible retention member or tether to form a loop suitable for coupling to an implant. In examples, the ring 292 may form a plurality of coupling tethers. FIG. 47, for example, illustrates a portion 300 of the ring 292 having been coupled to the tether manifold 302. At least two loop portions 304a, b may extend distally from the tether manifold 302. Each loop portion 304a, b may comprise a coupling tether for coupling to a portion of the implant. In examples, a greater number of rings 292 may be utilized to produce a desired number of coupling tethers. In examples, two portions of the ring 292 may be coupled to the tether manifold 302 to produce three resulting loop portions and coupling tethers. In examples, multiple rings 292 may be coupled to the tether manifold 302 to produce a desired number of coupling tethers (e.g., two rings 292 may be coupled to provide four coupling tethers or six coupling tethers; three rings 292 may be coupled to provide six coupling tethers or nine coupling tethers). A greater or lesser number of rings 292 may be utilized as desired.

FIG. 47 illustrates a partial cross-sectional view of a coupling of the ring 292 to the tether manifold 302. At least a portion of the tether manifold 302 may overlap the woven or braided ring 292 to couple the woven or braided ring to the tether manifold 302. For example, a length 306 of the tether manifold 302 may extend over the ring 292, with the free end 308 buried into the channel 310 of the tether manifold 302. The configuration may retain the ring 292 to the tether manifold 302. In examples, the free end 308 may be locked to the tether manifold 302 in a similar manner as shown in FIG. 44. In examples, the free end 308 may be glued or melted (e.g., reflowed) into the tether manifold 302 to retain the ring 292 to the tether manifold 302.

FIG. 48-50 illustrate a configuration in which the tether manifold 312 includes warp strands 314 woven with weft strands 316, and the plurality of coupling tethers 318 each comprise a continuation of the warp strands 314 of the tether manifold 312 lacking a weave with any weft strands 316.

For example, referring to FIG. 48, a weave 320 may be formed comprising a section 322 including warp strands 314 and weft strands 316. The length of the section 322 may be set as desired during the weaving process. At a desired point during the weaving process, the weave may discontinue use of the weft strands 316. A section 324 may be formed that comprises the warp strands 314 and lacking any weave with the weft strands 316 (because the weft strands 316 have been discontinued). The length of the section 324 may be set as desired. The length of the section 324 may be set based on a desired length of the coupling tethers in examples.

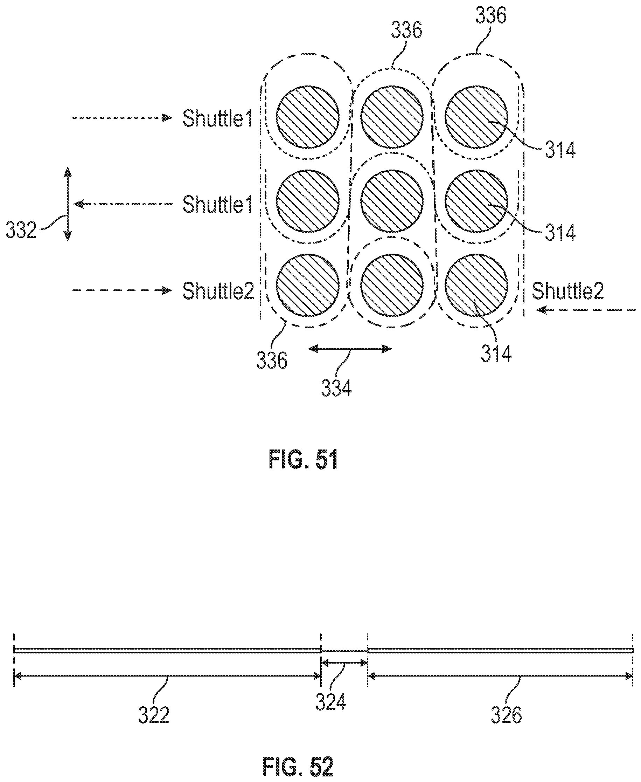

At a desired point during the weaving process, the use of the weft strands 316 may be resumed. A resulting section 326 may be formed with the section 324 between the sections 322, 326. The section 326 may include the weft strands 316. The length of the section 326 may be set as desired. FIG. 52 illustrates an exemplary length of the sections 322, 324, 326 that may be produced. The section 322, for example, may extend for a length of at least 50 inches, 60 inches, 70 inches, or 80 inches. The section 326, for example, may extend for a length of at least 50 inches, 60 inches, 70 inches, or 80 inches. The section 324, for example, may extend for a length of at least 1 inch, 2 inches, or 3 inches, in examples. Various other lengths may be utilized as desired. The lengths may be determined in the weaving process.

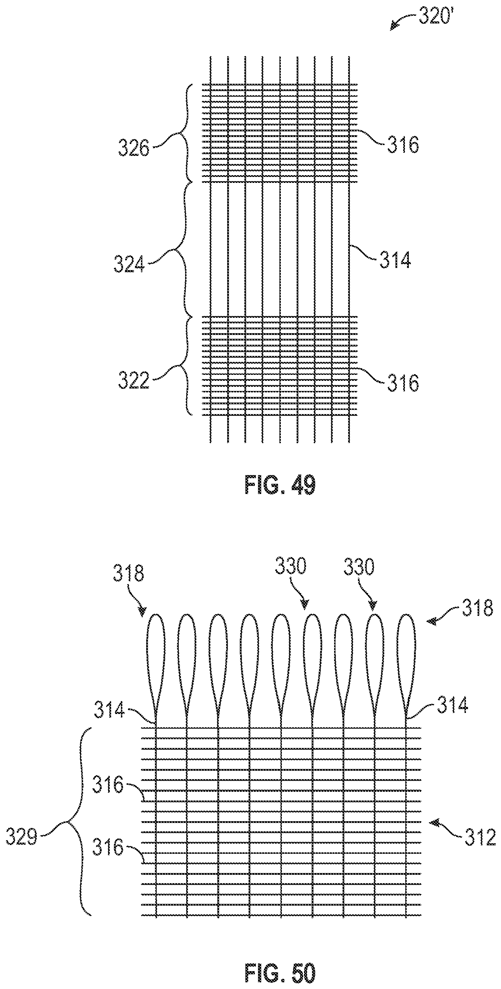

For example, the length 328 and the width 331 of the weave 320 may be cut to produce a desired size of the tether assembly. The width 331 may be set as desired. The width 331, for example, may be cut to a desired number of coupling tethers that may be utilized with the tether assembly. FIG. 49, for example, illustrates a resulting cut weave 320' that includes a width having nine warp strands 314, which would be utilized to produce nine coupling tethers. A greater or lesser number of warp strands may remain in the cut weave 320'.

The section 326 may be folded back onto the section 322, thus forming an overlapping section 329 (marked in FIG. 50). Gluing or melting (e.g., reflowing) may be utilized to bond the sections 326, 322 to each other. The fold of the section 326 may consequently form loops of the warp strands 314 that were positioned longitudinally between the sections 322, 326 in FIG. 49. The loops of the warp strands 314 may comprise looped coupling tethers 330. The coupling tethers 330 may be utilized to couple to a portion of an implant as desired. The length of the coupling tethers 330 may be a half of the length of the section 324 shown in FIG. 49 in examples.

In examples, the strands may comprise a braided suture. The strands comprising a warp strand may be a strength bearing braided suture, and/or the strands comprising a weft strand may be a strength bearing braided suture. In examples, any tether as disclosed herein may include a braided suture or other form of material.

The weave of the warp and weft strands shown in FIGS. 48-50 may comprise a flat weave or flat ribbon configuration. For example, the flat section 326 may be overlaid upon the flat section 322 to form the flat overlapping section 329. In examples, a stacked configuration may be utilized. FIG. 51 illustrates an exemplary warp and weft weave pattern in a stacked configuration. A vertical stack 332 and a horizontal stack 334 may be provided. The weft strands 336 may follow a pattern as represented in FIG. 51 (with a path of the weft strands of a first shuttle, or Shuttle 1 indicated in short dash and short dot dash lines, and a path of the weft strands of a second shuttle, or Shuttle 2 indicated in long dash and long dot dash lines). The stacked configuration may form a tether manifold as desired and/or a flexible retention member or tether. The stacked configuration may be layered upon itself to form looped coupling tethers in a similar manner as disclosed regarding FIG. 50. The layered stacked configuration may result in eighteen warp strands at the tether manifold. The stacked configuration may be glued or melted (e.g., reflowed) to bond the sections together. In examples, a greater or lesser number than nine warp strands may be utilized as desired.

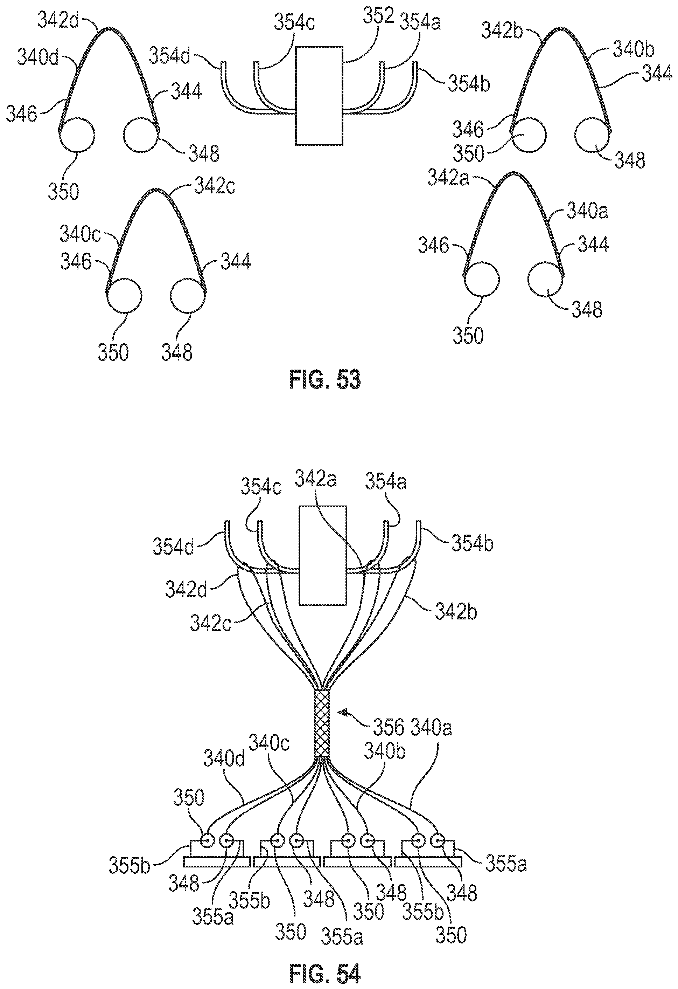

Other configurations may be utilized in examples. FIG. 53, for example, illustrates a configuration in which a plurality of strands 340a, b, c, d form respective loops 342a, b, c, d. Each strand 340a, b, c, d includes portions 344, 346 coupled to respective bobbins 348, 350. The loops 342a, b, c, d may each couple to a loop base 352 having a respective number of loop retainers 354a, b, c, d. The loop retainers 354a, b, c, d may comprise hooks or may have other configurations as desired. The loop base 352 and loop retainers 354a, b, c, d may hold the loops 342a, b, c, d during a weaving or braiding process.

The loops 342a, b, c, d may loop over a respective one of the loop retainers 354a, b, c, d. The bobbins 348, 350 for the respective strands 340a, b, c, d may be coupled to actuators 355a, b (marked in FIG. 54) that cause the bobbins 348, 350 to move to form a weave or braid of the strands 340a, b, c, d. FIG. 54, for example, illustrates a resulting weave or braid 356 formed by the motion of the bobbins 348, 350. The strands 340a, b, c, d are woven or braided together with the loops 342a, b, c, d remaining at the distal end of the weave or braid. The loop retainers 354a, b, c, d hold the loops 342a, b, c, d during the weaving or braiding process.

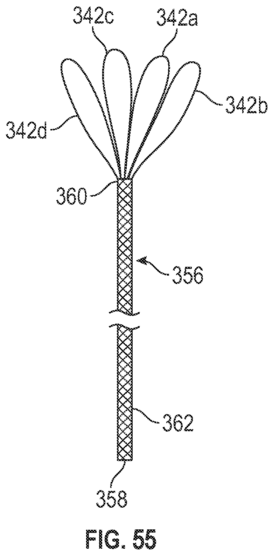

The formation of the weave or braid 356 of the strands 340a, b, c, d represented in FIG. 54 may continue for a desired length. At a desired time, the weave or braid 356 process may be discontinued and the proximal end of the weave or braid 356 may be cut. A resulting configuration is shown in FIG. 55. The tether manifold 360 may comprise the combination of the respective loops 342a, b, c, d, and the flexible retention tether 362 may comprise the weave or braid 356 of the strands 340a, b, c, d. The coupling tethers may comprise the loops 342a, b, c, d retained during the weaving or braiding process. The tether manifold 360 may comprise a weave or a braid of the strands 340a, b, c, d of the coupling tethers (comprising the loops 342a, b, c, d). The proximal end portion 358 of the weave or braid 356 may comprise the proximal free ends of the strands 340a, b, c, d that form the respective loops 342a, b, c, d.

It will be appreciated that any of the features of FIGS. 42-55 may be utilized solely or in combination with each other or any other example disclosed herein. In various examples, nine coupling tethers are illustrated and described. However, any greater or lesser number of loops or coupling tethers may be utilized (e.g., at least three, at least six, at least nine, at least twelve, etc.).

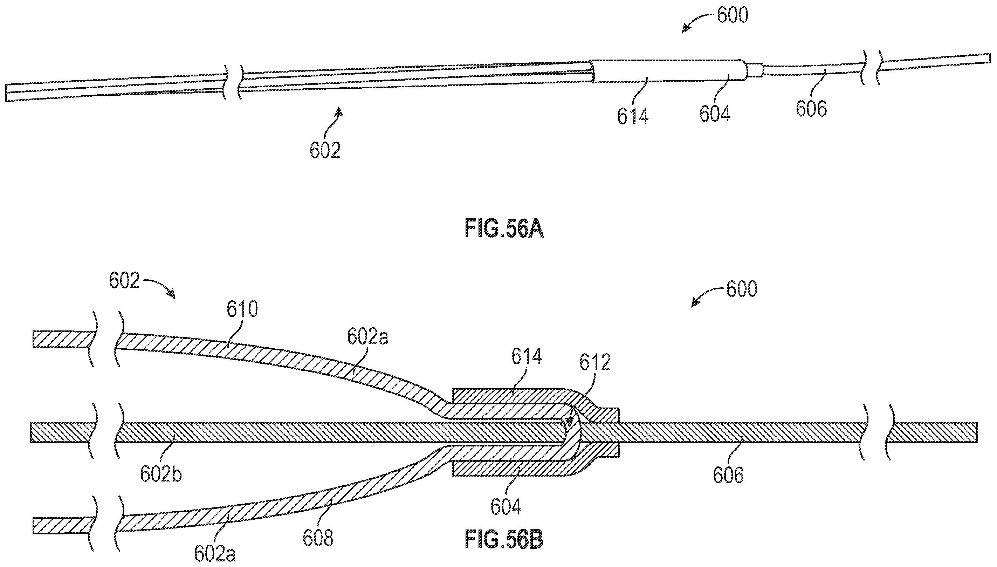

FIG. 56A illustrates an example of a release assembly 600 or subassembly. The release assembly 600 or subassembly may include the features of other release assemblies or subassemblies (e.g., release assembly or subassembly 28) unless stated otherwise. The release assembly 600 includes release tethers 602 having proximal ends coupled to a release tether manifold 604. Representative release tethers 602a, b are marked in the cross-sectional view of FIG. 56B. A retractable tether 606 (or other elongate structure) extends proximally from the release tether manifold 604.

The release tether manifold 604 may comprise a splice of the release tethers 602. For example, as shown in the cross-sectional view of FIG. 56B, a release tether 602a may splice through a release tether 602b to form two lengths 608, 610 each extending from the splice 612. Two release tether bodies accordingly may be utilized to form three release tethers 602. A greater or lesser number of release tethers may be utilized in examples.

The splice 612 may be covered with a sheath 614 that may secure the splice connection between the release tethers 602a, b. The sheath 614 may comprise a tube, or shrink tube, or reflowed material that secures the splice connection. The release tethers 602 may comprise cords, cables, wires, or other forms of tethers and may be flexible. The sheath 614 may comprise a polymer material (e.g., a plastic) that may secure the connection between the release tethers 602a, b. In examples, the splice connection may comprise a brummel splice, although other forms of splices may be utilized in examples. Knot connections (e.g., bowline knots, kreh knots, alpine loop knots, or other forms of knots) may be utilized in examples.

The release tethers 602, release tether manifold 604, and retractable tether 606 have sufficient flexibility and axial strength to withstand a proximal tension force used to retract and remove the release tethers 602, thereby allowing the coupling tethers to be detached from the implant. The release assembly 600 is preferably flexible to conform to a bend portion or deflection portion of an elongate shaft or shaft assemblies disclosed herein. Other forms of release assemblies or subassemblies may be utilized in examples.

Any of the features of FIGS. 56A or 56B may be utilized solely or in combination with each other or any other example disclosed herein.

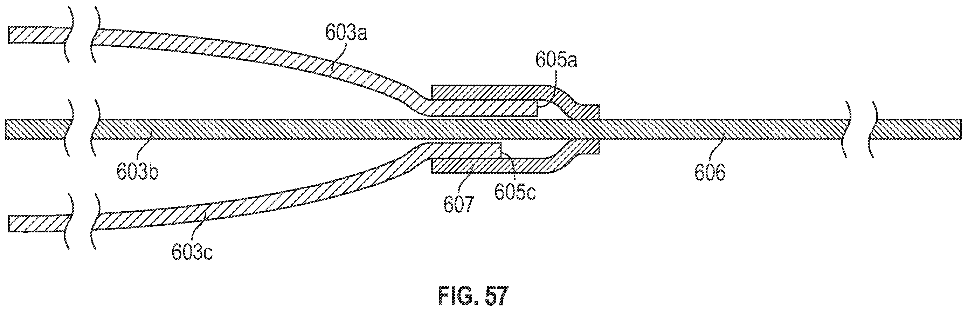

FIG. 57 illustrates a variation of the configuration of the release assembly 600 or subassembly. In the configuration of FIG. 57, a plurality of the release tethers (with three release tethers 603a, b, c shown in FIG. 57) are utilized. The release tethers 603a, 603c have respective proximal ends 605a, c that are positioned within the sheath 614 at the release tether manifold 607. The release tethers branch out from the release tether manifold 607. The proximal ends 605a, c join with the release tether 603b at the release tether manifold 607. The sheath 614 couples the ends 605a, c with the release tether 603b, with the ends 605a, c having lengths that are offset. As such, a tapered joint at the release tether manifold 607 may result.

Any of the features of FIG. 57 may be utilized solely or in combination with each other or any other example disclosed herein.

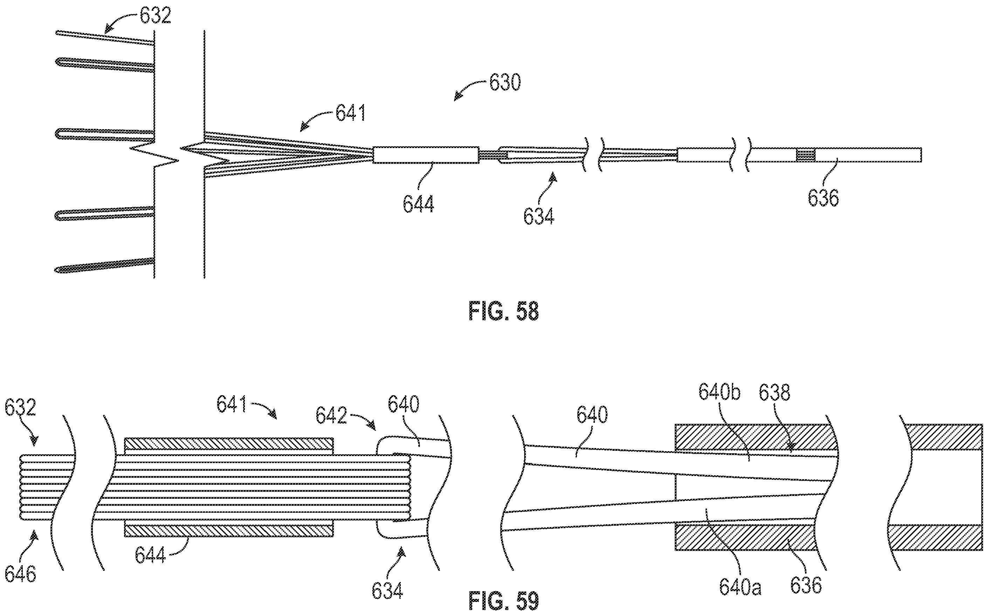

FIGS. 58 and 59 illustrate another example of a tether assembly 630 or subassembly. The tether assembly 630 or subassembly may include the features of other tether assemblies or subassemblies unless stated otherwise. The tether assembly 630 includes coupling tethers 632 having proximal ends coupled to a tether manifold 634. A flexible retention tether 636 extends proximally from the tether manifold 634.

The flexible retention tether 636 may comprise a wire or tube that extends distally to the tether manifold 634. The flexible retention tether 636 may include a receiving portion or lumen 638 (marked in FIG. 59) for receiving the tether manifold 634. The tether manifold 634, for example, may comprise arm lengths 640a, b (marked in FIG. 59) that may extend into the receiving portion or lumen 638. The arm lengths 640a, b may be crimped or otherwise coupled to the receiving portion or lumen 638 to secure the tether manifold 634 to the flexible retention tether 636. Other forms of flexible retention tethers and of coupling to a tether manifold may be utilized in examples.

The tether manifold 634 may comprise an elongate arm 640, which may include a loop portion 642 at its distal end. The loop portion 642 is shaped to facilitate attachment to the coupling tethers 632. The arm 640 may comprise a wire or cable that may be shaped to form the loop portion 642. The tether manifold 634 and flexible retention tether 636 may be constructed with an axial stiffness or column strength to allow a distal pushing force or compression force to be transmitted along the length of the tether manifold 634 and flexible retention tether 636 (e.g., to advance the coupled implant distally or expand the coupled implant). The tether manifold 634 and flexible retention tether 636 may have a lateral flexibility to allow for conforming to a bend portion or deflection portion of an elongate shaft or shaft assemblies disclosed herein.

The proximal end portions 641 of coupling tethers 632 couple to the tether manifold 634. The proximal end portions 641 of the coupling tethers 632 may loop around the loop portion 642 of the arm 640 in a similar manner as disclosed with respect to FIG. 14. For example, a single tether body may be looped alternatively around the arm 640 to form the multiple coupling tethers 632. In examples, other forms of coupling may be utilized.

In examples, a sheath 644 may be provided that may extend over the proximal end portions 641 of the coupling tethers 632. The sheath 644 may be positioned distal of the loop portion 642 of the arm 640 or may extend proximally to cover the loop portion 642 of the arm 640 in examples. The sheath 644 may serve to secure the connection of the coupling tethers 632 to the tether manifold 634 and may enhance a stiffness of the connection to improve transmission of a distal pushing force or compression force to be transmitted to the coupling tethers 632. The sheath 644 may comprise a tube, or shrink tube, or reflowed material at the proximal end portions 641 of the coupling tethers 632.

In examples, the coupling tethers 632 may have a length from the proximal end portions 641 of coupling tethers 632 to the loop portion, coupling portion, or distal end portion 646 of the coupling tethers 632 that extends the length of the coupling tethers 632 along a bend portion or deflection portion of an elongate shaft or shaft assemblies disclosed herein. As such, in examples, the coupling tethers 632 may extend through the bend portion or deflection portion (e.g., a portion corresponding to a primary or secondary flex portion of a rail shaft), with the tether manifold 634 and flexible retention tether 636 remaining proximal to such a portion. Other configurations may be utilized in examples.

Any of the features of FIGS. 58 or 59 may be utilized solely or in combination with each other or any other example disclosed herein.

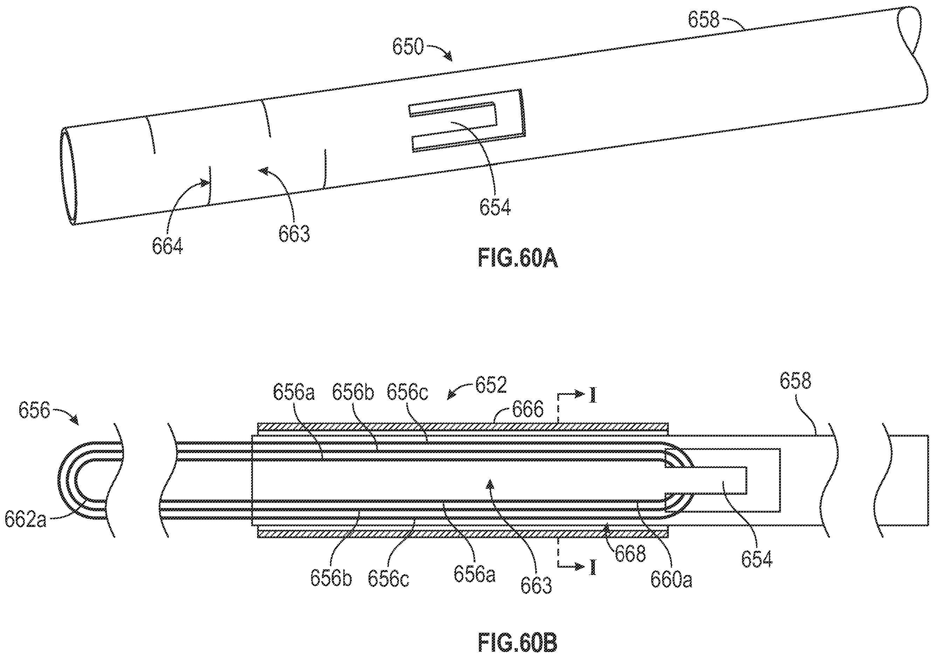

Variations in a configuration of the tether assembly or subassembly may be provided in examples. FIG. 60A, for example, illustrates a perspective view of a tether manifold 650 of a tether assembly 652 or subassembly (shown in partial cross-sectional view in FIG. 60B).

The tether manifold 650 may comprise one or more couplers 654 for coupling to the coupling tethers 656 (marked in FIG. 60B). The couplers 654 may comprise cut outs or tabs in a surface of material that the coupling tethers 656 may loop around to couple to. The cut outs or tabs may be in a surface (e.g., exterior surface) of the flexible retention tether 658. The couplers 654 may be equally spaced from each other circumferentially (with three couplers 654 spaced equidistant - e.g., 120 degrees from each other). Other spacing or numbers of couplers 654 (e.g., at least two, at least three, at least four, etc.) may be utilized as desired.

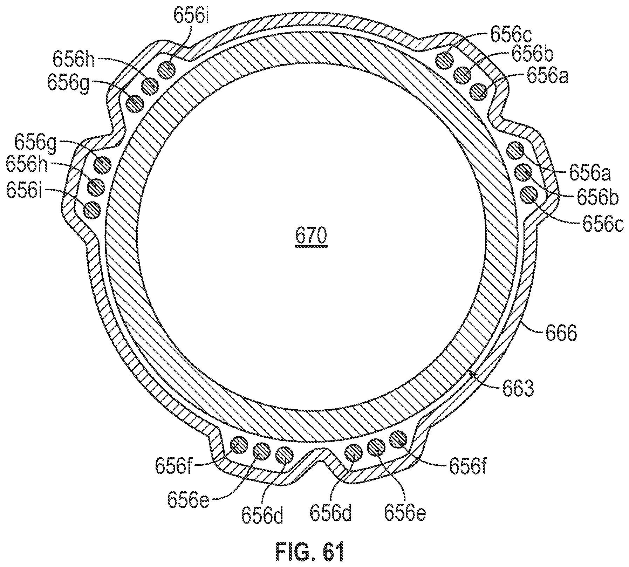

The coupling tethers 656 may loop around the couplers 654 as single body loops. Referring to FIG. 60B, for example, a coupling tether 656a may include two loops, with a proximal loop 660a looped around the coupler 654 and a distal loop 662a at distal end portion of the coupling tether 656a. Other coupling tethers (e.g., coupling tethers 656b, c) may be similarly looped. Other forms of connection (e.g., an alternating loop configuration as disclosed with respect to FIG. 14, or other forms of connection) may be utilized. A greater or lesser number of coupling tethers may be utilized as desired. With three couplers 654 and three coupling tethers 656 each, a total of nine coupling tethers 656a-i (as marked in FIG. 61) may be utilized. A greater or lesser number may be utilized as desired.

The coupling tethers 656 may extend along the outer surface 663 of the tether manifold 650 as represented in the cross-sectional view of FIG. 60B.

In examples, a friction feature 664 may be provided at the distal end portion of the tether manifold 650. The friction feature 664 may comprise cuts, ridges, or recesses in an outer surface 663 of the tether manifold 650 to enhance friction with the coupling tethers 656 that extend along the outer surface 663. In examples, the distal end portion of the tether manifold 650 may comprise a flexible material yet may have sufficient column strength for distal compressive forces.

In examples, a sheath 666 may extend over the proximal end portions 668 of the coupling tethers 656 that overlay the tether manifold 650. The sheath 666 may serve to secure the connection of the coupling tethers 656 to the tether manifold 650 and may enhance a stiffness of the connection to improve transmission of a distal pushing force or compression force to be transmitted to the coupling tethers 656. The sheath 666 may comprise a tube, or shrink tube, or reflowed material at the proximal end portions 668 of the coupling tethers 656. The sheath 666, for example, is shown in FIG. 61 to contour to a shape of the coupling tethers 656 through a reflow or shrink process. In examples, the sheath 666 may extend proximally to cover the couplers 654.

FIG. 61 illustrates a cross-sectional view of the tether manifold 650 along line I-I in FIG. 60B. The portion of the tether manifold 650 that the coupling tethers 656 overlay may comprise a tube having an interior lumen 670. The flexible retention tether 658 may further comprise the tube and the interior lumen 670 may extend proximally for the length of the flexible retention tether 658. The interior lumen 670 may be adapted for a nose cone shaft to extend through in an assembly of the delivery system. The interior lumen 670 may be adapted for a release assembly or subassembly as disclosed herein to extend through. In an example in which a release assembly extends through the interior lumen 670, the release tethers may be retracted proximally through the interior lumen 670. The coupling tethers 656 may be positioned symmetrically about the tether manifold 650. Other configurations may be utilized in examples. For example, the nose cone shaft may be positioned within the interior lumen 670 and the release assembly may be positioned exterior of the interior lumen 670 in examples.

Any of the features of FIGS. 60A-61 may be utilized solely or in combination with each other or any other example disclosed herein.

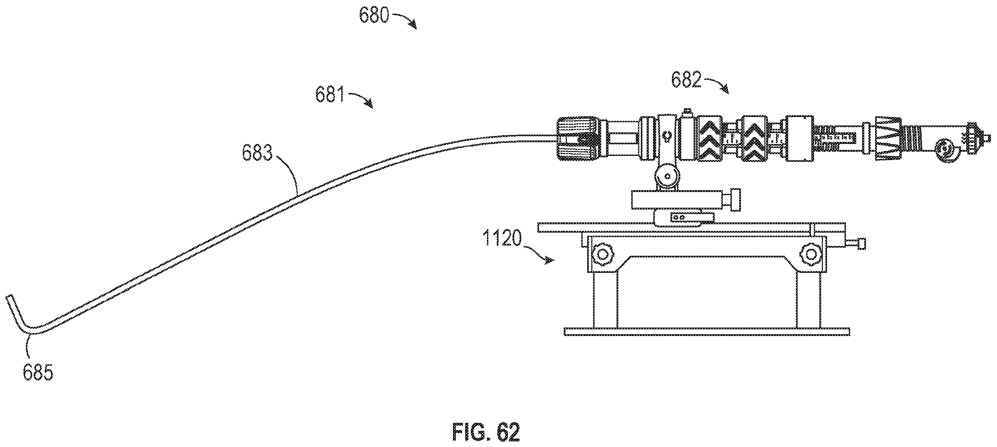

Variations in a configuration of a delivery system as disclosed herein may be provided. FIGS. 62-66 illustrate a delivery system 680 or delivery catheter that comprises a variation in the configuration of the delivery system 10 discussed with respect to FIGS. 1-31. The features of the delivery system 10 are utilized with the features of the delivery system 680 shown in FIGS. 62-66 unless stated otherwise. Other features of the delivery system 10 discussed with respect to FIGS. 1-31, such as the configuration of any shafts or subassemblies may be utilized with the delivery system 680. Features of other examples disclosed herein (e.g., examples of FIGS. 32-61) may be utilized with the delivery system 680 as desired.

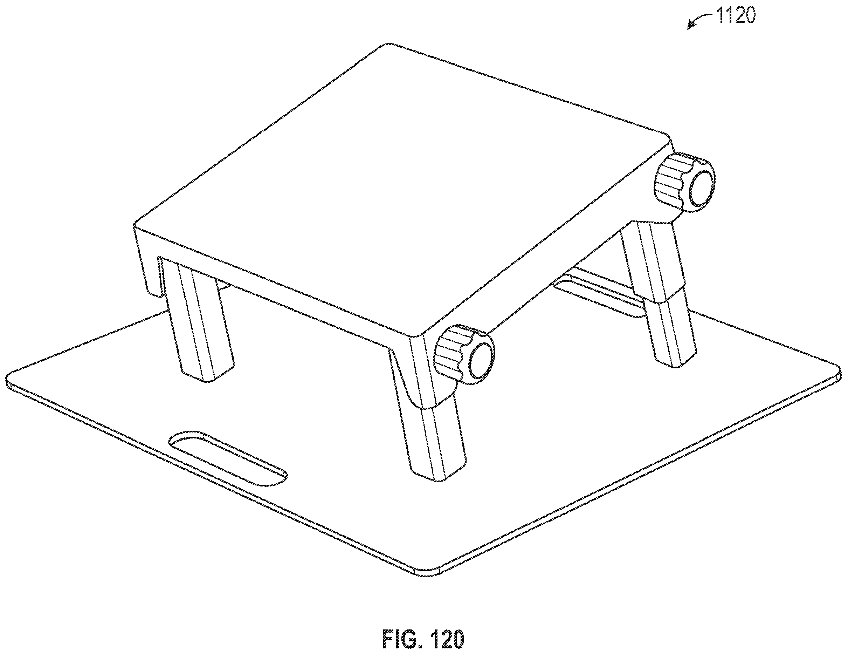

The delivery system 680 may be used to deploy a prosthesis, such as a prosthetic heart valve, to a location within the body of a subject. The delivery system 680 includes multiple components, devices, and/or subassemblies. As shown in FIG. 62, the delivery system 680 may include an elongate catheter, delivery device, or delivery catheter 681, and a stabilizer assembly 1120 (shown in a perspective view in FIG. 120), and other components as desired. The delivery catheter 681 may include an elongate shaft or shaft assembly 683 and a housing in the form of a handle 682. The housing may be at the proximal end portion of the elongate shaft 683 or shaft assembly. The elongate shaft 683 may include one or more shafts. A plurality of shafts may be provided according to examples herein, although in examples a single shaft may be utilized. The elongate shaft 683 or shaft assembly may be configured similarly as the elongate shaft or shaft assembly 18 unless stated otherwise. The elongate shaft 683 may be adapted to deflect about a bend portion 685 of the elongate shaft 683. Other shafts of the elongate shaft 683 may slide along the bend portion 685 to vary a depth of the distal end portion of the elongate shaft 683.

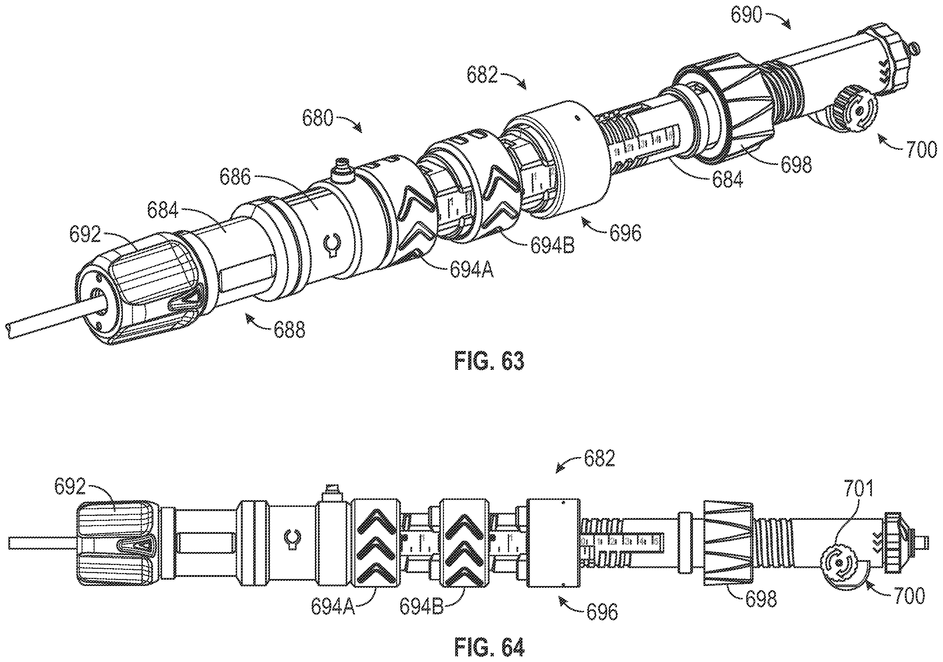

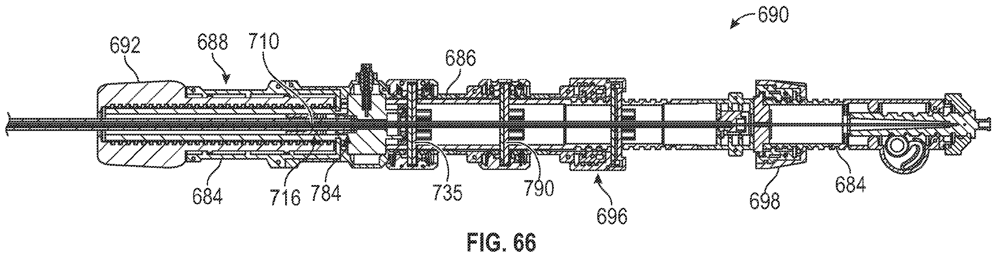

An example of a control mechanism or handle 682 is shown in FIGS. 63-66. The control mechanism or handle 682 comprises a variation of the handle 16 discussed previously with respect to FIGS. 1-31. The control mechanism or handle 682 may be adapted to control deflection of the elongate shaft 683 or shaft assembly, among other features (e.g., controlling depth of the elongate shaft 683, height of the elongate shaft 683, and/or expansion or release of the implant). The handle 682 comprises a housing including a first housing 684 and a second housing 686. The second housing 686 may be coupled to a rail shaft or subassembly. The first housing 684 may be coupled to one or more shafts that are adapted to slide relative to the rail shaft or subassembly to vary a depth of such shafts relative to the rail shaft or subassembly. The first housing 684 may include a distal portion 688 and a proximal portion 690, with the second housing 686 extending intermediate the distal portion 688 and proximal portion 690 and around the first housing 684 at an intermediate portion of the first housing 684 (as shown in the cross-sectional view of FIG. 66).

The handle 682 includes a capsule actuator or knob 692 (which may include the features of the capsule actuator or knob 34), respective actuator assemblies, deflection actuators, control knobs, or flex knobs 694A, B (which may include the features of the respective flex knobs 124A, B), a knob assembly 696 or depth actuator or depth knob, a coupling tether actuator or knob 698, and a release actuator or knob 700. The actuators may operate in similar manners as other respective actuators disclosed herein unless stated otherwise.

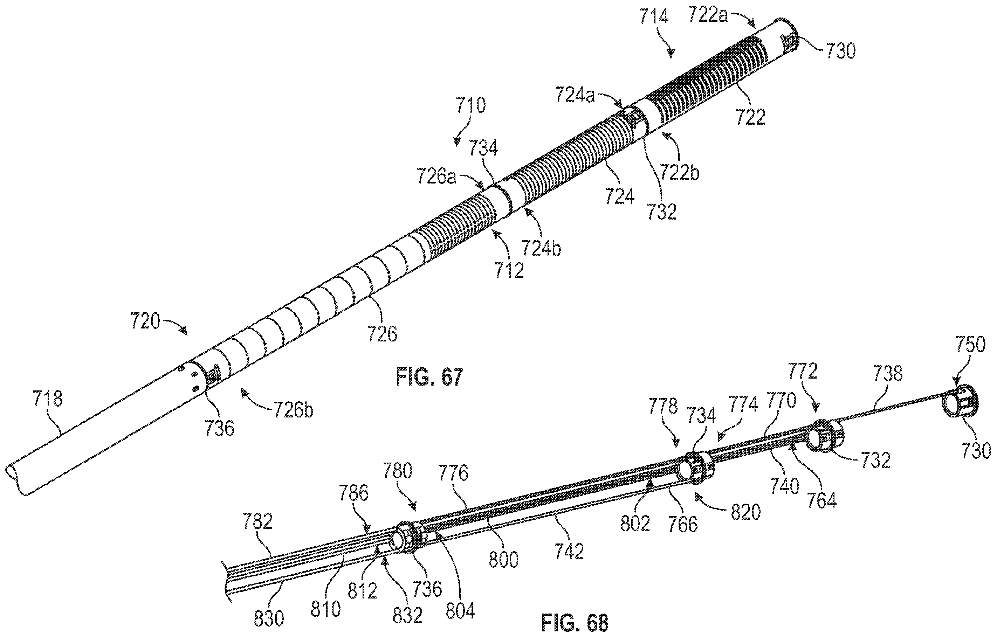

The configuration of one or more shafts or subassemblies may vary from a configuration disclosed with respect to the delivery system 10. FIGS. 67-70, for example, illustrate a variation of the rail shaft or subassembly 22 that may be utilized in examples herein.

Referring to FIG. 67, an elongate shaft 710 or rail shaft or subassembly is shown. The elongate shaft 710 may include the features of the rail shaft or subassembly 22 and may be adapted to be deflected in one or more planes. Other shafts of the delivery system 680, as disclosed herein may be adapted to slide relative to the elongate shaft 710 to vary a depth of those shafts along the elongate shaft 710.

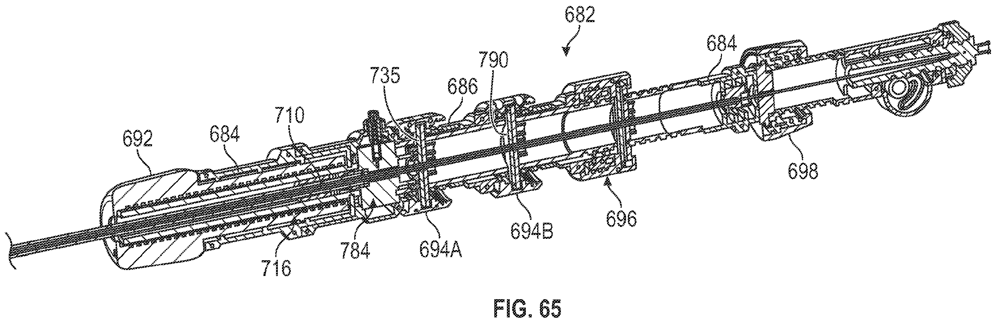

The elongate shaft 710 or rail shaft or rail subassembly includes an outer sheath 712. The outer sheath 712 has a distal end portion 714, a proximal end portion 716 (marked in FIGS. 65 and 66) and a length between the distal end portion 714 and the proximal end portion 716.

The outer sheath 712 includes a proximal shaft 718 or rail proximal shaft that may be configured similarly as the proximal shaft 112 unless stated otherwise. A distal end of the proximal shaft 718 may be adjacent to a hypotube 720 of the outer sheath 712. The hypotube 720 may include features of the hypotube 114 unless stated otherwise. The hypotube 720 may comprise a bend portion that is configured to form a bend for the other shafts or sheaths of the delivery system to follow. The other shafts or sheaths slide relative to the bend to vary a depth of the shafts or sheaths relative to the hypotube 720.

The distal end portion 714 of the outer sheath 712 may include one or more slotted portions or sections 722, 724, 726. Each slotted portion or section 722, 724, 726 may include a respective distal end portion 722a, 724a, 726a and proximal end portion 722b, 724b, 726b. A pattern of slots or cuts in each section 722, 724, 726 may define a direction or plane or deflection of the respective section 722, 724, 726.

The distalmost section 722 may include the features of the distal slotted hypotube section 126D unless stated otherwise. The distalmost section 722 may be adapted to bend or deflect in a plane to produce a primary deflection of the elongate shaft 710 (e.g., the medial-lateral trajectory). In a tricuspid implementation, a modification may be provided in which the distalmost section 722 provides the anterior-posterior trajectory.

The intermediate section 724 may include the features of the proximal slotted hypotube section 126P unless stated otherwise. The intermediate section 724 may be adapted to bend or deflect in a plane transverse or perpendicular to the plane of the distalmost section 722 to produce a secondary deflection of the elongate shaft 710 (e.g., the anterior-posterior trajectory). When adapted for deploying a prosthetic heart valve within a native tricuspid valve, a modification may be provided in which the intermediate section 724 provides the septal-lateral trajectory.

The proximal section 726 may be adapted to bend or deflect in the plane of the distalmost section 722 yet in an opposite direction within the plane. As such, the proximal section 726 is adapted to provide a height of the elongate shaft 710 or delivery catheter 681 in a direction opposite the direction of depth. The height direction is opposed to the direction of deflection of the distalmost section 722 to vary the height of the distal end of the delivery catheter (e.g., comprising the capsule or tip). Other directions of height (e.g., offset from the plane of the distalmost section 722) may be utilized in examples.

The proximal section 726 may be positioned distal and adjacent to the proximal shaft 718. In examples, the order of the slotted sections may be varied as desired. For example, the height flexing section may comprise an intermediate section, and the secondary deflection section (in the plane transverse to the height) may comprise a proximal section. Other variations may be provided in examples.

An assembly of pull tethers or pull wires may be utilized to actuate the elongate shaft 710 and deflect the hypotube 720 or bend portion of the outer sheath 712. FIG. 68 illustrates a perspective view of an assembly of pull tethers or pull wires that may be utilized (with the outer sheath 712 excluded from view for clarity in FIG. 68).

The assembly shown in FIG. 68 includes a plurality of connectors, ring-like structures or inserts 730, 732, 734, 736. The connectors, ring-like structures or inserts 730, 732, 734 may comprise pull tether or pull wire connectors, and can be used as attachment locations for the pull tethers or pull wires. The insert 730 may comprise an attachment location for the pull tether 738, the insert 732 may comprise an attachment location for the pull tether 740, and the insert 734 may comprise an attachment location for the pull tether 742.

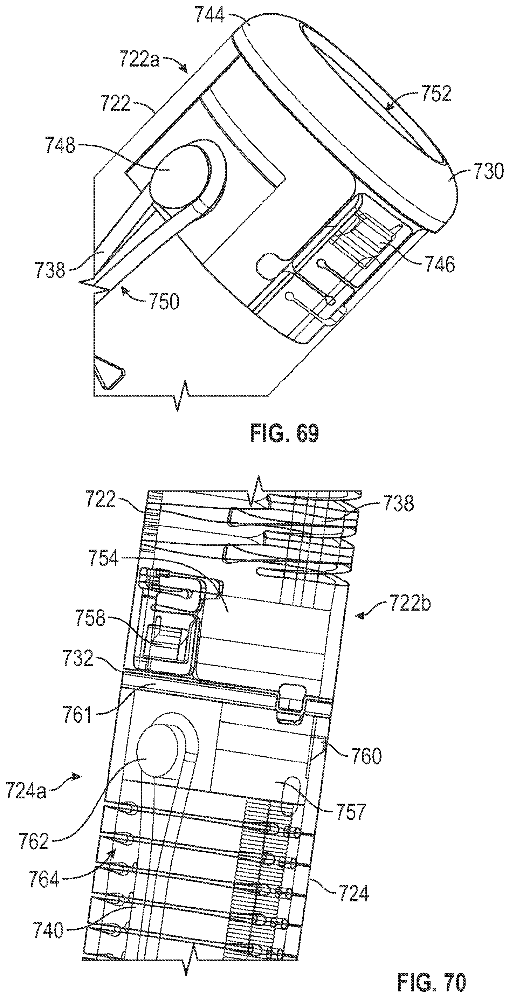

FIG. 69 illustrates a close-up perspective view of the distalmost insert 730. The insert 730 may include an outer flange or lip 744 that may abut and seat upon the distal end of the distalmost slotted section 722. The outer flange or lip 744 may impede proximal movement of the insert 730 upon a proximal tension force being applied by the pull tether 738. The insert 730 may include an alignment feature 746 such as a tab coupling with the distalmost slotted section 722 that may rotationally align the insert 730 relative to the distalmost slotted section 722. Further, torque may be transferred through the alignment feature 746. In examples, other forms of alignment features 746 may be utilized. The alignment feature 746 may comprise a groove or pocket, with a finger or tab of the distalmost slotted section 722 engaging the groove or pocket. The finger or tab may be pressed into the groove or pocket.

The insert may include a coupler 748 such as a post that may couple with a distal end portion 750 of the pull tether 738. The insert 730 may include a channel 752 for shafts or sheaths (e.g., a mid shaft or mid shaft subassembly, a tether assembly or subassembly, a release assembly or subassembly, and/or a nose cone shaft or subassembly) to pass through. The configuration of the insert 730 may be varied in examples.

FIG. 70 illustrates a close-up perspective view of the first intermediate insert 732. The first intermediate insert 732 may be positioned between the distalmost slotted section 722 and the intermediate slotted section 724. The first intermediate insert 732 may include a distal sleeve portion 754 and a proximal sleeve portion 756. The distal sleeve portion 754 inserts into the proximal end portion 722b of the distalmost slotted section 722. The proximal sleeve portion 756 inserts into the distal end portion 724a of the intermediate slotted section 724. The distal sleeve portion 754 may include an alignment feature 758 that may be configured similarly as the alignment feature 746 shown in FIG. 69. The proximal end portion 757 may include an alignment feature 760 that may be configured similarly as the alignment feature 746 shown in FIG. 69.

A central portion of the first intermediate insert 732 may include an outer flange 761 that may be positioned between the distalmost slotted section 722 and the intermediate slotted section 724. The outer flange 761 may abut and seat upon the distal end of the intermediate slotted section 724. The outer flange 761 may impede proximal movement of the insert 732 upon a proximal tension force being applied by the pull tether 740.

The first intermediate insert 732 may include a coupler 762 such as a post that may couple with a distal end portion 764 of the pull tether 740.

Referring to FIG. 68, the second intermediate insert 734 may be configured similarly as the first intermediate insert 732 yet adapted to couple to the distal end portion 766 of the pull tether 742. The second intermediate insert 734 may be positioned between the intermediate slotted section 724 and the proximal slotted section 726. The proximal insert 736 may be configured similarly as the first intermediate insert 732 and second intermediate insert 734, yet may lack a coupler 748, 762 for a pull tether.

The pull tethers or pull wires may include the distal pull tether 738 or pull wire having a distal end portion 750 and a proximal end portion (coupled to the pull tether adaptor 735 shown in FIG. 65). The pull tether 738 may extend along the length of the outer sheath 712. The distal end portion 750 may couple to the outer sheath 712 as shown in FIG. 69 and extends proximal from the coupling point or attachment point to the pull tether adaptor 735. The pull tether 738 may pass through lumens in the inserts 732, 734, 736 to extend to the pull tether adaptor 735. The distal end portion 750 of the distal pull tether 738 couples to the distal end portion 722a of the distal slotted portion 722.

The connection of the distal end portion 750 of the pull tether 738 to the coupler 748 may be a looped connection as shown in FIG. 69, with the length of the pull tether 738 looping about the coupler 748. The looped pull tether 738 may crimp to itself or the looped lengths of the pull tether 738 may extend proximally to the pull tether adaptor 735 along the length of the outer sheath 712.

A lumen in the form of a compression coil 770 may surround at least a portion of the pull tether 738 between the first intermediate insert 732 and the second intermediate insert 734. The compression coil 770 may operate in a similar manner as disclosed with respect to the compression coils 130 and may aid to reduce deflection of the intermediate slotted section 724 upon deflection of the distal slotted section 722. The compression coil 770 may include a distal end portion 772 and a proximal end portion 774, with the distal end portion 772 adapted to abut the first intermediate insert 732 and the proximal end portion 774 adapted to abut the second intermediate insert 734. The compression coil 770 has a larger diameter than the pull tether 738 and accordingly may be sized to not pass through the lumens of the first intermediate insert 732 and the second intermediate insert 734. The compression coil 770 is positioned proximal of the proximal end portion 722b of the distal slotted portion 722.

A compression coil 776 may surround at least a portion of the pull tether 738 between the second intermediate insert 734 and the proximal insert 736. The compression coil 776 may operate in a similar manner as disclosed with respect to the compression coils 130 and may aid to reduce deflection of the proximal slotted section 726 upon deflection of the distal slotted section 722. The compression coil 776 may include a distal end portion 778 and a proximal end portion 780, with the distal end portion 778 adapted to abut the second intermediate insert 734 and the proximal end portion 780 adapted to abut the proximal insert 736. The compression coil 776 is sized to not pass through the lumens of the second intermediate insert 734 and the proximal insert 736. The compression coil 776 is positioned proximal of the proximal end portion 724b of the intermediate slotted portion 724.

A tube 782 or hypotube may surround at least a portion of the distal pull tether 738 between the proximal insert 736 and the housing 784 or rail adaptor that the outer sheath 712 couples to. The tube 782 may include a distal end portion 786 and a proximal end portion (extending to the housing 784 or rail adaptor shown in FIG. 65). The distal end portion 786 of the tube 782 is adapted to abut the proximal insert 736.

The proximal end portion of the pull tether 738 may couple to the pull tether adaptor 735. The pull tether adaptor 735 is adapted to slide longitudinally along the handle 682, either proximally to produce axial tension in the pull tether 738 or distally to release or reduce the axial tension. The pull tether adaptor 735 is engaged with the control knob or flex knob 694A, with the flex knob 694A having a threaded coupling to the outer surface of the handle 682 and a non-threaded rotational coupling or fixed rotational coupling with the pull tether adaptor 735. Rotation of the flex knob 694A accordingly moves the knob 694A proximally or distally to move the pull tether adaptor 735 proximally or distally for axial tension or release of the axial tension of the pull tether 738. The deflection actuator or actuator assembly comprising the flex knob 694A and pull tether adaptor 735 applies a tension force to the pull tether 738 to deflect the elongate shaft 710. The elongate shaft 710 bends at the bend portion or distal slotted portion 722. In examples, the deflection actuator or actuator assembly may be utilized to distally axially push the pull tether 738 to actively unflex the rail shaft.

The pull tethers or pull wires may include the intermediate pull tether 740 or pull wire having a distal end portion 764 and a proximal end portion (coupled to the pull tether adaptor 790 shown in FIG. 65). The pull tether 740 may extend along the length of the outer sheath 712. The distal end portion 764 may couple to the outer sheath 712 as shown in FIG. 70 and extends proximal from the coupling point or attachment point to the pull tether adaptor 790. The pull tether 740 may pass through lumens in the inserts 734, 736 to extend to the pull tether adaptor 790. The distal end portion 764 of the intermediate pull tether 740 couples to the distal end portion 724a of the intermediate slotted portion 724.

The connection of the distal end portion 764 of the pull tether 740 to the coupler 762 may be a looped connection as shown in FIG. 70. The pull tether 740 may couple in a similar manner as the pull tether 738.

Referring again to FIG. 68, a lumen in the form of a compression coil 800 may surround at least a portion of the pull tether 740 between the second intermediate insert 734 and the proximal insert 736. The compression coil 800 may operate in a similar manner as disclosed with respect to the compression coils 130 and may aid to reduce deflection of the proximal slotted section 726 upon deflection of the intermediate slotted section 724. The compression coil 800 may include a distal end portion 802 and a proximal end portion 804, with the distal end portion 802 adapted to abut the second intermediate insert 734 and the proximal end portion 804 adapted to abut the proximal insert 736. The compression coil 800 has a larger diameter than the pull tether 740 and accordingly may be sized to not pass through the lumens of the second intermediate insert 734 and the proximal insert 736. The compression coil 800 is positioned proximal of the proximal end portion 724b of the intermediate slotted portion 724.

A tube 810 or hypotube may surround at least a portion of the pull tether 740 between the proximal insert 736 and the housing 784 or rail adaptor that the outer sheath 712 couples to. The tube 810 may include a distal end portion 812 and a proximal end portion (extending to the housing 784 or rail adaptor shown in FIG. 65). The distal end portion 812 of the tube 810 is adapted to abut the proximal insert 736.

The proximal end portion of the intermediate pull tether 740 may couple to the pull tether adaptor 790 (marked in FIG. 65). The pull tether adaptor 790 is adapted to slide longitudinally along the handle 682, either proximally to produce axial tension in the intermediate pull tether 740 or distally to release or reduce the axial tension. The pull tether adaptor 790 is engaged with the control knob or flex knob 694B, with the flex knob 694B having a threaded coupling to the outer surface of the handle 682 and a non-threaded rotational coupling with the pull tether adaptor 790. Rotation of the flex knob 694B accordingly moves the knob 694B proximally or distally to move the pull tether adaptor 790 proximally or distally for axial tension or release of the axial tension of the pull tether 740. The deflection actuator or actuator assembly comprising the flex knob 694B and pull tether adaptor 790 applies a tension force to the pull tether 740 to deflect the elongate shaft 710. The elongate shaft 710 bends at the bend portion or intermediate slotted portion 724. The elongate shaft 710 deflects in a plane that is transverse or perpendicular to the plane of deflection of the distal slotted section 722.

The pull tethers or pull wires may include the proximal pull tether 742 or pull wire having a distal end portion 820 and a proximal end portion (coupled to the knob assembly 696 shown in FIG. 65). The pull tether 742 may extend along the length of the outer sheath 712. The distal end portion 820 may couple to the outer sheath 712 in a similar manner as shown in FIG. 70 and extends proximal from the coupling point or attachment point to the knob assembly 696. The pull tether 742 may pass through a lumen in the proximal insert 736 to extend to the knob assembly 696. The knob assembly 696 may actuate the pull tether 742 to bend the elongate shaft 710 to vary the height of the distal end portion of the delivery catheter (e.g., comprising the capsule or tip).

The connection of the distal end portion 820 of the pull tether 742 to a coupler of the second intermediate insert 734 may be a looped connection similar to the connection shown in FIG. 70. The distal end portion 820 of the proximal pull tether 742 couples to the distal end portion 726a of the proximal slotted portion 726.

Referring again to FIG. 68, a tube 830 or hypotube may surround at least a portion of the pull tether 742 between the proximal insert 736 and the housing 784 or rail adaptor that the outer sheath 712 couples to. The tube 830 may include a distal end portion 832 and a proximal end portion (extending to the housing 784 or rail adaptor shown in FIG. 65). The distal end portion 832 of the tube 810 is adapted to abut the proximal insert 736.

Tension applied to the proximal pull tether 742 deflects the elongate shaft 710 in the plane of deflection of the distal slotted section 722, yet in an opposite direction. The tension may produce height in a direction opposite a depth of the delivery catheter 681.

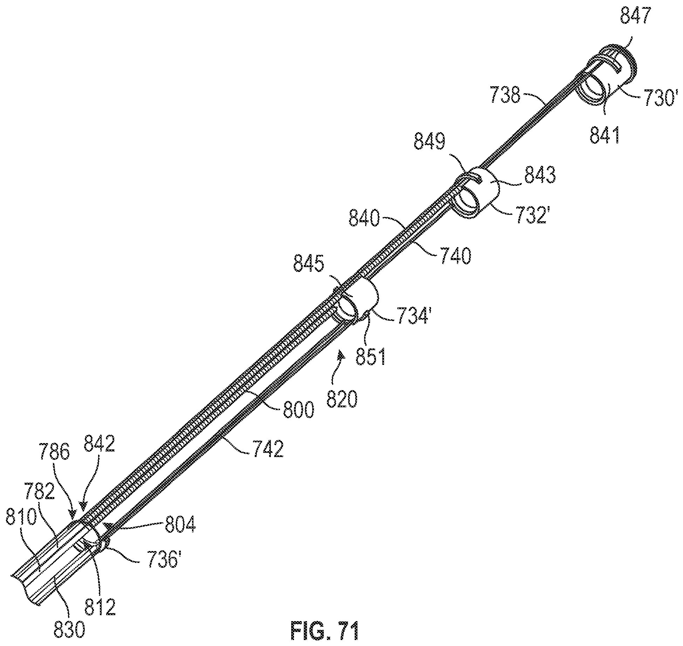

Variations in the configuration of the assemblies shown in FIGS. 67-70 may be provided. FIGS. 71-75, for example, illustrate a variation in which the lumens in the form of tubes 782, 810 abut the respective compression coils 800, 840. The distal end portions 786, 812 of the respective tubes 782, 810 abut the respective proximal end portions 842, 804 of the compression coils 840, 800. Features of the assemblies shown in FIGS. 67-70 apply to the features of the assembly of FIGS. 71-75 unless stated otherwise.

The lumen or compression coil 800 may be unconnected directly to the outer sheath 712 and may be slidable relative to the pull tether 740. The tube 810 may be unconnected directly to the outer sheath 712 and may be slidable relative to the pull tether 740. A force exerted against the compression coil 800 (e.g., by the insert 734', or via other forces upon the compression coil 800) may be transmitted through the tube 810 via the abutting contact between the compression coil 800 and the tube 810. The compression coil 800 may extend for the length of the proximal slotted section 726 before contacting the tube 810.

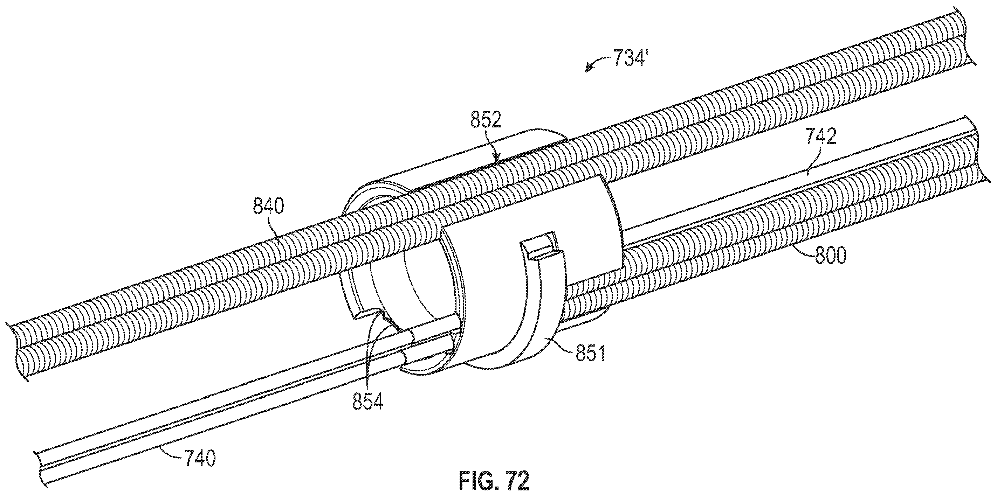

Referring to FIG. 72, an insert 734' comprises a variation of the second intermediate insert 734 shown in FIG. 68. The insert 734' includes a channel 852 for the compression coil 840 to extend through. The channel 852 is sized larger than the lumens 854 for the pull wire 740 to pass through, and has a larger diameter for passage of the compression coil 840.

The lumen or compression coil 840 comprises a compression coil having the lengths of the coils 770, 776 shown in FIG. 68, and extends continuously from the first intermediate insert 732' to the position of the proximal insert 736'. Features of the compression coils 770, 776 otherwise apply to the compression coil 840. The compression coil 840 may be unconnected directly to the outer sheath 712 and may be slidable relative to the pull tether 738. The tube 782 may be unconnected directly to the outer sheath 712 and may be slidable relative to the pull tether 738. A force exerted against the compression coil 840 (e.g., by the insert 732', or via other forces upon the compression coil 840) may be transmitted through the tube 782 via the abutting contact between the compression coil 840 and the tube 782. The compression coil 840 may extend for the length of the proximal slotted section 726 and the intermediate slotted section 724 before contacting the tube 782.

The inserts 730', 732', 734', 736' may comprise variations of the respective inserts 730, 732, 734, 736 shown in FIG. 68. The inserts 730', 732', 734' may each include respective molded sleeves 841, 843, 845 with respective plates or flanges 847, 849, 851 that engage the outer sheath 712. The insert 736' may lack a molded sleeve and may comprise a plate or flange for abutting the tube 830. Features of the inserts 730, 732, 734, 736 otherwise apply to the inserts 730', 732', 734', 736'.

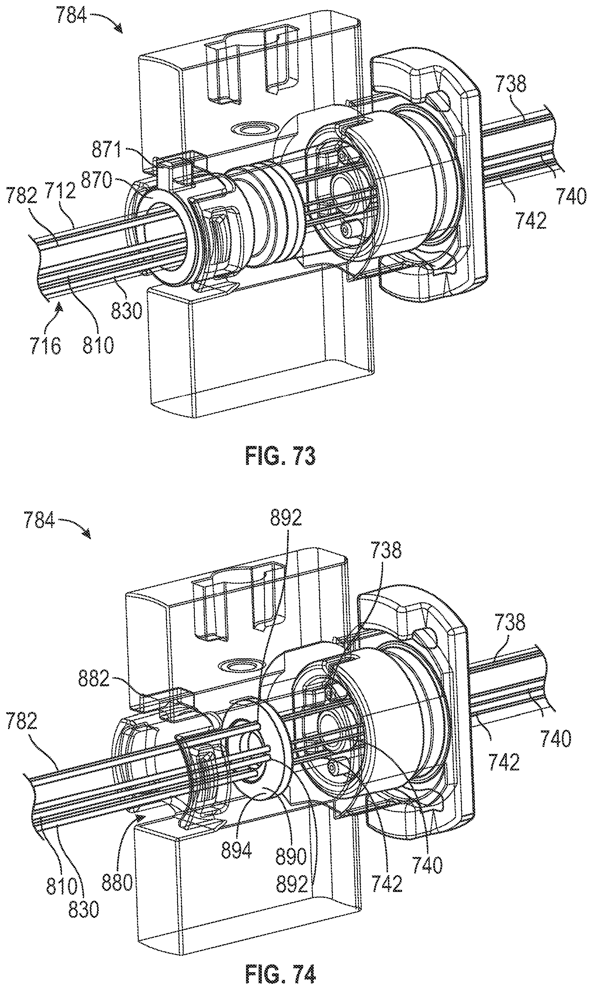

FIG. 73 illustrates a configuration of a housing 784 or rail adaptor that the proximal end portion 716 of the outer sheath 712 couples to. The proximal end portions of the tubes 782, 810, 830 are shown.

The housing 784 or rail adaptor may be slidably engaged with the proximal end portion 716 of the outer sheath 712. An outer sheath adaptor 870, for example, may be coupled to the proximal end portion 716 of the outer sheath 712 in a fixed and immobile manner. The outer sheath adaptor 870 accordingly may move longitudinally or axially with longitudinal or axial movement of the outer sheath 712 according to forces applied to the outer sheath 712. The outer sheath adaptor 870 may include an alignment feature 871 (e.g, a clocked or keyed protrusion) that may align the outer sheath adaptor 870 with the housing 784 or rail adaptor. The alignment feature 871 may maintain the rotational orientation of the outer sheath 712 and may be utilized to transmit a torque force in examples.

FIG. 74 illustrates a perspective view of the housing 784 or rail adaptor with the outer sheath adaptor 870 removed from view for clarity. The housing 784 includes a receiver 880 for sliding engagement with the outer sheath adaptor 870. The housing 784 may include an alignment receiver 882 or slot that receives the alignment feature 871. The alignment feature 871 may slide within the alignment receiver 882 or slot. The sliding motion may be an axial motion, longitudinally along the axis of the outer sheath 712. The alignment feature 871 may prevent rotation about the axis by the outer sheath 712.

The housing 784 or rail adaptor may include a support plate 890 including one or more openings 892 for the pull tethers 738, 740, 742 to pass through. The openings 892 may be positioned in rotational alignment with the position of the pull tethers 738, 740, 742 to allow the pull tethers 738, 740, 742 to pass proximally to their respective actuator assemblies. The openings 892 may be sized such that the proximal end portions of the respective lumens or tubes 782, 810, 830 do not pass through the support plate 890. The support plate 890 includes a surface 894 that abuts the proximal end portions of the tubes 782, 810, 830. In examples, the surface 894 may include a countersunk hole or annular ring for receiving each of the proximal end portions of the tubes 782, 810, 830. In examples, a plurality of plates may be stacked upon each other to produce the countersunk hole or annular ring (with a proximal plate having a smaller hole diameter than a hole diameter of a distal plate to form the countersunk hole or annular ring).

The support plate 890 is fixedly coupled to the housing 784 or held in an immobile configuration within the housing 784. As such, a proximal force asserted against any of the compression coils 840, 800 or tubes 782, 810, 830 bears upon the support plate 890. The support plate 890 has sufficient strength to support the load produced upon the compression coils 840, 800 or tubes 782, 810, 830 due to actuation of any of the pull tethers 738, 740, 742. A force exerted upon a compression coil 840, for example, is transmitted to the support plate 890 through the tube 782. A force exerted upon the compression coil 800, for example, is transmitted to the support plate 890 through the tube 810. The lack of a direct connection between the respective lumens in the form of compression coils 840, 800 and tubes 782, 810, 830 and the outer sheath 712 allows the force to be transmitted and borne by the support plate 890.

The sliding engagement of the housing 784 with the proximal end portion 716 of the outer sheath 712 allows the outer sheath 712 to slide relative to the support plate 890. As such, forces that may be received by the outer sheath 712 may produce movement of the outer sheath 712 relative to the housing 784, to reduce the possibility of adverse compression or damage to the outer sheath 712 during actuation of any of the pull tethers 738, 740, 742.

The forces being borne by the support plate 890, and the sliding movement of the outer sheath 712 relative to the housing 784 may reduce the presence of cross talk or interference between actuation of the respective pull tethers 738, 740, 742. A force of actuation of the pull tether 738 for example, may be borne by the support plate 890 rather than at the intermediate slotted portion 724, thus reducing deflection of the intermediate slotted portion 724 (and the proximal slotted portion 726) upon deflection of the distal slotted portion 722. Similarly, a force of actuation of the pull tether 740 may be borne by the support plate 890 rather than at the proximal slotted portion 726, thus reducing deflection of the proximal slotted portion 726 upon deflection of the intermediate slotted portion 724.

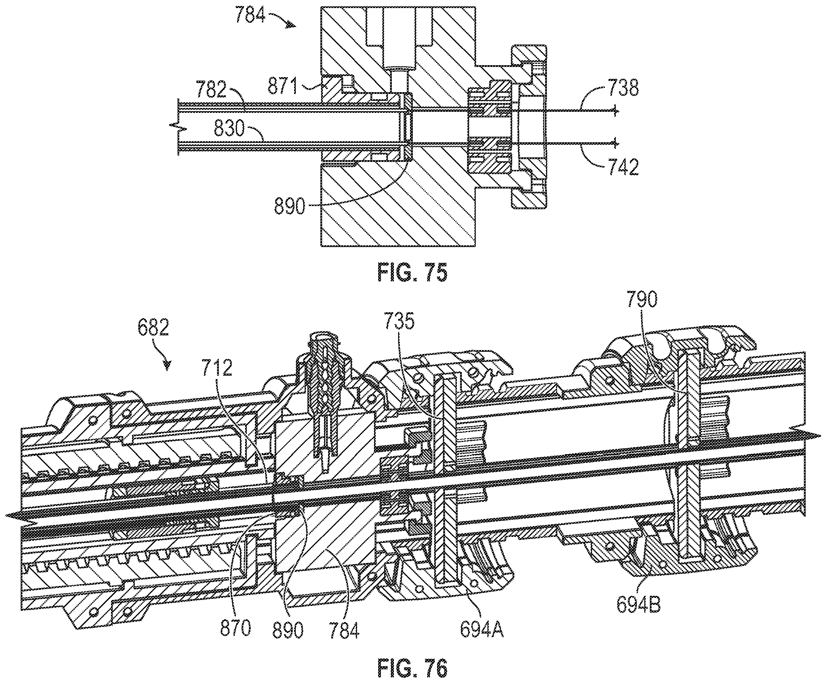

FIG. 75 illustrates a cross-sectional view of the assembly of FIGS. 73 and 74. An assembly of FIGS. 73-75 may be integrated into the handle 682. FIG. 76, for example, illustrates a representative configuration. The handle 682 accordingly may receive the support plate 890, the housing 784 or rail adaptor, and the actuator assemblies (in the form of control knobs or flex knob 694A, 694B and the respective adaptors 735, 790). The sliding engagement of the housing 784 or rail adaptor with the proximal end portion 716 of the outer sheath 712 allows the outer sheath 712 to slide relative to the handle 682 (and particularly the second housing 686 of the handle 682).

A configuration as shown in FIGS. 73-75 may be utilized with an assembly as shown in FIG. 71 and 72, or with an assembly as shown in FIGS. 67-70.

Any of the features of FIGS. 67-76 may be utilized solely or in combination with each other or any other example disclosed herein.

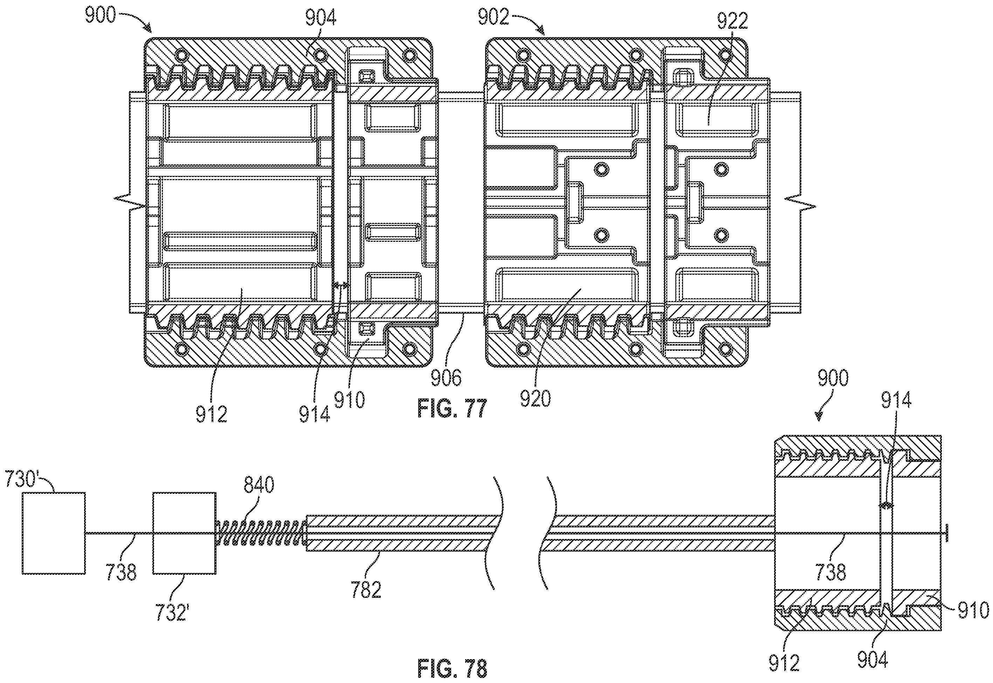

Variations in an actuator assembly or deflection actuator for the pull tethers 738, 740 may be provided in examples. FIGS. 77-80 illustrate a variation in which actuator assemblies 900, 902 are utilized and are configured to apply a tension force to the respective pull tethers 738, 740 and simultaneously apply a distal compressive force to the respective lumen (the tubes 782, 810 and/or compression coils 840, 800).

A cross-sectional view of the respective distal or primary actuator assembly 900 and the intermediate or secondary actuator assembly 902 is shown in FIG. 77. The distal or primary actuator assembly 900 includes a flex knob or control knob 904. The flex knob or control knob 904 is not threadably engaged with the outer surface of the handle of the delivery system. Rather, the handle (with the second housing 906 corresponding to the second housing 686) lacks a threaded engagement with the flex knob or control knob 904.

The flex knob or control knob 904 engages the pull tether adaptor 910 with a fixed rotational coupling (e.g., a non-threaded coupling) that allows the control knob 904 to rotate relative to the pull tether adaptor 910 and to apply a longitudinal or axial force to the pull tether adaptor 910. The pull tether adaptor 910 moves longitudinally with the control knob 904. The pull tether adaptor 910 couples to the proximal end portion of the pull tether 738 in a similar manner as the pull tether adaptor 735.

The flex knob or control knob 904 engages a lumen adaptor 912 with a threaded rotational coupling. As such, rotation of the control knob 904 causes the lumen adaptor 912 to slide longitudinally, with a first direction of rotation producing longitudinal retraction and a second direction producing longitudinal advancement.

The lumen adaptor 912 is adapted to contact the proximal end of the tube 782. As such, the length of the tube 782 may be greater than in an example as shown in FIGS. 73-75 and the use of the support plate 890 may be excluded. The lumen adaptor 912 may directly contact and abut the proximal end of the tube 782.

The fixed rotational coupling to the pull tether adaptor 910, and the threaded rotational coupling with the lumen adaptor 912 causes rotation of the control knob 904 to vary the spacing 914 between the lumen adaptor 912 and the pull tether adaptor 910. Rotation in a first direction may increase the spacing 914 and rotation in an opposite direction may decrease the spacing 914. As such, rotation of the control knob 904 in a single direction drives the pull tether adaptor 910 and the lumen adaptor 912 in opposite directions longitudinally.

The compression coil 840 and tube 782 may be in a configuration as discussed in regard to FIGS. 71 and 72. As such, a distal force applied to the tube 782 may be transmitted to the compression coil 840 and to the insert 732'. The tube 782 and compression coil 840 accordingly may stiffen and a reduced possibility of deflection of the intermediate slotted portion 724 (and proximal slotted portion 726) may result. Cross talk between deflection of one of the slotted portions and another of the slotted portions may be reduced. The actuator assembly 900 is adapted to simultaneously apply a tension force to the pull tether 738 that is equal and opposite to a distal compressive force applied to the lumen (e.g., the compression coil 840 and tube 782).

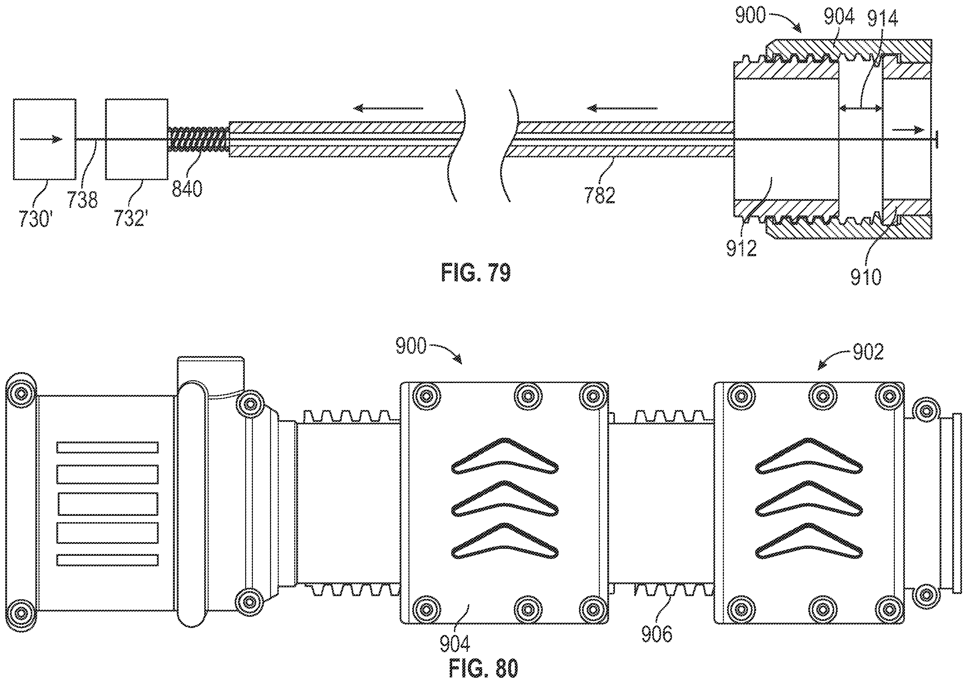

FIGS. 78 and 79 illustrate an exemplary operation of the actuator 900. Referring to FIG. 78, the lumen adaptor 912 and pull tether adaptor 910 are at a spacing 914 from each other. In FIG. 79, the control knob 904 has been rotated to increase the spacing 914 from each other. The pull tether adaptor 910 has been retracted to retract the pull tether 738 and accordingly deflect the distal slotted portion 722. The tube 782 and compression coil 840 have a distal compressive force applied by the lumen adaptor 912 that is transmitted to the first intermediate insert 732'. The proximal portions of the outer sheath 712 accordingly stiffen and a reduced possibility of cross talk exists. The control knob 904 may be rotated in an opposite direction to reduce the tension in the pull tether 738 and the compressive force against the insert 732'.

In examples, the threading and non-threading may be alternated such that the control knob 904 has a threaded engagement with the pull tether adaptor 910 and a non-threaded or fixed rotational coupling with the lumen adaptor 912.

The intermediate or secondary actuator assembly 902 may include the components of the distal or primary actuator assembly 900 and may operate in a similar manner. The intermediate or secondary actuator assembly 902, for example, may include a lumen adaptor 920 that abuts a proximal end portion of the tube 810, and a pull tether adaptor 922 that couples to a proximal end portion of the pull tether 740. Features of the distal or primary actuator assembly 900 may be utilized with the intermediate or secondary actuator assembly 902.

In examples, the configuration of the lumens may be varied such that compression coils extend along the length of the outer sheath 712 and contact the lumen adaptors 912, 920. In examples, the compression coils may be excluded and the tubes 782, 810 may extend distally to the inserts 732', 734'. Other configurations may be utilized in examples. In examples, the compression coils disclosed herein may comprise a compression tube with cuts that allow the compression tube to flex or deflect. The cut compression hypotubes may comprise lumens that may be utilized alternatively or in combination with the compression coils.

FIG. 80 illustrates an exterior side view of the actuator assemblies 900, 902 upon the second housing 906.

Any of the features of FIGS. 77-80 may be utilized solely or in combination with each other or any other example disclosed herein.

Referring to FIG. 65 and 66, the elongate shaft 710 or rail shaft or subassembly may be coupled to the second housing 686 (alternatively referred to as a rail housing). The first housing 684 may be slidable longitudinally or axially relative to the second housing 686. The first housing 684 may couple to other shafts or sheaths of the delivery system 680, such as the outer shaft sheath or subassembly 20, the mid shaft or mid shaft subassembly 24, the tether assembly or subassembly 26, the release assembly or subassembly 28, and the nose cone shaft or subassembly.

As disclosed herein, the elongate shaft 710 or rail shaft or subassembly may form a bend portion of the elongate shaft of the delivery catheter (comprising the combination of the assemblies 20, 24, 26, 28). The sliding motion of the first housing 684 relative to the second housing 686 may advance or retract the assemblies 20, 24, 26, 28 relative to the bend portion to vary the depth of the distal end portion of the elongate shaft of the delivery catheter relative to the bend portion of the elongate shaft 710 or rail shaft or subassembly. The first housing 684 and assemblies 20, 24, 26, 28 may be advanced distally relative to the second housing 686 to produce depth, and the first housing 684 and assemblies 20, 24, 26, 28 may be retracted proximally relative to the second housing 686 to reduce depth.

A knob assembly may be utilized to vary the depth of the distal end portion of the elongate shaft of the delivery catheter relative to the bend portion of the elongate shaft 710 or rail shaft or subassembly. A knob assembly may further be configured to actuate the height of the elongate shaft of the delivery catheter.

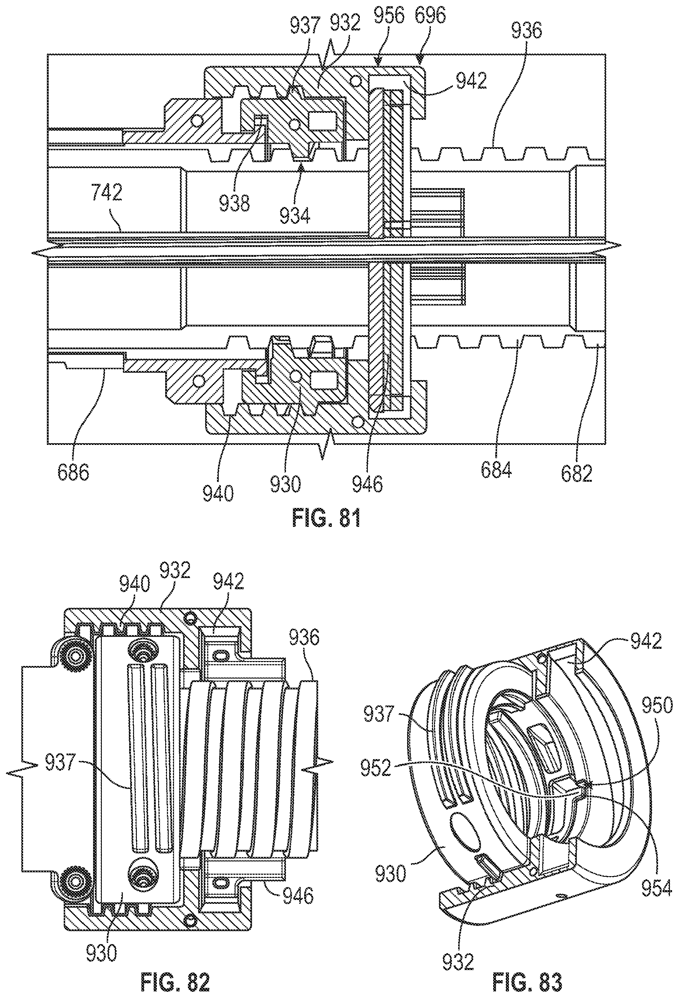

In examples, a knob assembly may be adapted to be rotated in a first direction to produce depth of the distal end portion of the elongate shaft of the delivery catheter relative to the bend portion of the elongate shaft 710 or rail shaft or subassembly. The knob assembly may be rotated in a second direction to produce height of the elongate shaft of the delivery catheter in a direction opposed to depth. FIGS. 81-104 illustrate examples of knob assemblies that may be utilized.

FIG. 81 illustrates a cross-sectional view of an example of a knob assembly 696. The knob assembly 696 is positioned on the handle 682. The knob assembly 696 may include a first portion 930 or interior body or longitudinally fixed body. The knob assembly 696 may include a second portion 932 or outer body or longitudinally slidable body. The first portion 930 may include interior threading 934 adapted to engage threading 936 on an outer surface of the first housing 684. The first portion 930 may include exterior threading 937 adapted to engage the second portion 932. The first portion 930 may include a fixed rotational coupling 938 to the second housing 686 such that the first portion 930 is longitudinally fixed relative to the second housing 686.

The first portion 930 is adapted to rotate such that the threaded engagement with the first housing 684 causes the first housing 684 to slide longitudinally relative to the second housing 686. As such, depth of the distal end portion of the delivery catheter varies relative to the bend portion of the elongate shaft 710 or rail shaft or subassembly.

The second portion 932 includes interior threading 940 adapted to engage the exterior threading 936 of the first portion 930. As such, rotation of the second portion 932 relative to the first portion 930 advances or retracts the second portion 932 relative to the first portion 930. The second portion 932 includes a fixed rotational coupling 942 with the pull tether adaptor 946 for the proximal pull tether 742 (marked in FIGS. 68 and 71 for example). The second portion 932 comprises a grip portion or control knob for the knob assembly 696 that is adapted for a user to grip and manipulate. An outer surface 956 of the second portion 932 is adapted for grip and manipulation.

A proximal pull tether assembly includes the proximal pull tether 742 and the pull tether adaptor 946. The distal end portion 820 of the proximal pull tether 742 couples to the elongate shaft of the delivery catheter, and particularly to the elongate shaft 710 or rail shaft or subassembly. The distal end portion 820 may couple to an insert 734, 734' as disclosed herein. The proximal end portion of the proximal pull tether 742 engages the pull tether adaptor 946. Proximal movement of the pull tether adaptor 946 produces height of the elongate shaft 710 or rail shaft or subassembly and accordingly the elongate shaft of the delivery system. Distal movement of the pull tether adaptor 946 reduces the tension and reduces the height.

The first portion 930 is engaged with the second portion 932 such that the first portion 930 rotates with the second portion 932, and the second portion 932 is adapted to release from the first portion 930 to allow the second portion 932 to rotate relative to the first portion 930. A catch mechanism 950 (marked in FIG. 83), for example, may be utilized to engage the first portion 930 with the second portion 932. The catch mechanism 950 may comprise a detent or other form of mechanism to retain the first portion 930 in rotational engagement with the second portion 932 and allowed to release. The catch mechanism 950 may be adapted to be overcome with a torque force. FIG. 83, for example, illustrates a form of the catch mechanism 950 including a protrusion 952 on the first portion 930 engaged with a recess 954 of the second portion 932 (alternatively the first portion 930 may include the recess and the second portion 932 may include the protrusion 952). The protrusion 952 may be deflectable and adapted to disengage from the recess 954 upon a sufficient torque force being applied to the catch mechanism 950.

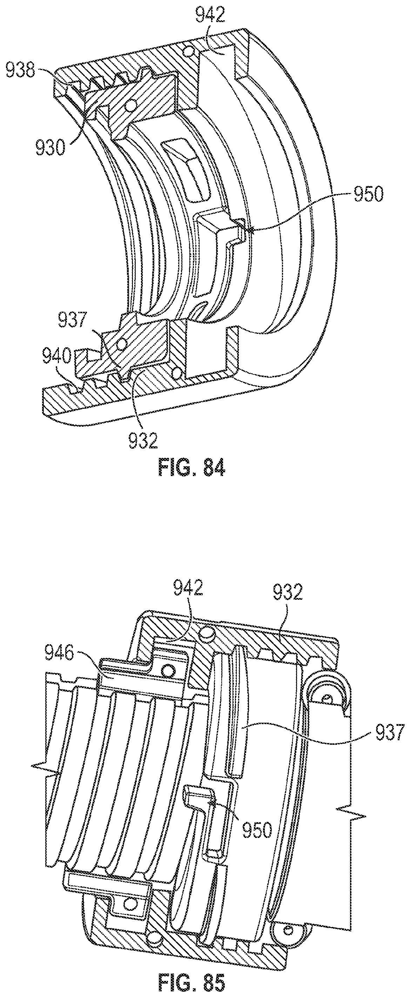

FIG. 82 illustrates a cross-sectional view of the second portion 932. FIG. 83 illustrates a perspective cross-sectional view of the second portion 932. FIG. 84 illustrates a perspective cross-sectional view of the second portion 932 and first portion 930. FIG. 85 illustrates a perspective cross-sectional view of the second portion 932 from an opposite side of the handle than shown in FIG. 82.

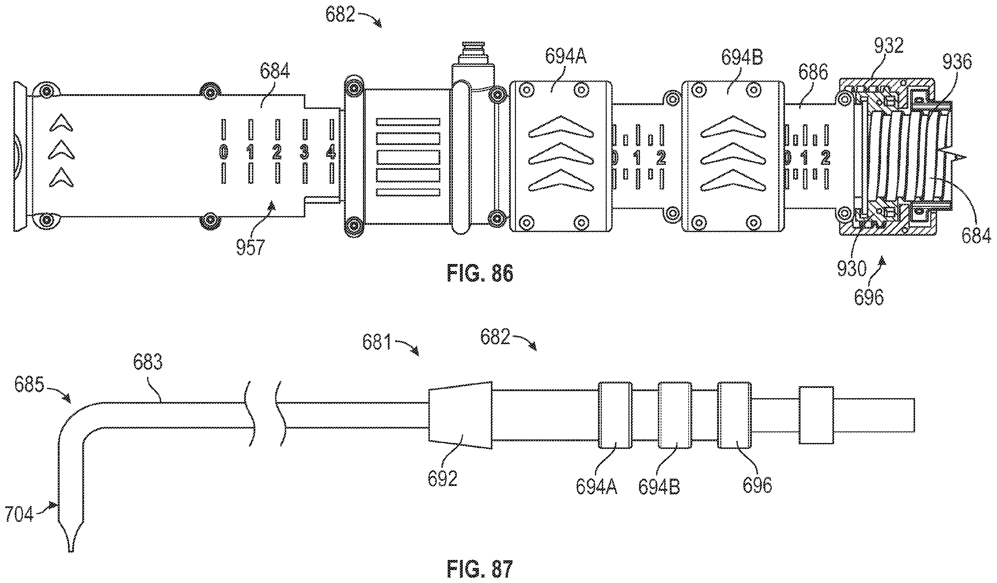

FIGS. 86-93 illustrate an exemplary operation of the knob assembly 696. The delivery catheter is shown in FIGS. 86 and 87 in a depth configuration, in which the assemblies 20, 24, 26, 28 of the elongate shaft 683 of the delivery catheter 681 are advanced distally relative to the bend portion 685 produced by the elongate shaft 710 or rail shaft or subassembly. The distal end portion 704 of the elongate shaft 683 is shown to extend distally or in a depth position relative to the bend portion 685. A corresponding indicator 957 on the handle 682 indicates the depth of the distal end portion 704, with the distal end of the second housing 686 serving as a datum point of the indicator 957.

In a configuration as shown in FIGS. 86 and 87, the knob assembly 696 may be rotated in a first direction to produce depth of the distal end portion 704 relative to the bend portion 685. The knob assembly 696 may be rotated such that the second portion 932 remains engaged with the first portion 930. The interior threading 934 of the first portion 930 longitudinally drives the threading 936 on the first housing 684 relative to the second housing 686 to produce the variation in depth. The assemblies 20, 24, 26, 28 of the elongate shaft 683 of the delivery catheter are advanced distally relative to the bend portion 685.

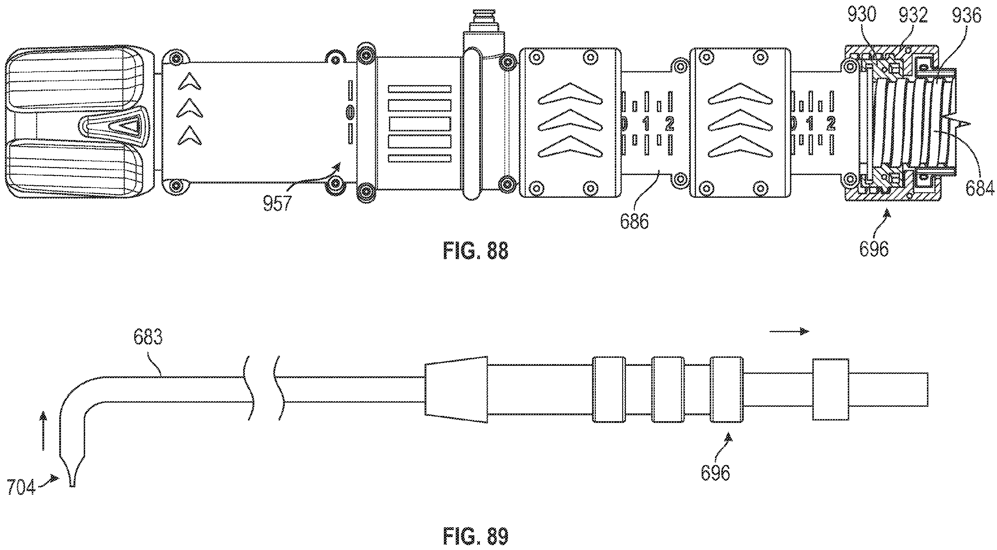

The knob assembly 696 may be rotated in a second opposite direction to reduce the depth of the distal end portion 704. For example, referring to FIGS. 88 and 89, the knob assembly 696 is rotated in the second direction to slide the first housing 684 proximal relative to the second housing 686. The assemblies 20, 24, 26, 28 of the elongate shaft 683 of the delivery catheter are retracted proximally relative to the bend portion 685 to reduce the depth.

In a configuration as shown in FIGS. 88 and 89, the first portion 930 remains engaged with the second portion 932 due to the coupling of the catch mechanism 950. As such, rotation of the second portion 932 continues to produce rotation of the first portion 930, which longitudinally drives the threading 936 on the first housing 684 relative to the second housing 686 to reduce the depth.

The rotation of the first portion 930 may continue until a desired point. The desired point may comprise a defined position of no depth (e.g., a zero point) as represented on the indicator 957 in FIG. 88. In examples, the rotation of the first portion 930 may be set to a desired point at which independent tension on the proximal pull tether 742 is desired to occur. Such a point may occur before the point of no depth or after, as desired. The tension on the proximal pull tether 742 deflects the proximal slotted portion 726 to produce height.

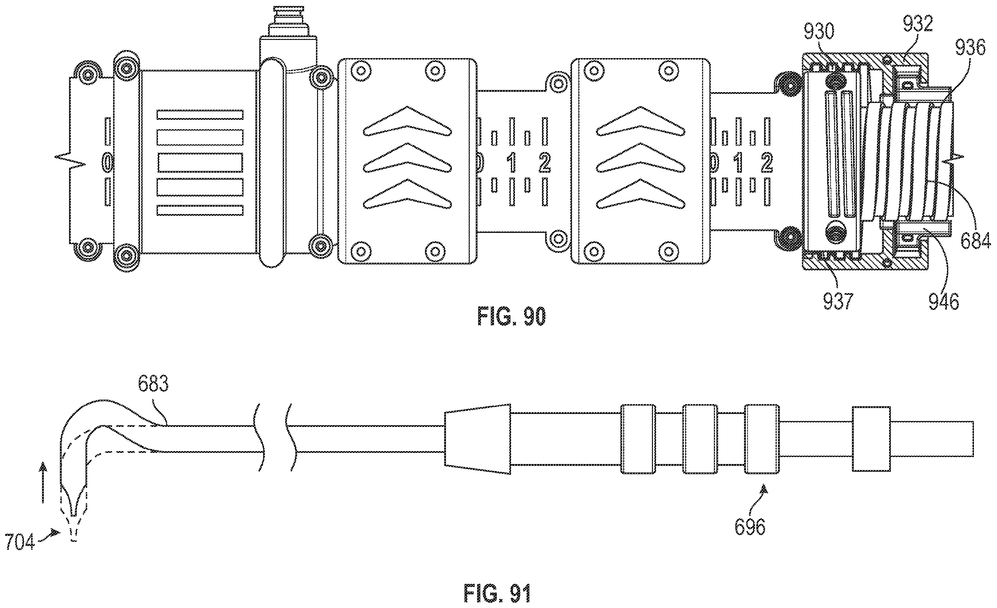

FIGS. 90 and 91, for example, illustrate a configuration in which the first portion 930 is impeded from further rotation. The cessation of rotation may be produced in a variety of manners and may comprise a cessation or stop point of the threading 936 on the first housing 684 in examples. The first portion 930 may contact a stop or be mechanically stopped from rotating, yet the user may continue to apply a rotation force to the second portion 932. The torque force applied overcomes the engagement of the catch mechanism 950, and the catch mechanism 950 releases the first portion 930 from the second portion 932. The second portion 932 is adapted to rotate relative to the first portion 930 along the exterior threading 937 of the first portion 930.

The second portion 932 may rotate to increase the spacing between the first portion 930 and the second portion 932. The pull tether adaptor 946 is driven proximally relative to the first portion 930 to produce tension in the proximal pull tether 742. The second portion 932 slides the adaptor 946 proximally relative to the handle 682 to produce the height. The height of the distal end portion 704 of the elongate shaft 683 increases as represented in FIG. 91. An upward bend in a direction opposed to the depth is produced.

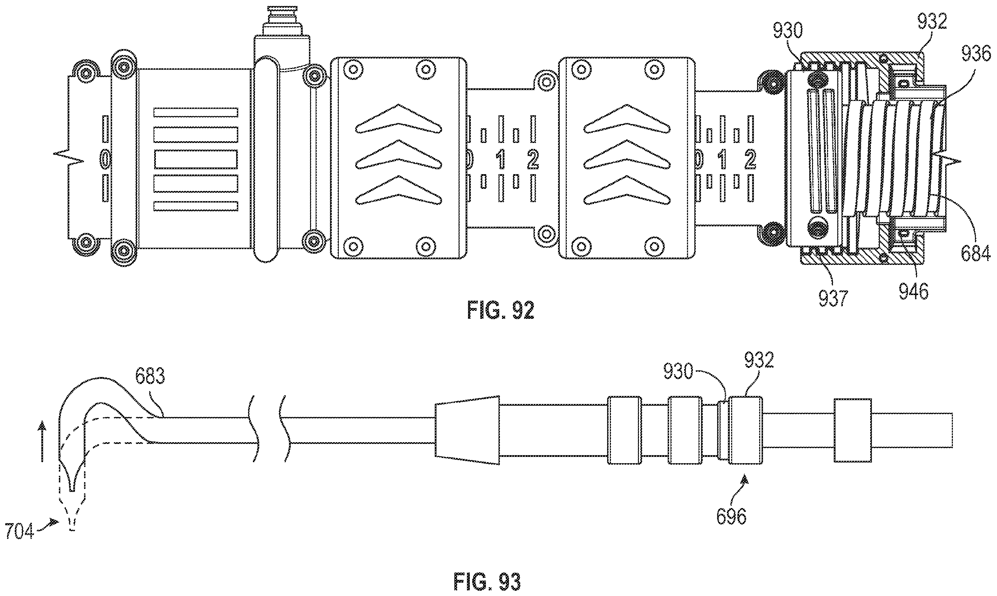

The second portion 932 may continue to be rotated independent of the first portion 930 for a desired distance. Referring to FIG. 92 for example, the second portion 932 may continue to be rotated to proximally retract the pull tether adaptor 946 and continue to increase the height of the distal end portion 704 of the elongate shaft 683.

The second portion 932 may be rotated in an opposite direction along the exterior threading 937 of the first portion 930 to reduce the height. The second portion 932 may be rotated until the second portion 932 reengages with the first portion 930. The catch mechanism 950 may reengage. A configuration as shown in FIGS. 88 and 89 results.

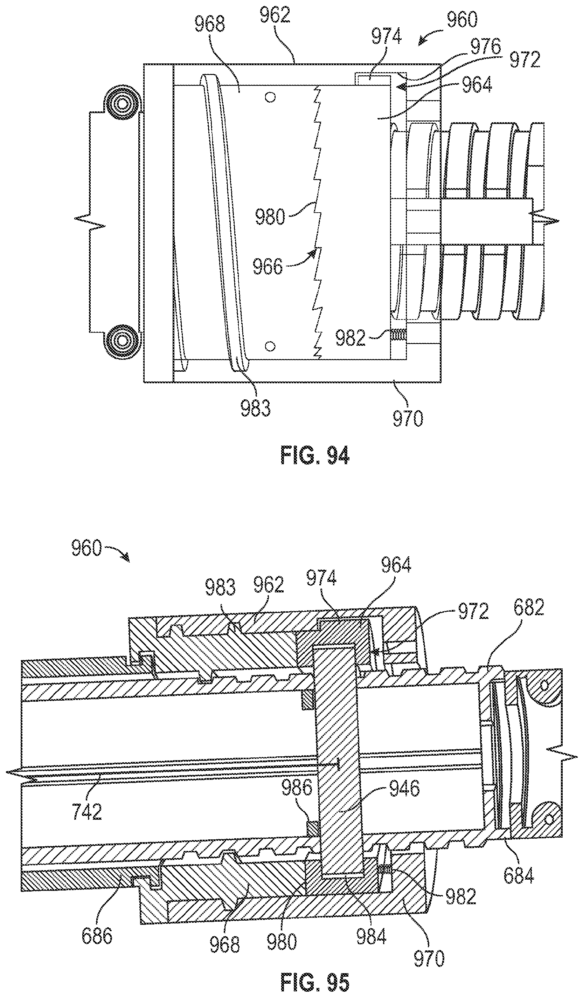

Variations in the configuration of the knob assembly 696 may be provided. FIGS. 94-98, for example, illustrate a variation in which the catch mechanism is adapted to be overcome with a longitudinal force.

FIG. 94, for example, illustrates a variation comprising a knob assembly 960 in which the second portion 962 includes a displacement body 964 or third body that is adapted to displace longitudinally to release the catch mechanism 966.

The first portion 968 of the knob assembly 960 includes the features of the first portion 930 of the knob assembly 696. The first portion 968 comprises a first body of the knob assembly 960. The second portion 962 of the knob assembly 960 includes an outer body 970 or second body of the knob assembly 960, and the displacement body 964. The outer body 970 surrounds a cavity 972 for the displacement body 964 to displace longitudinally within.

The displacement body 964 includes an alignment feature 974 that rotationally aligns the displacement body 964 with the outer body 970. The alignment feature 974, for example, comprises a tab (shown in FIG. 96) adapted to slide longitudinally within a slot 976 in the interior surface of the outer body 970.

The catch mechanism 966 may include ratchet surfaces 980 or friction surfaces on the displacement body 964 and first portion 968, and a spring body 982 pressing the ratchet surfaces 980 into engagement with each other longitudinally. The force of the spring body 982 is adapted to be overcome with a longitudinal force to release the ratchet surfaces 980 from each other.

The displacement body 964 includes a fixed rotational coupling 984 with the pull tether adaptor 946.

FIG. 95 illustrates a cross-sectional view of the knob assembly 960 upon the handle 682. The first housing 684 includes a stop 986 that is adapted to contact the pull tether adaptor 946 upon relative proximal movement of the first housing 684 relative to the pull tether adaptor 946.

In use, the spring body 982 presses the ratchet surfaces 980 together such that the outer body 970 rotates with the first portion 968. As such, rotation of the outer body 970 rotates the first portion 968 and correspondingly drives the first housing 684 relative to the second housing 686 to vary the depth as desired.

At a defined point in a reduction of depth (e.g., proximal movement of the first housing 684), the stop 986 contacts and presses the pull tether adaptor 946 with a proximal longitudinal force to overcome the force of the spring body 982. The catch mechanism 966 accordingly releases and the outer body 970 is able to rotate and move proximally independent of the first portion 968. FIG. 97, for example, illustrates the displacement of the displacement body 964 within the cavity 972 of the outer body 970.

The outer body 970 rotates about the exterior threading 983 of the first portion 968 to drive the pull tether adaptor 946 proximally. Height (as represented in FIGS. 90-93) is produced due to the proximal retraction of the pull tether adaptor 946. FIG. 98 illustrates the proximal movement of the outer body 970 relative to the first portion 968 to retract the pull tether adaptor 946. At a desired time, the outer body 970 may be rotated in an opposite direction to reduce the height and for the catch mechanism 966 to reengage. A configuration as shown in FIG. 95 results.

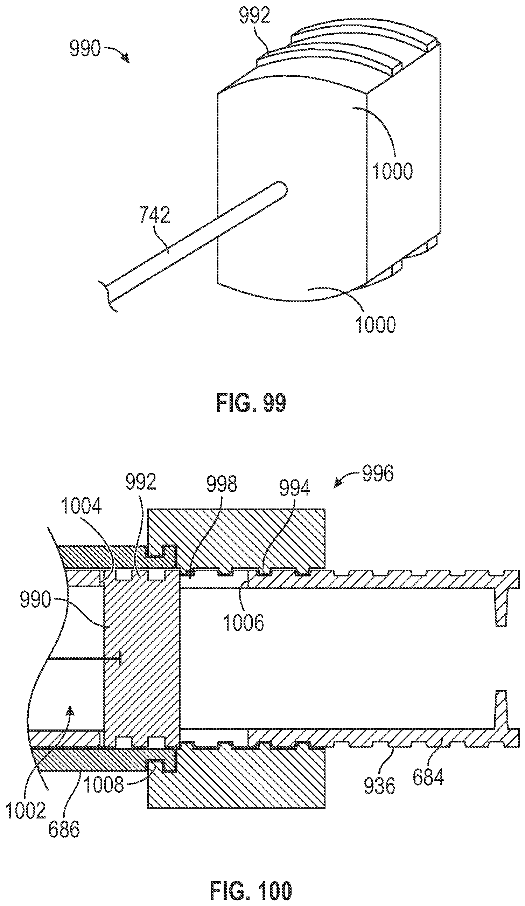

Variations in the configuration of the knob assembly 696 may be provided. FIGS. 99-104 illustrate a variation in which the pull tether adaptor 990 includes threading 992 for engaging the threading 994 (marked in FIG. 100) of the knob assembly 996.

FIG. 99 illustrates a perspective view of the pull tether adaptor 990. The pull tether adaptor 990 is shaped such that the threading 992 extends within cutouts or slots 998 (marked in FIG. 100) in the first housing 684. The pull tether adaptor 990 has an oblong shape, with the threading 992 positioned on the protruding ends 1000 of the pull tether adaptor 990. The threading 992 is positioned to be engaged by the threading 994 of the knob assembly 996.

Referring to FIG. 100, the pull tether adaptor 990 is shown in an interior cavity 1002 of the handle. The threading 992 extends within the slots 998. The first housing 684 includes stops 1004, 1006 comprising ends of the slots 998 that are adapted to contact and move the pull tether adaptor 990 at desired times.

The knob assembly 996 is illustrated in FIG. 100 with a fixed rotational coupling 1008 to the second housing 686 and a threaded coupling with the threading 936 of the first housing 684. The knob assembly 996 is axially fixed relative to the second housing 686.

The threaded coupling between the threading 994 of the knob assembly 996 and the threading 936 of the first housing 684 allows rotation of the knob assembly 996 to advance or retract the first housing 684 relative to the second housing 686 (and thereby vary the depth of the distal end portion of the delivery catheter). In a configuration as shown in FIG. 100, the knob assembly 996 is only threadably engaged with the first housing 684, thus allowing rotation of the knob assembly 996 to advance or retract the first housing 684. Upon retraction, the stop 1004 at the distal end of the slot 998 pushes the pull tether adaptor 990 proximally along with the first housing 684.

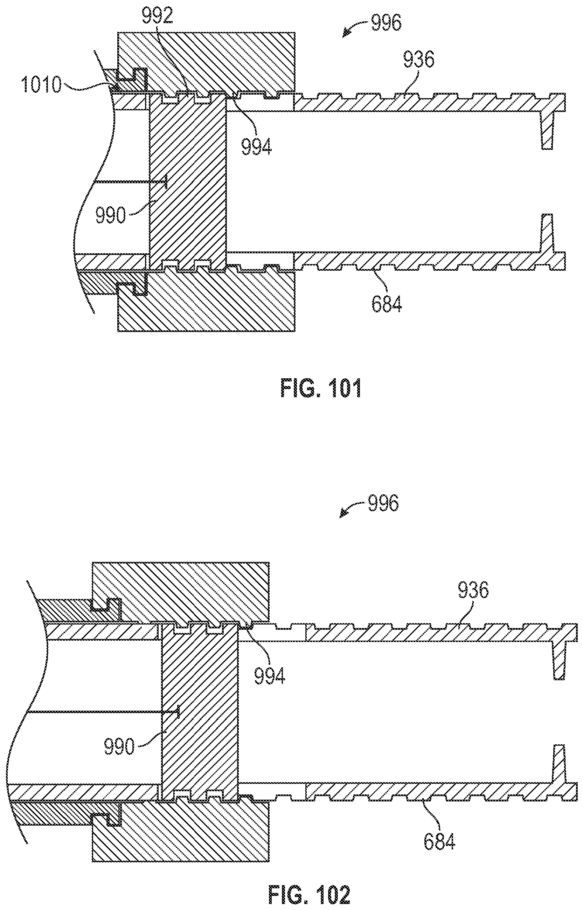

At a desired point, the threading 994 of the knob assembly 996 engages the threading 992 of the pull tether adaptor 990 as the pull tether adaptor 990 retracts proximally. FIG. 101, for example illustrates the engagement of both the threading 992 of the pull tether adaptor 990 and the threading 936 of the first housing 684 by the knob assembly 996. The pull tether adaptor 990 may be retracted proximally along with the first housing 684. In examples, the threading pitch of the pull tether adaptor 990 is the same as the threading pitch of the first housing 684.

The threading 936 of the first housing 684 ceases at a distal position 1010. The knob assembly 996 may continue to engage both the threading 992 of the pull tether adaptor 990 and the threading 936 of the first housing 684 until the first housing 684 retracts proximally to a distance that the threading 994 disengages from the threading 936 of the first housing 684. FIG. 102 illustrates the position prior to the threading 994 disengaging from the threading 936 of the first housing 684.

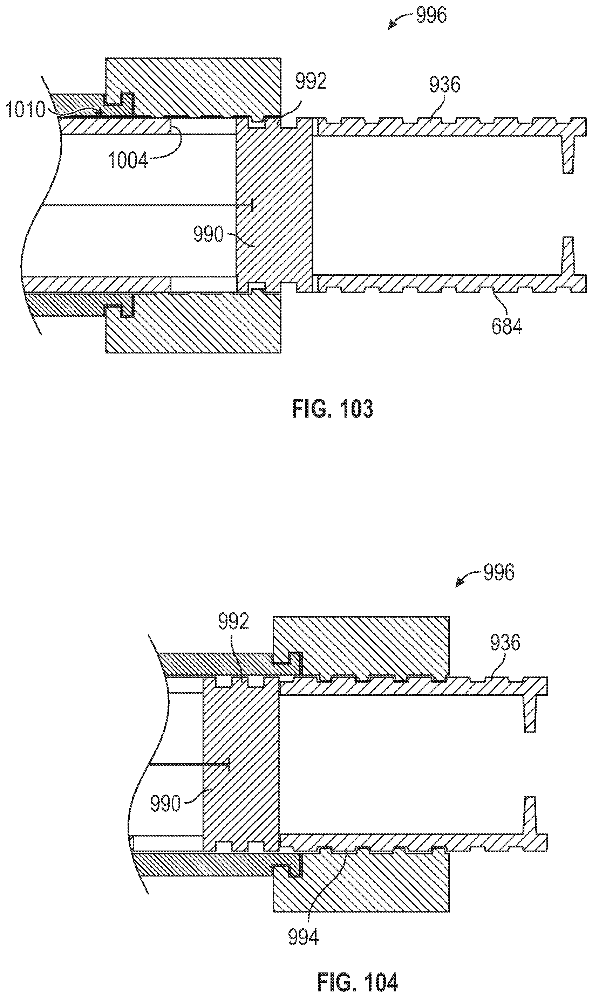

FIG. 103 illustrates the knob assembly 996 having disengaged from the threading 936 of the first housing 684. The threading 994 of the knob assembly 996 has moved to the distal position 1010 of the housing 684 that lacks threading. The knob assembly 996 continues to engage the pull tether adaptor 990 to retract the pull tether adaptor 990 relative to the first housing 684. Height (as represented in FIGS. 90-93) is produced due to the proximal retraction of the pull tether adaptor 990. The knob assembly 996 as such is adapted to alternatively engage the threading 936 of the first housing 684 and the threading 992 of the pull tether adaptor 990 to slide the adaptor 990 relative to the first housing 684. The threading 994 of the knob assembly 996 disengages from the threading 936 of the first housing 684 to cause the pull tether adaptor 990 to slide relative to the first housing 684.

The pull tether adaptor 990 may continue to be retracted to a desired point. To reduce the height, the knob assembly 996 may be rotated in an opposite direction. The pull tether adaptor 990 may advance distally until reaching the stop 1004. The pull tether adaptor 990 may press distally against the stop 1004 to push the first housing 684 distally until the threading 936 of the first housing 684 reengages with the threading 994 of the knob assembly 996.

The knob assembly 996 may return to a configuration as shown in FIG. 102. The knob assembly 996 may continue to rotate to produce depth, as represented in FIG. 104 for example. Variations in the configuration of the knob assemblies may be provided in examples herein. In examples, a separation mechanism such as a spring or magnet may be utilized to position the pull tether adaptor 990 prior to the stop 1004 pressing the pull tether adaptor 990 proximally. The separation mechanism may be overcome by the stop 1004 pressing the pull tether adaptor 990 proximally.

The knob assemblies may beneficially allow for a single knob assembly to operate both depth and height of the elongate shaft of the delivery catheter. A user may easily rotate in a first direction to produce depth and rotate in an opposite direction to reduce depth and produce height. Intuitive control of the depth and height may result. Additional knobs may not be required to vary depth and height separately. Further, the position or timing at which height is produced may be controlled utilizing the knob assemblies disclosed herein. The position or timing of release of a catch mechanism or of sliding motion of the pull tether adaptor relative to a housing may be set to produce a desired point that height occurs. Improved control of an implant deployment procedure may be produced.

Any of the features of FIGS. 86-104 may be utilized solely or in combination with each other or any other example disclosed herein.

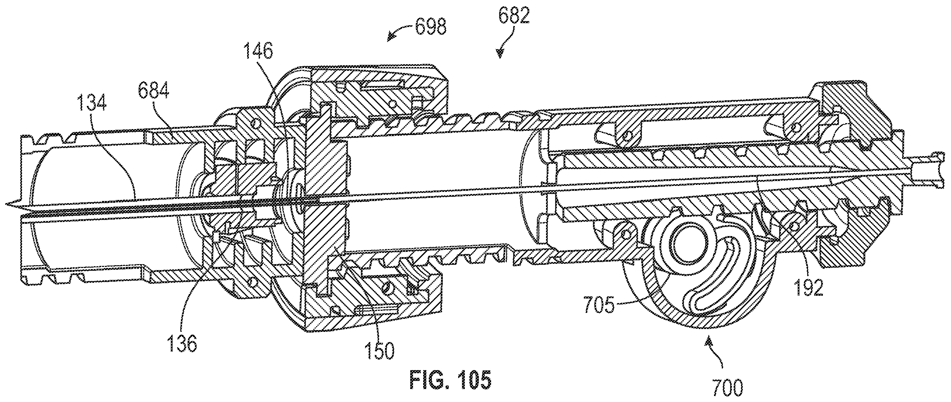

FIG. 105 illustrates a perspective cross-sectional view of the proximal end portion of the handle 682. The mid shaft adaptor 136 is coupled to the first housing 684 and is fixed in position relative to the first housing 684. The mid shaft or mid shaft subassembly 24 accordingly travels with the movement of the first housing 684.

The coupling tether adaptor 150 is coupled to the coupling tether actuator 698 in the form of a control knob. The coupling tether actuator 698 has a threaded coupling to the first housing 684 and a fixed rotational coupling to the coupling tether adaptor 150. Rotation of the coupling tether actuator 698 about the first housing 684 produces proximal or distal movement of the coupling tether adaptor 150, which may be relative to the mid shaft or mid shaft subassembly 24 or other shaft or sheaths of the delivery system 680 as desired. Expansion of the implant may be controlled with advancement (expansion) or retraction (contraction) of the coupling tether actuator 698 and the coupling tether adaptor 150 along the elongate shaft of the delivery catheter. Advancement may cause the implant to expand relative to the distal pusher 138 of the mid shaft or mid shaft subassembly 24 and retraction may cause the implant to retract into the distal pusher 138 of the mid shaft or mid shaft subassembly 24.

The retraction portion 192 or proximal end portion of the release assembly or subassembly 28 may couple to a release actuator 700 in the form of a control knob 701 (marked in FIG. 64). The control knob 701 may engage with the retraction portion 192 of the release assembly or subassembly 28 with a threaded connection to a ratchet mechanism 705. The ratchet mechanism 705 may allow for slight sequential rotation of the control knob 701 to gradually retract the release assembly or subassembly 28. The sequential rotation may allow for controlled release of the implant from the delivery system through sequential retraction of the release assembly or subassembly 28. The release tethers of the release assembly or subassembly 28 are retracted from the loop portion of the tether assembly. As such, the physician or technician is able to confirm proper placement of the implant with a slow retraction of the release assembly or subassembly 28 if desired. Other configurations may be utilized in examples.

In operation, the delivery catheter 681 may be entered into the patient's vasculature utilizing entry methods disclosed herein (e.g., transfemoral, transjugular, etc.). An entry as shown in FIG. 26 (e.g., transfemoral and percutaneous) may be utilized. Upon entry, the delivery catheter 681 may approach the right atrium via the inferior vena cava. Entry via the superior vena cava may be utilized in examples.

The rail shafts or subassemblies as disclosed herein may be utilized to deflect the elongate shaft or shaft assembly 683 of the delivery catheter 681. Upon approach to an implantation site, depth and/or height may be provided using mechanisms disclosed herein. Variations in depth and/or height may allow for navigation and accommodation of structure within the vasculature. For example, height may allow for enhanced alignment of the delivery catheter with a native tricuspid valve annulus. The height may be produced within a right atrium to aid in navigating the geometry of the right atrium. Various other deflections of the delivery catheter 681 may be utilized to position the delivery catheter in the desired orientation.

A distal tip of the delivery catheter 681 may be deflected to be aligned to be near perpendicular with the implantation site, as represented in FIG. 27 for example. The depth of the distal tip or distal end portion may be increased utilizing methods disclosed herein. The depth may position the distal tip to be at the annulus of the tricuspid valve (or the annulus of a mitral valve in mitral implantation).

At a desired point, the outer sheath shaft or subassembly may be retracted to at least partially expose a portion of an implant for deployment. A capsule of the outer sheath shaft or subassembly may be retracted. Ejection of the implant from the capsule occurs. FIG. 28, for example, illustrates a representative configuration. A proximal end portion of the implant may be retained within the distal pusher 138 of the mid shaft or mid shaft subassembly at this point. Distal or ventricular anchors of the implant may deploy to capture leaflets of the native valve as shown in FIG. 28 for example. Imaging (e.g., ultrasound and/or fluoroscopy) may be utilized to determine if capture has occurred. Deployment may continue upon visualization of the capture of the leaflets.

To produce further expansion of the implant, the coupling tether actuator 698 may be actuated to allow the coupling tethers 142 to expand for expansion of the implant. FIG. 18 and 29 illustrate a representative configuration. Imaging may confirm desired placement of the implant.

Upon confirmation that the implant is in a desired position (e.g., ventricular anchors or hook anchors are anchored with native valve leaflets and the implant is seated in the desired position within the annulus), the release actuator 700 may be actuated. As such, the implant may be ejected from the capsule while attached to the tether assembly (the coupling tethers 142) and then released from the tether assembly after confirming proper placement in the native valve (e.g., the native atrioventricular valve).

Prior to the release actuator 700 being actuated, the implant may be recaptured by retracting the coupling tethers 142. As such, during a deployment process, if the position of the implant is undesirable, then the coupling tethers 142 may be retracted. The implant may retract back into the distal pusher 138 of the mid shaft or mid shaft subassembly if desired. Repositioning of the implant may occur and then re-release of the implant may be provided, or the deployment sequence may be entirely aborted.

At a time of confirming proper placement, the release assembly or subassembly 28 may be retracted to release the implant from the delivery system. A configuration as shown in FIGS. 23 and 30 may result. The delivery system may be withdrawn with the implant remaining in position. FIG. 31, for example, illustrates a representative configuration. Other methods, systems, or apparatuses, may be utilized as desired. The method may be varied as desired. Other methods and features of a delivery system may be disclosed in International Application No. PCT/US2022/016150, filed on February 11, 2022, and titled "Delivery Systems for Replacement Heart Valves," and published as International Publication No. WO 2022/174057 on August 18, 2022.

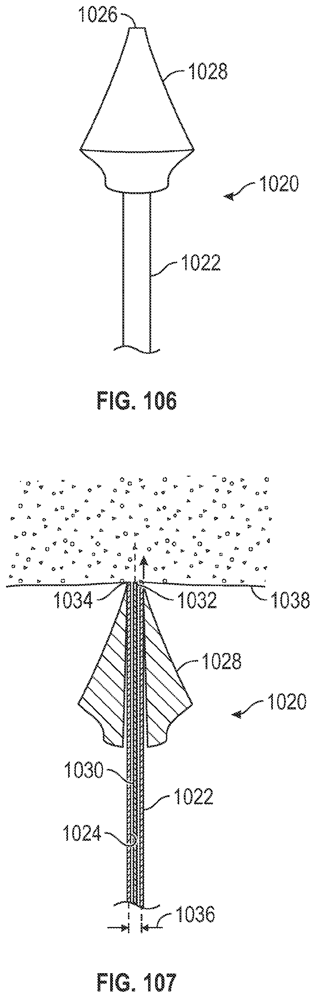

An interior guide wire lumen or sheath may further be provided as desired. FIG. 106, for example, illustrates a side view of a guide wire lumen or sheath 1020. The guide wire sheath 1020 includes a shaft 1022 having an interior lumen 1024 for passage of a guide wire therethrough (as marked in the cross-sectional view of FIG. 107). The interior lumen 1024 may extend to the tip 1026 of the nose body 1028 that is positioned at the distal end of the shaft 1022. The nose body 1028 may comprise a cone as shown in FIGS. 106 and 107, or may have other configurations in examples as desired.

The nose body 1028 may comprise the leading portion of the delivery catheter, which passes through the vasculature of the patient's body.

In examples, the guide wire 1030 (marked in FIG. 107) may be initially advanced to an implantation site (e.g., resting within a ventricle or other portion of a heart) and the delivery catheter may extend along the guide wire 1030 to the implantation site. The guide wire sheath 1020 slides along the guide wire 1030 (with the guide wire 1030 positioned in the interior lumen 1024) as the delivery catheter is advanced.

At a point in the procedure, it may be desirable to retract the guide wire 1030 proximally into the tip 1026 of the nose body 1028 such that the tip 1032 of the guide wire 1030 is within or proximate to the distal end 1034 of the guide wire sheath 1020. Such a feature may be utilized if it is desired to move or deflect the delivery catheter without interference from a guide wire 1030 protruding from the distal end 1034 of the guide wire sheath 1020.

An issue that may arise with retraction of the guide wire 1030 into or proximate the tip 1026 is later advancement or distal movement of the guide wire 1030. The diameter 1036 of the interior lumen 1024 of the shaft 1022 may be narrow or close to the diameter of the guide wire 1030. As such, advancement or distal movement of the guide wire 1030 relative to the distal end 1034 of the guide wire sheath 1020 may produce an inflexible tip 1032 of the guide wire 1030 because the interior lumen 1024 of the shaft 1022 constrains the guide wire 1030 to the longitudinal or axial shape of the interior lumen 1024. The guide wire 1030 (as marked in dashed lines in FIG. 107) accordingly may protrude with an inflexible tip 1032 that may puncture or otherwise damage surrounding tissue 1038 upon advancement of the guide wire 1030.

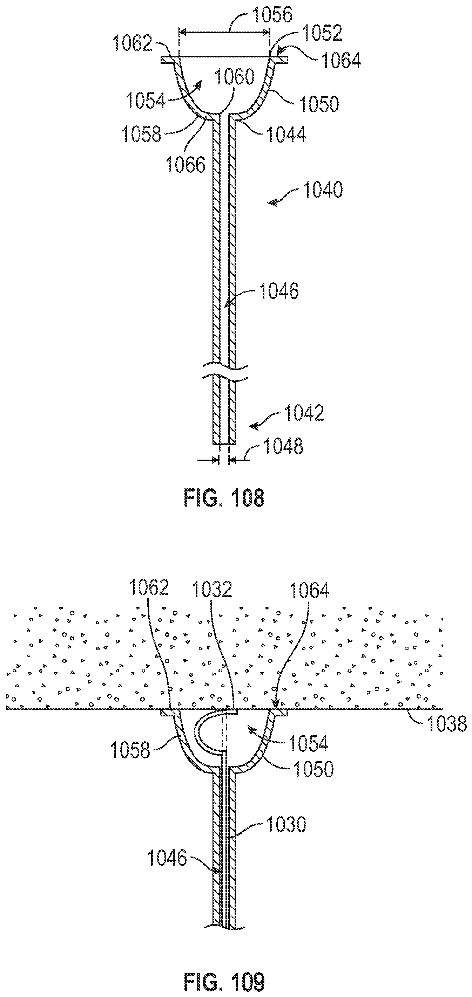

FIGS. 108-117 illustrate examples intended to address the issue of guide wire 1030 advancement or distal movement. FIG. 108, for example, illustrates a cross-sectional view of a guide wire sheath 1040 having a proximal end portion 1042 and a distal end 1044 and an interior lumen 1046 for passage of the guide wire 1030 therethrough. The interior lumen 1046 has a diameter 1048. The interior lumen 1046 is adapted for distal advance or proximal retraction of the guide wire 1030.

A spacer body 1050 is positioned at the distal end 1044 of the guide wire sheath 1040. The spacer body 1050 includes an opening 1052 for the guide wire 1030 to protrude therefrom.

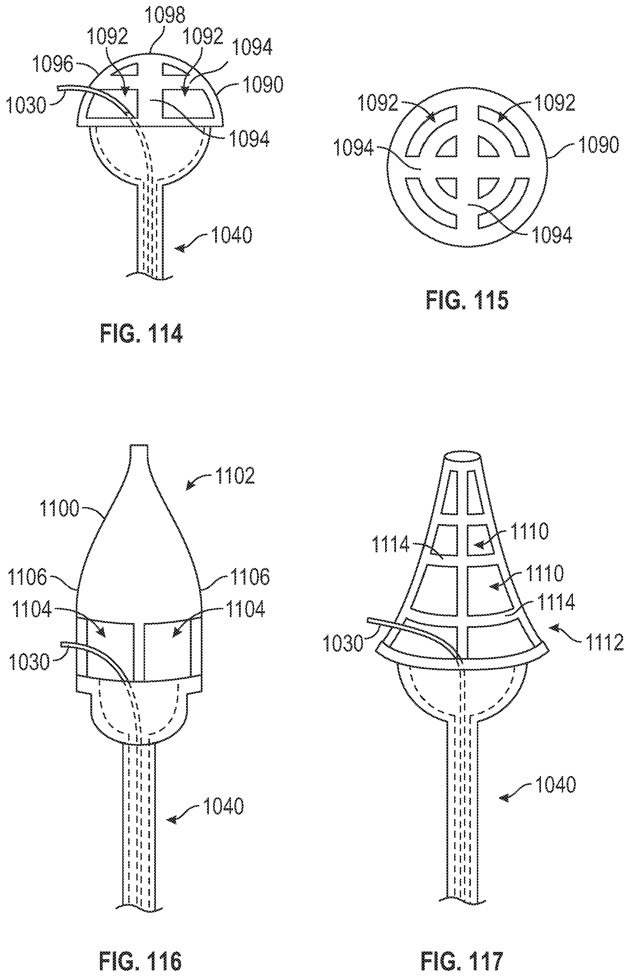

The spacer body 1050 has a cavity 1054 that has a diameter 1056 that is greater than the diameter 1048 of the interior lumen 1046 of the guide wire sheath 1040. The cavity 1054 is bound by an outer wall 1058 of the spacer body 1050, with the opening 1052 being positioned distal of an aperture 1060 of the spacer body 1050 for entry of the guide wire 1030 into the cavity 1054. The aperture 1060 may be continuous with the interior lumen 1046 of the guide wire sheath 1040 such that the guide wire 1030 passes from the interior lumen 1046 through the aperture 1060 and into the cavity 1054. The opening 1052 is positioned at the distal end 1062 of the spacer body 1050. The distal end 1062 may include a contact surface 1064 for abutment with a surface such as tissue located around an implantation site (e.g., heart wall tissue, chordae, leaflets, or other tissue). The spacer body 1050 may have a variety of shapes as desired, such as a dome shape as shown in FIGS. 108-115 or a conical shape as shown in FIGS. 116 and 117, among other shapes as desired (e.g., cuboid, rectangular, triangular, among others).

The distal end 1062 of the spacer body 1050 may be spaced axially or longitudinally from the proximal end 1066 of the spacer body 1050 such that the cavity 1054 extends for an axial or longitudinal distance from the distal end 1044 of the guide wire sheath 1040.

The cavity 1054 may have a variety of shapes and may comprise a dome shape as shown in FIGS. 108-113 or other shapes (e.g., conical, rectangular, oval, among others). The greater diameter 1056 of the cavity 1054 than the diameter 1048 of the interior lumen 1046 of the guide wire sheath 1040 allows the guide wire 1030 to deflect within the cavity 1054. The deflection may be a lateral deflection as represented in FIG. 109. As such, the restraint of the distal end or tip 1032 of the guide wire 1030 by the interior lumen 1024 may be excluded.

FIG. 109 illustrates a cross-section view of an exemplary operation of the spacer body 1050. The distal end 1062 or contact surface 1064 of the spacer body 1050 may contact or be proximate the tissue 1038. The guide wire 1030 may be advanced distally through the interior lumen 1046. The tip 1032 of the guide wire 1030, upon contact with the tissue 1038, is able to deflect laterally or buckle within the cavity 1054 such that the tip 1032 of the guide wire 1030 does not puncture or otherwise damage the tissue 1038. The distal end 1062 or contact surface 1064 may be backed away or spaced from the surface of the tissue 1038 such that the tip 1032 of the guide wire 1030 may slide laterally along the surface of the tissue 1038 to avoid puncture of the tissue 1038. As such, reduce possibility of puncture or damage may result. A similar result occurs if the distal end 1062 or contact surface 1064 is spaced from the surface of the tissue 1038 and the guide wire 1030 is advanced to contact the tissue 1038. The cavity 1054 provides the space for deflection.

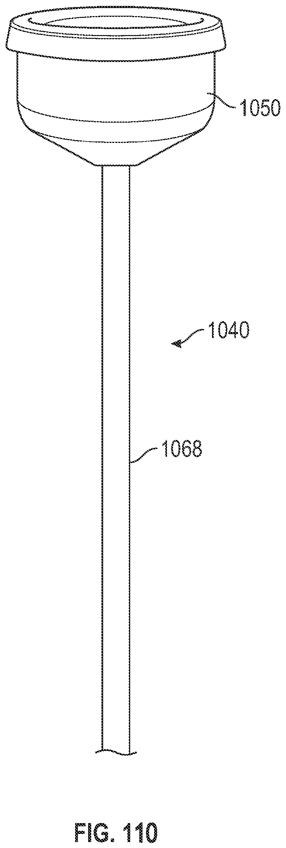

FIG. 110 illustrates a side perspective view of the shaft 1068 of the guide wire sheath 1040 coupled to the spacer body 1050. The spacer body 1050 may comprise a nose body of the delivery catheter, comprising a leading portion or tip of the delivery catheter. The shaft 1068 of the guide wire sheath 1040 may extend proximally along the elongate shaft of the delivery catheter and through the handle of the delivery catheter.

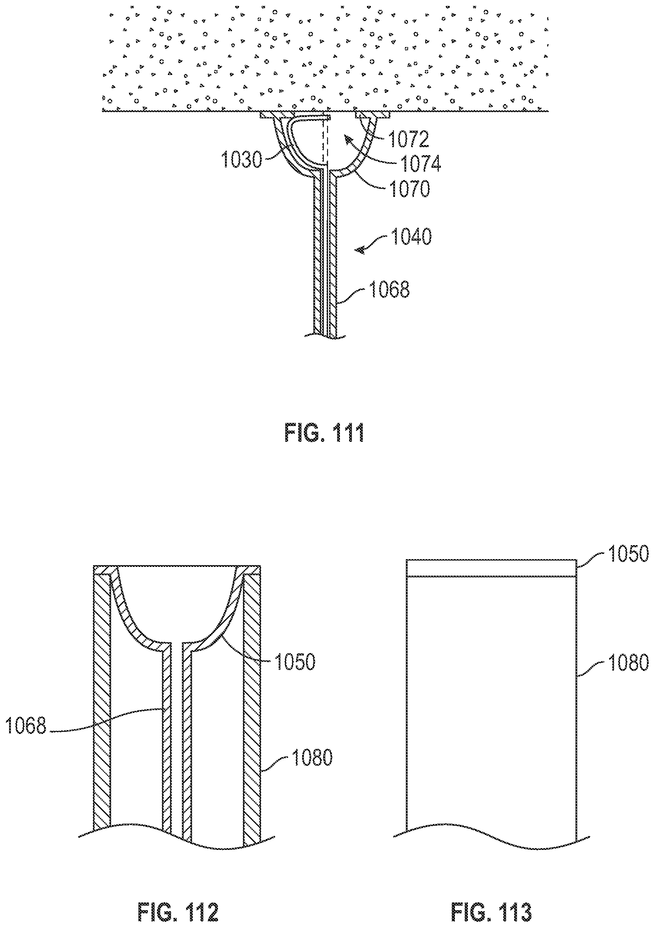

Variations in the configuration of the spacer body 1050 may be provided. FIG. 111, for example, illustrates a variation in which the spacer body 1070 includes a ridge 1072 extending radially inwards towards the cavity 1074. The ridge 1072 may aid the guide wire 1030 to deflect laterally by providing a laterally extending surface in the interior of the cavity 1074 for the guide wire 1030 to rest upon.

FIG. 112 illustrates a variation in which a sheath 1080 surrounds the shaft 1068 of the guide wire sheath 1040 and abuts the spacer body 1050. The sheath 1080 may stabilize the spacer body 1050 from lateral deflection. FIG. 113 illustrates a side view of such a configuration.

FIG. 114 illustrates a variation in which the spacer body 1090 includes a plurality of openings 1092 for the guide wire to protrude therefrom. The spacer body 1090 may include at least one rib 1094 separating the plurality of openings 1092.

In examples, one or more of the openings 1092 may be positioned on a side wall 1096 of the spacer body 1090. The side walls 1096 may extend distally to a distal end 1098 of the spacer body 1090. The guide wire 1030 may protrude laterally from the openings 1092 to allow for lateral exit of the guide wire 1030 from the spacer body 1090 to further reduce the possibility of puncture or other damage to tissue from axial advancement of the guide wire 1030. FIG. 115 illustrates an end view of the spacer body 1090 shown in FIG. 114.

FIG. 116 illustrates a variation in which the distal end 1100 of the spacer body 1102 comprises a conical tip, with the openings 1104 on the side walls 1106 of the spacer body 1102. The guide wire 1030 laterally exits from the spacer body 1102. FIG. 117 illustrates a variation in which a plurality of openings 1110 are provided along a conical spacer body 1112 for lateral exit of the guide wire 1030. The openings 1110 may be separated by ribs 1114.

Further variations in the configuration of the spacer bodies may be provided in examples. Furthermore, it will be understood that any of the features of FIGS. 106-117 may be utilized solely or in combination with each other or any other example disclosed herein.

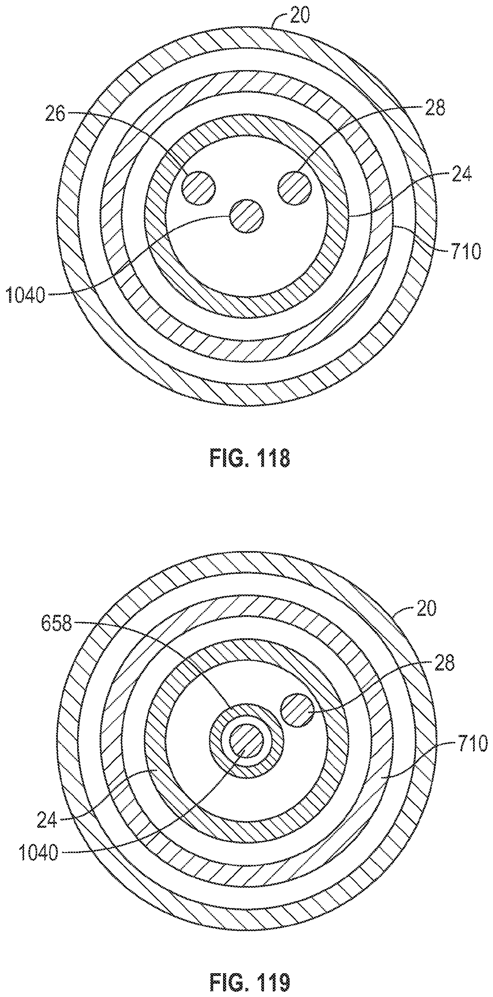

The sheaths or shafts or assemblies of the delivery catheter 681 may be arranged as shown in the transverse cross-sectional view of FIG. 118. The guide wire sheath 1040 or nose cone shaft or subassembly may comprise the innermost sheath or layer, with the release assembly or subassembly 28 extending adjacent to the guide wire sheath 1040. The tether assembly or subassembly 26 may extend adjacent to the guide wire sheath 1040. The mid shaft or mid shaft subassembly 24 may surround such inner assemblies. The rail shaft or subassembly or elongate shaft 710 may surround the mid shaft or mid shaft subassembly 24. The outer sheath shaft or subassembly 20 may surround the rail shaft or subassembly or elongate shaft 710. Other configurations of sheaths or shafts, or assemblies disclosed herein may be utilized in a configuration as shown in FIG. 118. Variations in the order of the sheaths or shafts or assemblies may be provided.

FIG. 119, for example, illustrates a variation according to a configuration as shown in FIGS. 60A-61. The flexible retention tether 658 surrounds the guide wire sheath 1040 or nose cone shaft or subassembly. The release assembly or subassembly 28 extends exterior to the flexible retention tether 658 (or may extend interior in examples). Other configurations may be utilized in examples.

FIG. 120 illustrates a configuration of a stabilizer assembly 1120 for a delivery catheter that may be utilized in examples herein. The delivery catheter 681 may be stabilized upon the stabilizer assembly 1120 in a configuration as shown in FIG. 62 for example. The stabilizer is for coupling with the handle of the delivery catheter to stabilize the delivery catheter during deployment of the prosthetic heart valve to a native valve (e.g., a native atrioventricular valve).

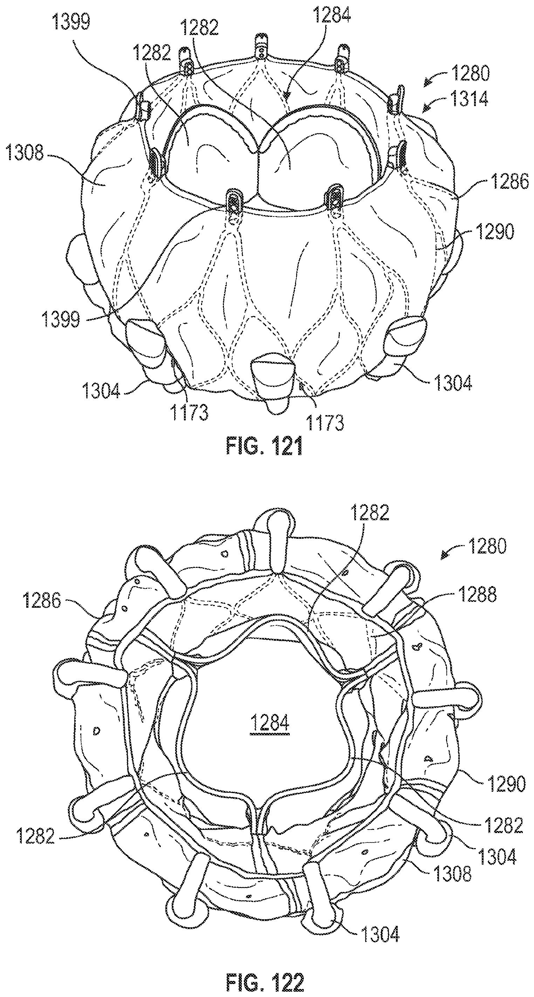

FIGS. 121-123 illustrate an exemplary prosthetic valve that may be implanted in a body using delivery systems and/or features described herein. Additional details of implants or prosthetic valves that may be utilized are disclosed in U.S. Provisional Application No. 63/436,051, filed December 29, 2022, and U.S. Provisional Application No. 63/533,458, filed August 18, 2023.

FIG. 121 illustrates a perspective view of a prosthetic valve 1280 that may be utilized in any example herein. The prosthetic valve 1280 may include the features of any other prosthetic valve or example disclosed herein.

The prosthetic valve 1280 includes one or more prosthetic valve leaflets 1282. The prosthetic valve leaflets 1282 may be positioned within a flow channel 1284 of the prosthetic valve 1280. The prosthetic valve leaflets 1282 are supported by a valve body 1286 and may extend radially inward from the valve body 1286 in the flow channel 1284. The prosthetic valve leaflets 1282 are disposed within a lumen of the valve body 1286 for allowing one-way flow.

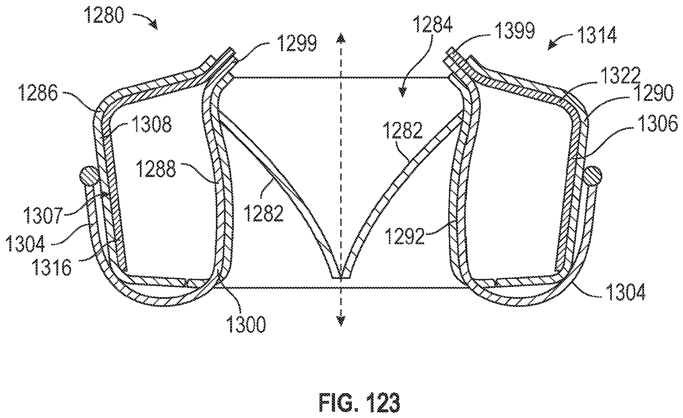

The valve body 1286 may include an inner body 1288 (marked in FIG. 122) and an outer body 1290. The inner body 1288 and/or outer body 1290 preferably take the form of collapsible and re-expandable metallic frames. Each frame may comprise a self-expanding frame, which may be made of a shape memory material (e.g., nitinol). The inner body 1288 may include the features of other examples of inner bodies disclosed herein unless stated otherwise. The inner body 1288 may include an inner frame 1292 (marked in FIG. 123). The inner frame 1292 supports the prosthetic valve leaflets 1282. The inner frame 1292 may include a plurality of struts that may be separated by spaces or openings. The struts are preferably arranged to form rows of connected cells.

The outer body 1290 may comprise a sealing body, and may include an outer frame 1306 (marked in FIG. 123) and a sealing skirt 1308, outer skirt, or fabric skirt positioned upon the outer frame 1306. The outer frame 1306 is positioned radially outward of the inner frame 1292. An outer surface 1307 of the outer frame 1306 faces radially outward from the prosthetic valve 1280. The outer surface 1307 is for pressing against tissue of the native heart valve. The sealing skirt 1308 extends along the outer surface 1307 of the outer frame 1306.

A proximal end portion 1314 of the outer frame 1306 may be coupled to a proximal end portion 1299 of the inner frame 1292.

In examples, the outer frame 1306 has a tapered shape such that a downstream portion or distal end portion 1316 has a smaller diameter than an intermediate portion 1322 of the outer frame 1306. The intermediate portion 1322, for example, may have a diameter in a range of about 35 millimeters to about 60 millimeters, with the distal end portion 1316 having a lesser diameter.

The outer frame 1306 may include one or more grip features 1173 (marked in FIG. 121) disposed along the outer surface. Features of the grip features are disclosed in U.S. Provisional Application No. 63/436,051, filed December 29, 2022, and U.S. Provisional Application No. 63/533,458, filed August 18, 2023. The grip features may comprise barbs (or may have any other form capable of engaging surrounding tissue). The barbs are preferably disposed along the struts of the outer frame 1306. The barbs may extend through the sealing skirt 1308 for penetrating the tissue of the native heart valve.

The anchors 1304 are shaped for placement behind native valve leaflets. The anchors may be formed with hook shapes or may be hook arm anchors or ventricular anchors. The anchors 1304 may include the features of any other anchors disclosed herein. The anchors 1304 may be coupled to the distal end portion 1300 of the inner frame 1292. The anchors 1304 are adapted to hook over (i.e., extend around) a native valve leaflet to help anchor the prosthetic valve within the native valve. The capsule of the delivery system may retract to allow the anchors 1304 to rotate in position to press the native valve leaflets against the outer frame 1306.

The outer frame 1306 may include one or more couplers 1399 in the form of eyelets or suture eyelets for coupling with the coupling tethers as disclosed herein. The coupling tethers may pass through the suture eyelets in a configuration as shown in FIGS. 18 and 20 for example. Nine couplers 1399 are described in examples; however, a greater or lesser number may be utilized as desired. The couplers 1399 may be positioned at an inlet end portion of the prosthetic valve 1280. The couplers 1399 may be positioned at strut arm ends or tabs of the frame of the prosthetic valve 1280.

The prosthetic valve 1280 may be expanded at an implantation site and released from the delivery system utilizing any of the method disclosed herein. The frames of the prosthetic valve 1280 may be self-expanding and may be made of a shape memory material (e.g., nitinol) in examples. Other materials may be utilized in examples. The prosthetic valve 1280 may be adapted to be restrained in a compressed configuration and then released to self-expand at an implantation site.

The examples of prosthetic valves may be utilized in a tricuspid valve as disclosed herein or may be utilized in other deployment locations such as a native mitral valve, or other deployment locations. Deployment to aortic or pulmonary valves, or other implantation sites are also contemplated and are considered to be within the scope of the present disclosure.

The delivery systems disclosed herein may be utilized with any example disclosed herein.

Various modifications of the examples disclosed herein may be provided. Features of examples may be modified, substituted, excluded, or combined across examples as desired. Combinations of features across examples may be provided as desired. Combinations of features may be provided across examples with other features of such examples being excluded if desired.

The various examples of sealing skirts disclosed herein may have a variety of forms, including cloth skirts, foam skirts, or braided skirts as desired. Various materials may be utilized as desired.

The implants disclosed herein may include prosthetic heart valves or other forms of implants, such as stents or filters, or diagnostic devices, among others. The implants may be expandable implants configured to move from a compressed or undeployed state to an expanded or deployed state. The implants may be compressible implants configured to be compressed inward to have a reduced outer profile and to move the implant to the compressed or undeployed state.

Various forms of delivery apparatuses may be utilized with the examples disclosed herein. The delivery apparatuses as disclosed herein may be utilized for aortic, mitral, tricuspid, and pulmonary replacement and repair as well. The delivery apparatuses may comprise delivery apparatuses for delivery of other forms of implants, such as stents or filters, or diagnostic devices, among others.

The implants and the systems disclosed herein may be used in transcatheter mitral or tricuspid implantation, as well as transcatheter aortic valve replacement (TAVR) or replacement of other native heart valves (e.g., pulmonary valves). The delivery apparatuses and the systems disclosed herein may be utilized for transarterial access, including transfemoral access, to a patient's heart. When used for transcatheter mitral valve replacement, the delivery procedure is preferably performed using a transseptal delivery technique wherein the delivery catheter is advanced into the right atrium, through a hole in the septum and then into the left atrium for accessing the native mitral valve. The delivery apparatuses and systems may also be utilized in other transcatheter percutaneous procedures, including transarterial procedures, which may be transfemoral or transjugular. Transapical procedures, among others, may also be utilized. Other procedures may be utilized as desired.

In addition, the methods herein are not limited to the methods specifically described and may include methods of utilizing the systems and apparatuses disclosed herein. The steps of the methods may be modified, excluded, or added to, with systems, apparatuses, and methods disclosed herein. The examples disclosed herein may comprise systems for implantation within a human body in examples.

For purposes of this description, certain aspects, advantages, and novel features of the examples of this disclosure are described herein. The disclosed methods, apparatuses, and systems should not be construed as limiting in any way. Instead, the present disclosure is directed toward all novel and nonobvious features and aspects of the various disclosed examples, along and in various combinations and sub-combinations with one another. The methods, apparatuses, and systems are not limited to any specific aspect or feature or combination thereof, nor do the disclosed examples require that any one or more specific advantages be present or problems be solved. Features, elements, or combinations of one example can be combined into other examples herein.

In closing, it is to be understood that although aspects of the present specification are highlighted by referring to specific examples, one skilled in the art will readily appreciate that these disclosed examples are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings herein. Lastly, the terminology used herein is for the purpose of describing particular examples only, and is not intended to limit the scope of systems, apparatuses, and methods as disclosed herein. Accordingly, the systems, apparatuses, and methods are not limited to that precisely as shown and described.

Certain examples of systems, apparatuses, and methods are described herein, including the best mode known to the inventors for carrying out the same. Of course, variations on these described examples will become apparent to those of ordinary skill in the art upon reading the foregoing description. The inventor expects skilled artisans to employ such variations as appropriate, and the inventors intend for the systems, apparatuses, and methods to be practiced otherwise than specifically described herein. Moreover, any combination of the above-described examples in all possible variations thereof is encompassed by the systems, apparatuses, and methods unless otherwise indicated herein or otherwise clearly contradicted by context.

Groupings of alternative examples, elements, or steps of the systems, apparatuses, and methods are not to be construed as limitations. Each group member may be referred to and claimed individually or in any combination with other group members disclosed herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses an approximation that may vary, yet is capable of performing the desired operation or process discussed herein.

The terms "a," "an," "the" and similar referents used in the context of describing the systems, apparatuses, and methods (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the systems, apparatuses, and methods and does not pose a limitation on the scope of the systems, apparatuses, and methods otherwise claimed.

## Claims

1. A system for replacing the function of a native atrioventricular valve, the system comprising:
a prosthetic heart valve (176) including a self-expanding frame and a plurality of leaflets disposed within a lumen of the frame for allowing one-way flow of blood;
a delivery catheter (12) having an elongate shaft (18) for advancing the prosthetic heart valve (176) through a patient's vasculature to the native atrioventricular valve, the elongate shaft (18) including a capsule (39) along a distal end portion of the elongate shaft (18) for maintaining the prosthetic heart valve (176) in a compressed state;
a tether assembly (26) extending through the elongate shaft (18) of the delivery catheter (12), the tether assembly (26) including a plurality of coupling tethers (142a-c) disposed along a distal end portion of the tether assembly (26), each of the coupling tethers (142a-c) comprising a loop portion (188a-c), with the loop portions (188a-c) extending through respective openings (193a-c) in the prosthetic heart valve (176); and
a release assembly (28) including one or more flexible release tethers (180) configured to extend through loop portions (188a-c) of the coupling tethers (142a-c) to secure the prosthetic heart valve (176) to the tether assembly (26), wherein the one or more release tethers (180) are retractable for removal from the one or more loop portions (188a-c) and thereby allowing the tether assembly (26) to be released from the prosthetic heart valve (176);
wherein the prosthetic heart valve (176) may be ejected from the capsule (39) and allowed to expand and operate while still attached to the tether assembly (26) and wherein the prosthetic heart valve (176) may be released from the tether assembly (26) after confirming proper deployment and operation within the native atrioventricular valve;
wherein the tether assembly (26) further comprises:
a tether manifold (144) attached to the plurality of coupling tethers (142a-c); and
a flexible retention tether (146) coupled to the tether manifold (144) and extending proximally from the tether manifold (144) for engaging a tether actuator (698);
and/or wherein the release assembly (28) further comprises:
a release tether manifold (182) that a plurality of the release tethers (180) branches out from; and
a retractable tether (184) coupled to the release tether manifold (182) and extending proximally from the release tether manifold (182) for engaging a release actuator (700).

2. The system of claim 1, wherein the tether manifold (144) comprises a wire forming a loop, and the plurality of coupling tethers (142a-c) loop around the loop of the tether manifold (144);
preferably wherein the plurality of coupling tethers (142a-c) comprises a continuous suture looped multiple times around the loop of the tether manifold (144) to form each of the plurality of coupling tethers (142a-c).

3. The system of any of claims 1 or 2, wherein the flexible retention tether (146) is woven or braided.

4. The system of any of claims 1-3, wherein each of the release tethers (180) extends through at least three of the loop portions (188a-c) for securing the loop portions (188a-c) to the prosthetic heart valve (176); and/or
wherein the release assembly (28) includes at least three of the release tethers (180) or only one release tether (180).

5. The system of any of claims 1-4, wherein the capsule (39) is retractable for ejecting the prosthetic heart valve (176) from the capsule (39).

6. The system of any of claims 1-5, wherein the elongate shaft (18) includes a plurality of shafts, at least one of the plurality of shafts comprising a rail shaft (22) adapted to form a bend portion for bending other of the shafts of the elongate shaft (18).

7. The system of claim 6, wherein the rail shaft (22) is adapted to bend in a first direction in a first plane and in a second direction in a second plane, the second plane extending transverse to the first plane;
preferably wherein the rail shaft (22) is adapted to slide relative to the capsule (39) to vary a depth of the capsule (39) relative to the bend portion, and the rail shaft (22) is adapted to bend in a third direction in the first plane, the third direction being opposed to the first direction to vary a height of the capsule (39);
more preferably wherein the delivery catheter (12) includes a handle (682) positioned at a proximal end portion of the elongate shaft (18), the handle (682) including a knob assembly for actuating a pull tether for bending the rail shaft (22) in the third direction to vary the height of the capsule (39).

8. The system of claim 7, wherein the delivery catheter (12) includes a handle (682) positioned at a proximal end portion of the elongate shaft (18), the handle (682) including a deflection actuator (694) adapted to deflect the elongate shaft (18) in the first direction in the first plane.

9. The system of any of claims 1-8, wherein the delivery catheter (12) includes a handle (682) positioned at a proximal end portion of the elongate shaft (18), the handle (682) including a tether actuator (698) for advancing or retracting the tether assembly (26) along the elongate shaft (18).

10. The system of claim 9, wherein the tether actuator (698) is adapted to advance the tether assembly (26) to expand the prosthetic heart valve (176) from a distal pusher (138) of the elongate shaft (18).

11. The system of claim 10, wherein the distal pusher (138) compresses an inlet end portion of the prosthetic heart valve (176).

12. The system of claim 10 or claim 11, wherein the tether actuator (698) is adapted to retract the tether assembly (26) to retract the prosthetic heart valve (176) into the distal pusher (138).

13. The system of any of claims 1-12, wherein the delivery catheter (12) includes a handle (682) positioned at a proximal end portion of the elongate shaft (18), the handle (682) including a release actuator (700) operable to retract the release assembly (28) to retract the one or more release tethers (180) from the one or more loop portions (188a-c).

14. The system of any of claims 1-13, wherein the delivery catheter (12) includes a handle (682) positioned at a proximal end portion of the elongate shaft (18), and wherein the system further comprises a stabilizer (1120) for coupling to the handle (682) for stabilizing the delivery catheter (12) during deployment of the prosthetic heart valve (176) to the native atrioventricular valve.

15. The system of any of claims 1-14, wherein the prosthetic heart valve (176) includes at least nine of the openings (193a-c); and/or.
wherein the openings (193a-c) are positioned at an inlet end portion of the prosthetic heart valve (176); and/or
wherein the openings (193a-c) are eyelets; and/or
wherein the native atrioventricular valve is a native tricuspid valve.

## Patentansprüche

1. Ein System zum Ersetzen der Funktion einer nativen Atrioventrikular-Klappe, das System umfassend:
eine prothetische Herzklappe (176), die einen selbstexpandierenden Rahmen und eine Vielzahl von Klappensegeln beinhaltet, die innerhalb eines Lumens des Rahmens angeordnet sind, um Blutfluss in einer Richtung zu ermöglichen;
einen Zuführkatheter (12) mit einem länglichen Schaft (18) zum Vorschieben der prothetischen Herzklappe (176) durch das Gefäßsystem eines Patienten zu der nativen Atrioventrikular-Klappe, wobei der längliche Schaft (18) eine Kapsel (39) entlang eines distalen Endabschnitts des länglichen Schafts (18) beinhaltet, um die prothetische Herzklappe (176) in einem komprimierten Zustand zu halten;
eine Fadenanordnung (26), die sich durch den länglichen Schaft (18) des Zuführungskatheters (12) erstreckt, wobei die Fadenanordnung (26) eine Vielzahl von Koppelfäden (142a-c) beinhaltet, die entlang eines distalen Endabschnitts der Fadenanordnung (26) angeordnet sind, wobei jeder der Koppelfäden (142a-c) einen Schlaufenabschnitt (188a-c) umfasst, wobei sich die Schlaufenabschnitte (188a-c) durch jeweilige Öffnungen (193a-c) in der prothetischen Herzklappe (176) erstrecken; und
eine Freigabeanordnung (28), die einem oder mehreren flexiblen Freigabefäden (180) beinhaltet, die ausgebildet sind, sich durch Schlaufenabschnitte (188a-c) der Koppelfäden (142a-c) zu erstrecken, um die prothetische Herzklappe (176) an der Fadenanordnung (26) zu sichern, wobei der eine oder die mehreren Freigabefäden (180) zurückziehbar sind, um aus dem einen oder den mehreren Schlaufenabschnitten (188a-c) entfernt zu werden, und wodurch ermöglicht ist, dass die Fadenanordnung (26) von der prothetischen Herzklappe (176) gelöst wird;
wobei die prothetische Herzklappe (176) aus der Kapsel (39) ausgestoßen werden und expandieren und funktionieren kann, während sie noch an der Fadenanordnung (26) angebracht ist, und wobei die prothetische Herzklappe (176) von der Fadenanordnung (26) gelöst werden kann, nachdem eine ordnungsgemäße Ausbringung und Funktion in der nativen Atrioventrikular-Klappe bestätigt wurde;
wobei die Fadenanordnung (26) ferner umfasst:
einen Fadenverteiler (144), der an der Vielzahl von Koppelfäden (142a-c) angebracht ist; und
einen flexiblen Rückhaltefaden (146), der mit dem Fadenverteiler (144) gekoppelt ist und sich proximal von dem Fadenverteiler (144) erstreckt, um ein Fadenbetätigungselement (698) in Eingriff zu nehmen;
und/oder wobei die Freigabeanordnung (28) ferner umfasst:
einen Freigabefadenverteiler (182), von dem eine Vielzahl der Freigabefäden (180) aus abzweigen; und
einen einziehbaren Faden (184), der mit dem Freigabefadenverteiler (182) gekoppelt ist und sich proximal von dem Freigabefadenverteiler (182) erstreckt, um ein Freigabebetätigungselement (700) in Eingriff zu nehmen.

2. Das System nach Anspruch 1, wobei der Fadenverteiler (144) einen Draht, der eine Schlaufe bildet, umfasst, und die Vielzahl von Koppelfäden (142a-c) um die Schlaufe des Fadenverteilers (144) geschlungen sind;
vorzugsweise wobei die Vielzahl von Koppelfäden (142a-c) einen kontinuierlichen Nahtfaden umfassen, der mehrfach um die Schlaufe des Fadenverteilers (144) geschlungen ist, um jeden der Vielzahl von Koppelfäden (142a-c) zu bilden.

3. Das System nach einem der Ansprüche 1 oder 2, wobei der flexible Rückhaltefaden (146) gewebt oder geflochten ist.

4. Das System nach einem der Ansprüche 1-3, wobei jeder der Freigabefäden (180) sich durch mindestens drei der Schlaufenabschnitte (188a-c) erstreckt, um die Schlaufenabschnitte (188a-c) an der prothetischen Herzklappe (176) zu sichern; und/oder
wobei die Freigabeanordnung (28) mindestens drei der Freigabefäden (180) oder nur einen Freigabefaden (180) beinhaltet.

5. Das System nach einem der Ansprüche 1-4, wobei die Kapsel (39) zurückziehbar ist, um die prothetische Herzklappe (176) aus der Kapsel (39) auszustoßen.

6. Das System nach einem der Ansprüche 1-5, wobei der längliche Schaft (18) eine Vielzahl Schäfte umfasst, wobei mindestens einer der mehreren Schäfte einen Führungsschaft (22) umfasst, der eingerichtet ist, einen Biegeabschnitt zu bilden, um andere der Schäfte des länglichen Schafts (18) zu biegen.

7. Das System nach Anspruch 6, wobei der Führungsschaft (22) eingerichtet ist, in einer ersten Richtung in einer ersten Ebene und in einer zweiten Richtung in einer zweiten Ebene zu biegen, wobei sich die zweite Ebene senkrecht zu der ersten Ebene erstreckt;
vorzugsweise wobei der Führungsschaft (22) eingerichtet ist, relativ zu der Kapsel (39) zu gleiten, um eine Tiefe der Kapsel (39) relativ zu dem Biegeabschnitt zu variieren, und der Führungsschaft (22) eingerichtet ist, in einer dritten Richtung in der ersten Ebene zu biegen, wobei die dritte Richtung der ersten Richtung entgegengesetzt ist, um eine Höhe der Kapsel (39) zu variieren;
besonders bevorzugt wobei der Zuführkatheter (12) einen Griff (682) beinhaltet, der an einem proximalen Endabschnitt des länglichen Schafts (18) positioniert ist, wobei der Griff (682) eine Drehknopfanordnung beinhaltet zum Betätigen eines Zugfadens zum Biegen des Führungsschafts (22) in der dritten Richtung, um die Höhe der Kapsel (39) zu variieren.

8. Das System nach Anspruch 7, wobei der Zuführkatheter (12) einen Griff (682) beinhaltet, der an einem proximalen Endabschnitt des länglichen Schafts (18) positioniert ist, wobei der Griff (682) ein Deflektionsbetätigungselement (694) umfasst, das eingerichtet ist, den länglichen Schaft (18) in der ersten Richtung in der ersten Ebene abzulenken.

9. Das System nach einem der Ansprüche 1-8, wobei der Zuführkatheter (12) einen Griff (682) umfasst, der an einem proximalen Endabschnitt des länglichen Schafts (18) positioniert ist, wobei der Griff (682) ein Fadenbetätigungselement (698) zum Vorschieben oder Zurückziehen der Fadenanordnung (26) entlang des länglichen Schafts (18) beinhaltet.

10. Das System nach Anspruch 9, wobei das Fadenbetätigungselement (698) eingerichtet ist, die Fadenanordnung (26) vorzuschieben, um die prothetische Herzklappe (176) aus einem distalen Schubelement (138) des länglichen Schafts (18) zu expandieren.

11. Das System nach Anspruch 10, wobei das distale Schubelement (138) einen Einlassendabschnitt der prothetischen Herzklappe (176) komprimiert.

12. Das System nach Anspruch 10 oder Anspruch 11, wobei das Fadenbetätigungselement (698) eingerichtet ist, die Fadenanordnung (26) zurückzuziehen, um die prothetische Herzklappe (176) in das distale Schubelement (138) zurückzuziehen.

13. Das System nach einem der Ansprüche 1-12, wobei der Zuführkatheter (12) einen Griff (682) beinhaltet, der an einem proximalen Endabschnitt des länglichen Schafts (18) positioniert ist, wobei der Griff (682) ein Freigabebetätigungselement (700) beinhaltet, das betätigbar ist, um die Freigabeanordnung (28) zurückzuziehen, um den einen oder die mehreren Freigabefäden (180) aus dem einen oder den mehreren Schlaufenabschnitten (188a-c) zurückzuziehen.

14. Das System nach einem der Ansprüche 1-13, wobei der Zuführkatheter (12) einen Griff (682) umfasst, der an einem proximalen Endabschnitt des länglichen Schafts (18) positioniert ist, und wobei das System ferner einen Stabilisator (1120) umfasst, der zum Koppeln mit dem Griff (682) eingerichtet ist, um den Zuführkatheter (12) während der Freisetzung der prothetischen Herzklappe (176) an der nativen Atrioventrikular-Klappe zu stabilisieren.

15. Das System nach einem der Ansprüche 1-14, wobei die prothetische Herzklappe (176) mindestens neun der Öffnungen (193a-c) umfasst; und/oder
wobei die Öffnungen (193a-c) an einem Einlassendabschnitt der prothetischen Herzklappe (176) positioniert sind; und/oder
wobei die Öffnungen (193a-c) Ösen sind; und/oder
wobei die native Atrioventrikular-Klappe eine native Trikuspidalklappe ist.

## Revendications

1. Système pour le remplacement de la fonction d'une valvule atrioventriculaire native, le système comprenant :
une valvule cardiaque prothétique (176) comportant un cadre autodéployable et une pluralité de feuillets disposés à l'intérieur d'une lumière du cadre destinés à permettre un écoulement unidirectionnel de sang ;
un cathéter de pose (12) présentant une tige allongée (18) destiné à faire avancer la valvule cardiaque prothétique (176) à travers le système vasculaire d'un patient vers la valvule atrioventriculaire native, la tige allongée (18) comportant une capsule (39) le long d'une partie extrémité distale de la tige allongée (18) afin de maintenir la valvule cardiaque prothétique (176) dans un état comprimé ;
un ensemble attache (26) s'étendant à travers la tige allongée (18) du cathéter de pose (12), l'ensemble attache (26) comportant une pluralité d'attaches d'accouplement (142a-c) disposées le long d'une partie extrémité distale de l'ensemble attache (26), chacune des attaches d'accouplement (142a-c) comprenant une partie boucle (188a-c), les parties boucle (188a-c) s'étendant à travers des ouvertures respectives (193a-c) dans la valvule cardiaque prothétique (176) ; et
un ensemble de libération (28) comportant une ou plusieurs attaches de libération (180) flexibles conçues pour s'étendre à travers des parties boucle (188a-c) des attaches d'accouplement (142a-c) afin de fixer fermement la valvule cardiaque prothétique (176) à l'ensemble attache (26), ladite une ou lesdites plusieurs attaches de libération (180) étant rétractables pour le retrait de ladite une ou desdites plusieurs parties boucle (188a-c) et permettant ainsi la libération de l'ensemble attache (26) de la valvule cardiaque prothétique (176) ;
la valvule cardiaque prothétique (176) pouvant être éjectée à partir de la capsule (39) et autorisée à se déployer et à fonctionner tout en étant toujours attachée à l'ensemble attache (26) et la valvule cardiaque prothétique (176) pouvant être libérée de l'ensemble attache (26) après confirmation d'un déploiement et d'une fonctionnement appropriés à l'intérieur de la valvule atrioventriculaire native ;
l'ensemble attache (26) comprenant en outre :
un collecteur (144) d'attache attaché à la pluralité d'attaches d'accouplement (142a-c) ; et
une attache de retenue (146) flexible accouplée au collecteur (144) d'attache et s'étendant de manière proximale à partir du collecteur (144) d'attache afin de venir en prise avec un actionneur (698) d'attache ;
et/ou l'ensemble de libération (28) comprenant en outre :
un collecteur (182) d'attache de libération à partir duquel se ramifient une pluralité des attaches de libération (180) ; et
une attache rétractable (184) accouplée au collecteur (182) d'attache de libération et s'étendant de manière proximale à partir du collecteur (182) d'attache de libération afin venir en prise avec un actionneur de libération (700).

2. Système selon la revendication 1, le collecteur (144) d'attache comprenant un fil formant une boucle et la pluralité d'attaches d'accouplement (142a-c) bouclant autour de la boucle du collecteur (144) d'attache ;
de préférence la pluralité d'attaches d'accouplement (142a-c) comprenant une suture continue bouclée plusieurs fois autour de la boucle du collecteur (144) d'attache afin de former chacune de la pluralité d'attaches d'accouplement (142a-c).

3. Système selon l'une quelconque des revendications 1 ou 2, l'attache de retenue (146) flexible étant tissée ou tressée.

4. Système selon l'une quelconque des revendications 1 à 3, chacune des attaches de libération (180) s'étendant à travers au moins trois des parties boucle (188a-c) afin de fixer fermement les parties boucle (188a-c) à la valvule cardiaque prothétique (176) ; et/ou
l'ensemble de libération (28) comportant au moins trois des attaches de libération (180) ou seulement une attache de libération (180).

5. Système selon l'une quelconque des revendications 1 à 4, la capsule (39) étant rétractable afin d'éjecter la valvule cardiaque prothétique (176) à partir de la capsule (39).

6. Système selon l'une quelconque des revendications 1 à 5, la tige allongée (18) comportant une pluralité de tiges, au moins une tige parmi la pluralité de tiges comprenant une tige rail (22) conçue pour former une partie courbée destinée à courber d'autres tiges parmi les tiges de la tige allongée (18).

7. Système selon la revendication 6, la tige rail (22) étant conçue pour se courber dans un premier sens dans un premier plan et dans un deuxième sens dans un second plan, le second plan s'étendant transversalement par rapport au premier plan ;
de préférence la tige rail (22) étant conçue pour coulisser par rapport à la capsule (39) afin de faire varier une profondeur de la capsule (39) par rapport à la partie courbée et la tige rail (22) étant conçue pour se courber dans un troisième sens dans le premier plan, le troisième sens étant opposé au premier sens afin de faire varier une hauteur de la capsule (39) ;
plus préférablement le cathéter de pose (12) comportant une poignée (682) positionnée au niveau d'une partie extrémité proximale de la tige allongée (18), la poignée (682) comportant un ensemble bouton destiné à actionner une attache de traction afin de courber la tige rail (22) dans le troisième sens afin de faire varier la hauteur de la capsule (39).

8. Système selon la revendication 7, le cathéter de pose (12) comportant une poignée (682) positionnée au niveau d'une partie extrémité proximale de la tige allongée (18), la poignée (682) comportant un actionneur de déviation (694) conçu pour dévier la tige allongée (18) dans le premier sens dans le premier plan.

9. Système selon l'une quelconque des revendications 1 à 8, le cathéter de pose (12) comportant une poignée (682) positionnée au niveau d'une partie extrémité proximale de la tige allongée (18), la poignée (682) comportant un actionneur (698) d'attache destiné à faire avancer ou à rétracter l'ensemble attache (26) le long de la tige allongée (18).

10. Système selon la revendication 9, l'actionneur (698) d'attache étant conçu pour faire avancer l'ensemble attache (26) afin de déployer la valvule cardiaque prothétique (176) à partir d'un poussoir distal (138) de la tige allongée (18).

11. Système selon la revendication 10, le poussoir distal (138) comprimant une partie extrémité d'entrée de la valvule cardiaque prothétique (176).

12. Système selon la revendication 10 ou la revendication 11, l'actionneur (698) d'attache étant conçu pour rétracter l'ensemble attache (26) afin de rétracter la valvule cardiaque prothétique (176) dans le poussoir distal (138).

13. Système selon l'une quelconque des revendications 1 à 12, le cathéter de pose (12) comportant une poignée (682) positionnée au niveau d'une partie extrémité proximale de la tige allongée (18), la poignée (682) comportant un actionneur de libération (700) pouvant fonctionner pour la rétraction de l'ensemble de libération (28) afin de rétracter ladite une ou lesdits plusieurs attaches de libération (180) de ladite une ou desdites plusieurs parties boucle (188a-c).

14. Système selon l'une quelconque des revendications 1 à 13, le cathéter de pose (12) comportant une poignée (682) positionnée au niveau d'une partie extrémité proximale de la tige allongée (18) et le système comprenant en outre un stabilisateur (1120) destiné à être accouplé à la poignée (682) afin de stabiliser le cathéter de pose (12) pendant le déploiement de la valvule cardiaque prothétique (176) sur la valvule atrioventriculaire native.

15. Système selon l'une quelconque des revendications 1 à 14, la valvule cardiaque prothétique (176) comportant au moins neuf des ouvertures (193a-c) ; et/ou
les ouvertures (193a-c) étant positionnées au niveau d'une partie extrémité d'entrée de la valvule cardiaque prothétique (176) ; et/ou
les ouvertures (193a-c) étant des œillets ; et/ou
la valvule atrioventriculaire native étant une valvule tricuspide native.
